(19) Europäisches Patentamt
European Patent Office
Office européen des brevets

(11) EP 2 407 561 A2

(12) EUROPEAN PATENT APPLICATION

(43) Date of publication:
**18.01.2012 Bulletin 2012/03**

(51) Int Cl.:
***C12Q 1/68*** *(2006.01)* ***C12N 15/12*** *(2006.01)*
***C07K 14/745*** *(2006.01)*

(21) Application number: **11181186.5**

(22) Date of filing: **06.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **06.03.2006 US 779474 P**
**12.09.2006 US 834945 P**

(62) Document number(s) of the earlier application(s) in accordance with Art. 76 EPC:
**07752537.6 / 1 996 186**

(71) Applicant: **Humagene, Inc.**
**Scottsdale, AZ 85255 (US)**

(72) Inventor: **Osther, Kurt**
**Scottsdale, AZ 85259 (US)**

(74) Representative: **Inspicos A/S**
**Kogle Allé 2**
**P.O. Box 45**
**2970 Hørsholm (DK)**

Remarks:
This application was filed on 14-09-2011 as a divisional application to the application mentioned under INID code 62.

# (54) A method for the preparation of recombinant human fibrinogen

(57) The present invention discloses a novel method for the preparation of recombinant human proteins expressed in human cells. Specifically, the present invention relates to novel methods for the preparation of human recombinant thrombin and human recombinant fibrinogen. Moreover, the method employs serum-free culturing conditions and therefore provides recombinant human proteins expressed in human cells of increased safety to the patient when used in human medical treatments. In addition, the immunogenic response to the recombinant human proteins expressed in human cells may be lower. Human recombinant thrombin is expressed in the human embryonic kidney 293 cell line and the protein can be prepared using two different routes, one starting from a point mutated prothrombin with gla and Kringle 1 and 2 domains, and maintaining these domains during the process; the other one starting with prothrombin (non-mutated), via a prethrombin with a HPC4-Kringle 2 domain and subjecting this prethrombin to a point mutation. Human recombinant fibrinogen is expressed in the human embryonic kidney 293 cell line.

EP 2 407 561 A2

SEQUENCE LISTING

<110> HumaGene Inc.

<120> Method for the preparation of human recombinant thrombin and fibrinogen

<130> 17499EP01

<150> US 60/779,474
<151> 2006-03-06

<150> US 60/843,945
<151> 2006-09-12

<150> EP 07752537.6
<151> 2007-03-06

<160> 17

<170> PatentIn version 3.5

<210> 1
<211> 1743
<212> DNA
<213> artificial

<220>
<223> Recombinant human thrombin precursor molecule

<400> 1
gccaacacct tcttggagga ggtgcgcaag ggcaacctgg agcgagagtg cgtggaggag 60

acgtgcagct acgaggaggc cttcgaggct ctggagtcct ccacggctac ggatgtgttc 120

tgggccaagt acacagcttg tgagacagcg aggacgcctc gagataagct tgctgcatgt 180

ctggaaggta actgtgctga gggtctgggt acgaactacc gagggcatgt gaacatcacc 240

cggtcaggca ttgagtgcca gctatggagg agtcgctacc cacataagcc tgaaatcaac 300

tccactaccc atcctggggc cgacctacag gagaatttct gccgcaaccc cgacagcagc 360

accacgggac cctggtgcta cactacagac cccaccgtga ggaggcagga atgcagcatc 420

cctgtctgtg gccaggatca agtcactgta gcgatgactc cacgctccga aggctccagt 480

gtgaatctgt cacctccatt ggagcagtgt gtccctgatc gggggcagca gtaccagggg 540

cgcctggcgg tgaccacaca tgggctcccc tgcctggcct gggccagcgc acaggccaag 600

gccctgagca agcaccagga cttcaactca gctgtgcagc tggtggagaa cttctgccgc 660

aacccagacg gggatgagga gggcgcgtgg tgctatgtgg ccgggaagcc tggcgacttt 720

gggtactgcg acctcaacta ttgtgaggag gccgtggagg aggagacagg agatgggctg 780

gatgaggact cagacagggc catcgaaggg cgtaccgcca ccagtgagta ccagactttc 840

ttcaatccga ggacctttgg ctcgggagag gcagactgtg ggctgcgacc tctgttcgag 900

```
aagaagtcgc tggaggacaa aaccgaaaga gagctcctgg aatcctacat cgacgggcgc      960

attgtggagg gctcggatgc agagatcggc atgtcacctt ggcaggtgat gcttttccgg     1020

aagagtcccc aggagctgct gtgtggggcc agcctcatca gtgaccgctg ggtcctcacc     1080

gccgcccact gcctcctgta cccgccctgg gacaagaact tcaccgagaa tgaccttctg     1140

gtgcgcattg gcaagcactc ccgcaccagg tacgagcgaa acattgaaaa gatatccatg     1200

ttggaaaaga tctacatcca ccccaggtac aactggcggg agaacctgga ccgggacatt     1260

gccctgatga agctgaagaa gcctgttgcc ttcagtgact acattcaccc tgtgtgtctg     1320

cccgacaggg agacggcagc cagcttgctc caggctggat acaaggggcg ggtgacaggc     1380

tggggcaacc tgaaggagac gtggacagcc aacgttggta aggggcagcc cagtgtcctg     1440

caggtggtga acctgcccat tgtggagcgg ccggtctgca aggactccac ccggatccgc     1500

atcactgaca acatgttctg tgctggttac aagcctgatg aagggaaacg aggggatgcc     1560

tgtgaaggtg acagtggggg accctttgtc atgaagagcc cctttaacaa ccgctggtat     1620

caaatgggca tcgtctcatg gggtgaaggc tgtgaccggg atgggaaata tggcttctac     1680

acacatgtgt tccgcctgaa gaagtggata cagaaggtca ttgatcagtt tggagagtag     1740

tga                                                                   1743
```

```
<210>  2
<211>  579
<212>  PRT
<213>  artificial

<220>
<223>  Recombinant human thrombin precursor molecule

<400>  2

Ala Asn Thr Phe Leu Glu Glu Val Arg Lys Gly Asn Leu Glu Arg Glu
1               5                   10                  15

Cys Val Glu Glu Thr Cys Ser Tyr Glu Glu Ala Phe Glu Ala Leu Glu
            20                  25                  30

Ser Ser Thr Ala Thr Asp Val Phe Trp Ala Lys Tyr Thr Ala Cys Glu
        35                  40                  45

Thr Ala Arg Thr Pro Arg Asp Lys Leu Ala Ala Cys Leu Glu Gly Asn
    50                  55                  60

Cys Ala Glu Gly Leu Gly Thr Asn Tyr Arg Gly His Val Asn Ile Thr
65                  70                  75                  80
```

```
Arg Ser Gly Ile Glu Cys Gln Leu Trp Arg Ser Arg Tyr Pro His Lys
                85                  90                  95

Pro Glu Ile Asn Ser Thr Thr His Pro Gly Ala Asp Leu Gln Glu Asn
            100                 105                 110

Phe Cys Arg Asn Pro Asp Ser Ser Thr Thr Gly Pro Trp Cys Tyr Thr
        115                 120                 125

Thr Asp Pro Thr Val Arg Arg Gln Glu Cys Ser Ile Pro Val Cys Gly
    130                 135                 140

Gln Asp Gln Val Thr Val Ala Met Thr Pro Arg Ser Glu Gly Ser Ser
145                 150                 155                 160

Val Asn Leu Ser Pro Pro Leu Glu Gln Cys Val Pro Asp Arg Gly Gln
                165                 170                 175

Gln Tyr Gln Gly Arg Leu Ala Val Thr Thr His Gly Leu Pro Cys Leu
            180                 185                 190

Ala Trp Ala Ser Ala Gln Ala Lys Ala Leu Ser Lys His Gln Asp Phe
        195                 200                 205

Asn Ser Ala Val Gln Leu Val Glu Asn Phe Cys Arg Asn Pro Asp Gly
    210                 215                 220

Asp Glu Glu Gly Ala Trp Cys Tyr Val Ala Gly Lys Pro Gly Asp Phe
225                 230                 235                 240

Gly Tyr Cys Asp Leu Asn Tyr Cys Glu Glu Ala Val Glu Glu Glu Thr
                245                 250                 255

Gly Asp Gly Leu Asp Glu Asp Ser Asp Arg Ala Ile Glu Gly Arg Thr
            260                 265                 270

Ala Thr Ser Glu Tyr Gln Thr Phe Phe Asn Pro Arg Thr Phe Gly Ser
        275                 280                 285

Gly Glu Ala Asp Cys Gly Leu Arg Pro Leu Phe Glu Lys Lys Ser Leu
    290                 295                 300

Glu Asp Lys Thr Glu Arg Glu Leu Leu Glu Ser Tyr Ile Asp Gly Arg
305                 310                 315                 320
```

Ile Val Glu Gly Ser Asp Ala Glu Ile Gly Met Ser Pro Trp Gln Val
325 330 335

Met Leu Phe Arg Lys Ser Pro Gln Glu Leu Leu Cys Gly Ala Ser Leu
340 345 350

Ile Ser Asp Arg Trp Val Leu Thr Ala Ala His Cys Leu Leu Tyr Pro
355 360 365

Pro Trp Asp Lys Asn Phe Thr Glu Asn Asp Leu Leu Val Arg Ile Gly
370 375 380

Lys His Ser Arg Thr Arg Tyr Glu Arg Asn Ile Glu Lys Ile Ser Ala
385 390 395 400

Leu Glu Lys Ile Tyr Ile His Pro Arg Tyr Asn Trp Arg Glu Asn Leu
405 410 415

Asp Arg Asp Ile Ala Leu Met Lys Leu Lys Lys Pro Val Ala Phe Ser
420 425 430

Asp Tyr Ile His Pro Val Cys Leu Pro Asp Arg Glu Thr Ala Ala Ser
435 440 445

Leu Leu Gln Ala Gly Tyr Lys Gly Arg Val Thr Gly Trp Gly Asn Leu
450 455 460

Lys Glu Thr Trp Thr Ala Asn Val Gly Lys Gly Gln Pro Ser Val Leu
465 470 475 480

Gln Val Val Asn Leu Pro Ile Val Glu Arg Pro Val Cys Lys Asp Ser
485 490 495

Thr Arg Ile Arg Ile Thr Asp Asn Met Phe Cys Ala Gly Tyr Lys Pro
500 505 510

Asp Glu Gly Lys Arg Gly Asp Ala Cys Glu Gly Asp Ser Gly Gly Pro
515 520 525

Phe Val Met Lys Ser Pro Phe Asn Asn Arg Trp Tyr Gln Met Gly Ile
530 535 540

Val Ser Trp Gly Glu Gly Cys Asp Arg Asp Gly Lys Tyr Gly Phe Tyr
545 550 555 560

Thr His Val Phe Arg Leu Lys Lys Trp Ile Gln Lys Val Ile Asp Gln
565 570 575

Phe Gly Glu

<210> 3
<211> 1743
<212> DNA
<213> Homo sapiens

<400> 3
gccaacacct tcttggagga ggtgcgcaag ggcaacctgg agcgagagtg cgtggaggag 60

acgtgcagct acgaggaggc cttcgaggct ctggagtcct ccacggctac ggatgtgttc 120

tgggccaagt acacagcttg tgagacagcg aggacgcctc gagataagct tgctgcatgt 180

ctggaaggta actgtgctga gggtctgggt acgaactacc gagggcatgt gaacatcacc 240

cggtcaggca ttgagtgcca gctatggagg agtcgctacc cacataagcc tgaaatcaac 300

tccactaccc atcctggggc cgacctacag gagaatttct gccgcaaccc cgacagcagc 360

accacgggac cctggtgcta cactacagac cccaccgtga ggaggcagga atgcagcatc 420

cctgtctgtg gccaggatca agtcactgta gcgatgactc cacgctccga aggctccagt 480

gtgaatctgt cacctccatt ggagcagtgt gtccctgatc gggggcagca gtaccagggg 540

cgcctggcgg tgaccacaca tgggctcccc tgcctggcct gggccagcgc acaggccaag 600

gccctgagca agcaccagga cttcaactca gctgtgcagc tggtggagaa cttctgccgc 660

aacccagacg gggatgagga gggcgcgtgg tgctatgtgg ccgggaagcc tggcgacttt 720

gggtactgcg acctcaacta ttgtgaggag gccgtggagg aggagacagg agatgggctg 780

gatgaggact cagacagggc catcgaaggg cgtaccgcca ccagtgagta ccagactttc 840

ttcaatccga ggacctttgg ctcgggagag gcagactgtg ggctgcgacc tctgttcgag 900

aagaagtcgc tggaggacaa aaccgaaaga gagctcctgg aatcctacat cgacgggcgc 960

attgtggagg gctcggatgc agagatcggc atgtcacctt ggcaggtgat gcttttccgg 1020

aagagtcccc aggagctgct gtgtggggcc agcctcatca gtgaccgctg ggtcctcacc 1080

gccgcccact gcctcctgta cccgccctgg gacaagaact tcaccgagaa tgaccttctg 1140

gtgcgcattg gcaagcactc ccgcaccagg tacgagcgaa acattgaaaa gatatccatg 1200

ttggaaaaga tctacatcca ccccaggtac aactggcggg agaacctgga ccgggacatt 1260

gccctgatga agctgaagaa gcctgttgcc ttcagtgact acattcaccc tgtgtgtctg 1320

cccgacaggg agacggcagc cagcttgctc caggctggat acaaggggcg ggtgacaggc 1380

tggggcaacc tgaaggagac gtggacagcc aacgttggta aggggcagcc cagtgtcctg 1440

caggtggtga acctgcccat tgtggagcgg ccggtctgca aggactccac ccggatccgc 1500

atcactgaca acatgttctg tgctggttac aagcctgatg aagggaaacg aggggatgcc 1560

tgtgaaggtg acagtggggg acccttgtc atgaagagcc cctttaacaa ccgctggtat 1620

caaatgggca tcgtctcatg gggtgaaggc tgtgaccggg atgggaaata tggcttctac 1680

acacatgtgt tccgcctgaa gaagtggata cagaaggtca ttgatcagtt tggagagtag 1740

tga 1743

<210> 4
<211> 579
<212> PRT
<213> homo sapiens

<400> 4

```
Ala Asn Thr Phe Leu Glu Glu Val Arg Lys Gly Asn Leu Glu Arg Glu
1               5                   10                  15

Cys Val Glu Glu Thr Cys Ser Tyr Glu Glu Ala Phe Glu Ala Leu Glu
            20                  25                  30

Ser Ser Thr Ala Thr Asp Val Phe Trp Ala Lys Tyr Thr Ala Cys Glu
        35                  40                  45

Thr Ala Arg Thr Pro Arg Asp Lys Leu Ala Ala Cys Leu Glu Gly Asn
    50                  55                  60

Cys Ala Glu Gly Leu Gly Thr Asn Tyr Arg Gly His Val Asn Ile Thr
65                  70                  75                  80

Arg Ser Gly Ile Glu Cys Gln Leu Trp Arg Ser Arg Tyr Pro His Lys
                85                  90                  95

Pro Glu Ile Asn Ser Thr Thr His Pro Gly Ala Asp Leu Gln Glu Asn
            100                 105                 110

Phe Cys Arg Asn Pro Asp Ser Ser Thr Thr Gly Pro Trp Cys Tyr Thr
        115                 120                 125

Thr Asp Pro Thr Val Arg Arg Gln Glu Cys Ser Ile Pro Val Cys Gly
    130                 135                 140
```

```
Gln Asp Gln Val Thr Val Ala Met Thr Pro Arg Ser Glu Gly Ser Ser
145                 150                 155                 160

Val Asn Leu Ser Pro Pro Leu Glu Gln Cys Val Pro Asp Arg Gly Gln
                165                 170                 175

Gln Tyr Gln Gly Arg Leu Ala Val Thr Thr His Gly Leu Pro Cys Leu
            180                 185                 190

Ala Trp Ala Ser Ala Gln Ala Lys Ala Leu Ser Lys His Gln Asp Phe
        195                 200                 205

Asn Ser Ala Val Gln Leu Val Glu Asn Phe Cys Arg Asn Pro Asp Gly
    210                 215                 220

Asp Glu Glu Gly Ala Trp Cys Tyr Val Ala Gly Lys Pro Gly Asp Phe
225                 230                 235                 240

Gly Tyr Cys Asp Leu Asn Tyr Cys Glu Glu Ala Val Glu Glu Glu Thr
                245                 250                 255

Gly Asp Gly Leu Asp Glu Asp Ser Asp Arg Ala Ile Glu Gly Arg Thr
            260                 265                 270

Ala Thr Ser Glu Tyr Gln Thr Phe Phe Asn Pro Arg Thr Phe Gly Ser
        275                 280                 285

Gly Glu Ala Asp Cys Gly Leu Arg Pro Leu Phe Glu Lys Lys Ser Leu
    290                 295                 300

Glu Asp Lys Thr Glu Arg Glu Leu Leu Glu Ser Tyr Ile Asp Gly Arg
305                 310                 315                 320

Ile Val Glu Gly Ser Asp Ala Glu Ile Gly Met Ser Pro Trp Gln Val
                325                 330                 335

Met Leu Phe Arg Lys Ser Pro Gln Glu Leu Leu Cys Gly Ala Ser Leu
            340                 345                 350

Ile Ser Asp Arg Trp Val Leu Thr Ala Ala His Cys Leu Leu Tyr Pro
        355                 360                 365

Pro Trp Asp Lys Asn Phe Thr Glu Asn Asp Leu Leu Val Arg Ile Gly
    370                 375                 380
```

```
Lys His Ser Arg Thr Arg Tyr Glu Arg Asn Ile Glu Lys Ile Ser Met
385                 390                 395                 400

Leu Glu Lys Ile Tyr Ile His Pro Arg Tyr Asn Trp Arg Glu Asn Leu
                405                 410                 415

Asp Arg Asp Ile Ala Leu Met Lys Leu Lys Lys Pro Val Ala Phe Ser
            420                 425                 430

Asp Tyr Ile His Pro Val Cys Leu Pro Asp Arg Glu Thr Ala Ala Ser
        435                 440                 445

Leu Leu Gln Ala Gly Tyr Lys Gly Arg Val Thr Gly Trp Gly Asn Leu
    450                 455                 460

Lys Glu Thr Trp Thr Ala Asn Val Gly Lys Gly Gln Pro Ser Val Leu
465                 470                 475                 480

Gln Val Val Asn Leu Pro Ile Val Glu Arg Pro Val Cys Lys Asp Ser
                485                 490                 495

Thr Arg Ile Arg Ile Thr Asp Asn Met Phe Cys Ala Gly Tyr Lys Pro
            500                 505                 510

Asp Glu Gly Lys Arg Gly Asp Ala Cys Glu Gly Asp Ser Gly Gly Pro
        515                 520                 525

Phe Val Met Lys Ser Pro Phe Asn Asn Arg Trp Tyr Gln Met Gly Ile
    530                 535                 540

Val Ser Trp Gly Glu Gly Cys Asp Arg Asp Gly Lys Tyr Gly Phe Tyr
545                 550                 555                 560

Thr His Val Phe Arg Leu Lys Lys Trp Ile Gln Lys Val Ile Asp Gln
                565                 570                 575

Phe Gly Glu
```

<210> 5
<211> 1257
<212> DNA
<213> artificial

<220>
<223> recombinant human thrombin precursor molecule

<400> 5
ctgtcacctc cattggagca gtgtgtccct gatcgggggc agcagtacca ggggcgcctg 60

gcggtgacca cacatgggct cccctgcctg gcctgggcca gcgcacaggc caaggccctg 120

agcaagcacc aggacttcaa ctcagctgtg cagctggtgg agaacttctg ccgcaaccca 180

gacggggatg aggagggcgc gtggtgctat gtggccggga agcctggcga ctttgggtac 240

tgcgacctca actattgtga ggaggccgtg gaggaggaga caggagatgg gctggatgag 300

gactcagaca gggccatcga agggcgtacc gccaccagtg agtaccagac tttcttcaat 360

ccgaggacct ttggctcggg agaggcagac tgtgggctgc gacctctgtt cgagaagaag 420

tcgctggagg acaaaaccga aagagagctc ctggaatcct acatcgacgg gcgcattgtg 480

gagggctcgg atgcagagat cggcatgtca ccttggcagg tgatgctttt ccggaagagt 540

ccccaggagc tgctgtgtgg ggccagcctc atcagtgacc gctgggtcct caccgccgcc 600

cactgcctcc tgtacccgcc ctgggacaag aacttcaccg agaatgacct tctggtgcgc 660

attggcaagc actcccgcac caggtacgag cgaaacattg aaaagatatc cgccttggaa 720

aagatctaca tccaccccag gtacaactgg cgggagaacc tggaccggga cattgccctg 780

atgaagctga agaagcctgt tgccttcagt gactacattc accctgtgtg tctgcccgac 840

agggagacgg cagccagctt gctccaggct ggatacaagg ggcgggtgac aggctggggc 900

aacctgaagg agacgtggac agccaacgtt ggtaaggggc agcccagtgt cctgcaggtg 960

gtgaacctgc ccattgtgga gcggccggtc tgcaaggact ccacccggat ccgcatcact 1020

gacaacatgt tctgtgctgg ttacaagcct gatgaaggga aacgagggga tgcctgtgaa 1080

ggtgacagtg gggacccttt tgtcatgaag agccccttta acaaccgctg gtatcaaatg 1140

ggcatcgtct catggggtga aggctgtgac cgggatggga aatatggctt ctacacacat 1200

gtgttccgcc tgaagaagtg gatacagaag gtcattgatc agtttggaga gtagtga 1257

<210> 6
<211> 1311
<212> DNA
<213> artificial

<220>
<223> recombinant human thrombin precursor molecule

<400> 6
gcagcaaagc ttgaagacca agtagatccg cggctcattg atgggaaggt cgacctgtca 60

cctccattgg agcagtgtgt ccctgatcgg gggcagcagt accaggggcg cctggcggtg 120

accacacatg ggctcccctg cctggcctgg gccagcgcac aggccaaggc cctgagcaag 180

caccaggact tcaactcagc tgtgcagctg gtggagaact tctgccgcaa cccagacggg 240

gatgaggagg gcgcgtggtg ctatgtggcc gggaagcctg gcgactttgg gtactgcgac 300

ctcaactatt gtgaggaggc cgtggaggag gagacaggag atgggctgga tgaggactca 360

gacagggcca tcgaagggcg taccgccacc agtgagtacc agactttctt caatccgagg 420

acctttggct cgggagaggc agactgtggg ctgcgacctc tgttcgagaa gaagtcgctg 480

gaggacaaaa ccgaaagaga gctcctggaa tcctacatcg acgggcgcat tgtggagggc 540

tcggatgcag agatcggcat gtcaccttgg caggtgatgc ttttccggaa gagtccccag 600

gagctgctgt gtggggccag cctcatcagt gaccgctggg tcctcaccgc cgcccactgc 660

ctcctgtacc cgccctggga caagaacttc accgagaatg accttctggt gcgcattggc 720

aagcactccc gcaccaggta cgagcgaaac attgaaaaga tatccgcctt ggaaaagatc 780

tacatccacc ccaggtacaa ctggcgggag aacctggacc gggacattgc cctgatgaag 840

ctgaagaagc ctgttgcctt cagtgactac attcaccctg tgtgtctgcc cgacagggag 900

acggcagcca gcttgctcca ggctggatac aaggggcggg tgacaggctg gggcaacctg 960

aaggagacgt ggacagccaa cgttggtaag gggcagccca gtgtcctgca ggtggtgaac 1020

ctgcccattg tggagcggcc ggtctgcaag gactccaccc ggatccgcat cactgacaac 1080

atgttctgtg ctggttacaa gcctgatgaa gggaaacgag gggatgcctg tgaaggtgac 1140

agtgggggac cctttgtcat gaagagcccc tttaacaacc gctggtatca aatgggcatc 1200

gtctcatggg gtgaaggctg tgaccgggat gggaaatatg gcttctacac acatgtgttc 1260

cgcctgaaga agtggataca gaaggtcatt gatcagtttg gagagtagtg a 1311

<210> 7
<211> 435
<212> PRT
<213> artificial

<220>
<223> human recombinant prethrombin

<400> 7

Ala Ala Lys Leu Glu Asp Gln Val Asp Pro Arg Leu Ile Asp Gly Lys
1 5 10 15

Val Asp Leu Ser Pro Pro Leu Glu Gln Cys Val Pro Asp Arg Gly Gln
20 25 30

Gln Tyr Gln Gly Arg Leu Ala Val Thr Thr His Gly Leu Pro Cys Leu
35 40 45

Ala Trp Ala Ser Ala Gln Ala Lys Ala Leu Ser Lys His Gln Asp Phe
50 55 60

Asn Ser Ala Val Gln Leu Val Glu Asn Phe Cys Arg Asn Pro Asp Gly
65 70 75 80

Asp Glu Glu Gly Ala Trp Cys Tyr Val Ala Gly Lys Pro Gly Asp Phe
85 90 95

Gly Tyr Cys Asp Leu Asn Tyr Cys Glu Glu Ala Val Glu Glu Glu Thr
100 105 110

Gly Asp Gly Leu Asp Glu Asp Ser Asp Arg Ala Ile Glu Gly Arg Thr
115 120 125

Ala Thr Ser Glu Tyr Gln Thr Phe Phe Asn Pro Arg Thr Phe Gly Ser
130 135 140

Gly Glu Ala Asp Cys Gly Leu Arg Pro Leu Phe Glu Lys Lys Ser Leu
145 150 155 160

Glu Asp Lys Thr Glu Arg Glu Leu Leu Glu Ser Tyr Ile Asp Gly Arg
165 170 175

Ile Val Glu Gly Ser Asp Ala Glu Ile Gly Met Ser Pro Trp Gln Val
180 185 190

Met Leu Phe Arg Lys Ser Pro Gln Glu Leu Leu Cys Gly Ala Ser Leu
195 200 205

Ile Ser Asp Arg Trp Val Leu Thr Ala Ala His Cys Leu Leu Tyr Pro
210 215 220

Pro Trp Asp Lys Asn Phe Thr Glu Asn Asp Leu Leu Val Arg Ile Gly
225 230 235 240

Lys His Ser Arg Thr Arg Tyr Glu Arg Asn Ile Glu Lys Ile Ser Ala
245 250 255

Leu Glu Lys Ile Tyr Ile His Pro Arg Tyr Asn Trp Arg Glu Asn Leu
260 265 270

Asp Arg Asp Ile Ala Leu Met Lys Leu Lys Lys Pro Val Ala Phe Ser
275 280 285

Asp Tyr Ile His Pro Val Cys Leu Pro Asp Arg Glu Thr Ala Ala Ser
    290                 295                 300

Leu Leu Gln Ala Gly Tyr Lys Gly Arg Val Thr Gly Trp Gly Asn Leu
305                 310                 315                 320

Lys Glu Thr Trp Thr Ala Asn Val Gly Lys Gly Gln Pro Ser Val Leu
                325                 330                 335

Gln Val Val Asn Leu Pro Ile Val Glu Arg Pro Val Cys Lys Asp Ser
            340                 345                 350

Thr Arg Ile Arg Ile Thr Asp Asn Met Phe Cys Ala Gly Tyr Lys Pro
        355                 360                 365

Asp Glu Gly Lys Arg Gly Asp Ala Cys Glu Gly Asp Ser Gly Gly Pro
    370                 375                 380

Phe Val Met Lys Ser Pro Phe Asn Asn Arg Trp Tyr Gln Met Gly Ile
385                 390                 395                 400

Val Ser Trp Gly Glu Gly Cys Asp Arg Asp Gly Lys Tyr Gly Phe Tyr
                405                 410                 415

Thr His Val Phe Arg Leu Lys Lys Trp Ile Gln Lys Val Ile Asp Gln
            420                 425                 430

Phe Gly Glu
        435

<210> 8
<211> 1311
<212> DNA
<213> artificial

<220>
<223> Human Prethrombin wild type with HPC4 protein

<400> 8
gcagcaaagc ttgaagacca agtagatccg cggctcattg atgggaaggt cgacctgtca 60

cctccattgg agcagtgtgt ccctgatcgg gggcagcagt accaggggcg cctggcggtg 120

accacacatg ggctcccctg cctggcctgg gccagcgcac aggccaaggc cctgagcaag 180

caccaggact tcaactcagc tgtgcagctg gtggagaact tctgccgcaa cccagacggg 240

gatgaggagg gcgcgtggtg ctatgtggcc gggaagcctg gcgactttgg gtactgcgac 300

ctcaactatt gtgaggaggc cgtggaggag gagacaggag atgggctgga tgaggactca 360

gacagggcca tcgaagggcg taccgccacc agtgagtacc agactttctt caatccgagg 420

acctttggct cgggagaggc agactgtggg ctgcgacctc tgttcgagaa gaagtcgctg 480

gaggacaaaa ccgaaagaga gctcctggaa tcctacatcg acgggcgcat tgtggagggc 540

tcggatgcag agatcggcat gtcaccttgg caggtgatgc ttttccggaa gagtccccag 600

gagctgctgt gtggggccag cctcatcagt gaccgctggg tcctcaccgc cgcccactgc 660

ctcctgtacc cgccctggga caagaacttc accgagaatg accttctggt gcgcattggc 720

aagcactccc gcaccaggta cgagcgaaac attgaaaaga tatccatgtt ggaaaagatc 780

tacatccacc ccaggtacaa ctggcgggag aacctggacc gggacattgc cctgatgaag 840

ctgaagaagc ctgttgcctt cagtgactac attcaccctg tgtgtctgcc cgacagggag 900

acggcagcca gcttgctcca ggctggatac aaggggcggg tgacaggctg gggcaacctg 960

aaggagacgt ggacagccaa cgttggtaag gggcagccca gtgtcctgca ggtggtgaac 1020

ctgcccattg tggagcggcc ggtctgcaag gactccaccc ggatccgcat cactgacaac 1080

atgttctgtg ctggttacaa gcctgatgaa gggaaacgag gggatgcctg tgaaggtgac 1140

agtgggggac cctttgtcat gaagagcccc tttaacaacc gctggtatca aatgggcatc 1200

gtctcatggg gtgaaggctg tgaccgggat gggaaatatg gcttctacac acatgtgttc 1260

cgcctgaaga agtggataca gaaggtcatt gatcagtttg gagagtagtg a 1311

<210> 9
<211> 435
<212> PRT
<213> artificial

<220>
<223> Human prethrombin wild type with HPC4 protein

<400> 9

Ala Ala Lys Leu Glu Asp Gln Val Asp Pro Arg Leu Ile Asp Gly Lys
1 5 10 15

Val Asp Leu Ser Pro Pro Leu Glu Gln Cys Val Pro Asp Arg Gly Gln
20 25 30

Gln Tyr Gln Gly Arg Leu Ala Val Thr Thr His Gly Leu Pro Cys Leu
35 40 45

Ala Trp Ala Ser Ala Gln Ala Lys Ala Leu Ser Lys His Gln Asp Phe
50 55 60

```
Asn Ser Ala Val Gln Leu Val Glu Asn Phe Cys Arg Asn Pro Asp Gly
65                  70                  75                  80

Asp Glu Glu Gly Ala Trp Cys Tyr Val Ala Gly Lys Pro Gly Asp Phe
                85                  90                  95

Gly Tyr Cys Asp Leu Asn Tyr Cys Glu Glu Ala Val Glu Glu Glu Thr
            100                 105                 110

Gly Asp Gly Leu Asp Glu Asp Ser Asp Arg Ala Ile Glu Gly Arg Thr
        115                 120                 125

Ala Thr Ser Glu Tyr Gln Thr Phe Phe Asn Pro Arg Thr Phe Gly Ser
    130                 135                 140

Gly Glu Ala Asp Cys Gly Leu Arg Pro Leu Phe Glu Lys Lys Ser Leu
145                 150                 155                 160

Glu Asp Lys Thr Glu Arg Glu Leu Leu Glu Ser Tyr Ile Asp Gly Arg
                165                 170                 175

Ile Val Glu Gly Ser Asp Ala Glu Ile Gly Met Ser Pro Trp Gln Val
            180                 185                 190

Met Leu Phe Arg Lys Ser Pro Gln Glu Leu Leu Cys Gly Ala Ser Leu
        195                 200                 205

Ile Ser Asp Arg Trp Val Leu Thr Ala Ala His Cys Leu Leu Tyr Pro
    210                 215                 220

Pro Trp Asp Lys Asn Phe Thr Glu Asn Asp Leu Leu Val Arg Ile Gly
225                 230                 235                 240

Lys His Ser Arg Thr Arg Tyr Glu Arg Asn Ile Glu Lys Ile Ser Met
                245                 250                 255

Leu Glu Lys Ile Tyr Ile His Pro Arg Tyr Asn Trp Arg Glu Asn Leu
            260                 265                 270

Asp Arg Asp Ile Ala Leu Met Lys Leu Lys Lys Pro Val Ala Phe Ser
        275                 280                 285

Asp Tyr Ile His Pro Val Cys Leu Pro Asp Arg Glu Thr Ala Ala Ser
    290                 295                 300
```

Leu Leu Gln Ala Gly Tyr Lys Gly Arg Val Thr Gly Trp Gly Asn Leu
305                 310                 315                 320

Lys Glu Thr Trp Thr Ala Asn Val Gly Lys Gly Gln Pro Ser Val Leu
                325                 330                 335

Gln Val Val Asn Leu Pro Ile Val Glu Arg Pro Val Cys Lys Asp Ser
            340                 345                 350

Thr Arg Ile Arg Ile Thr Asp Asn Met Phe Cys Ala Gly Tyr Lys Pro
        355                 360                 365

Asp Glu Gly Lys Arg Gly Asp Ala Cys Glu Gly Asp Ser Gly Gly Pro
    370                 375                 380

Phe Val Met Lys Ser Pro Phe Asn Asn Arg Trp Tyr Gln Met Gly Ile
385                 390                 395                 400

Val Ser Trp Gly Glu Gly Cys Asp Arg Asp Gly Lys Tyr Gly Phe Tyr
                405                 410                 415

Thr His Val Phe Arg Leu Lys Lys Trp Ile Gln Lys Val Ile Asp Gln
            420                 425                 430

Phe Gly Glu
        435

<210> 10
<211> 1911
<212> DNA
<213> homo sapiens

<400> 10
gtcctaagtg tggtgggcac agcatggact gcagatagtg gtgaaggtga ctttctagct 60

gaaggaggag gcgtgcgtgg cccaagggtt gtggaaagac atcaatctgc ctgcaaagat 120

tcagactggc ccttctgctc tgatgaagac tggaactaca aatgcccttc tggctgcagg 180

atgaaagggt tgattgatga agtcaatcaa gattttacaa acagaataaa taagctcaaa 240

aattcactat ttgaatatca gaagaacaat aaggattctc attcgttgac cactaatata 300

atggaaattt tgagaggcga tttttcctca gccaataacc gtgataatac ctacaaccga 360

gtgtcagagg atctgagaag cagaattgaa gtcctgaagc gcaaagtcat agaaaaagta 420

cagcatatcc agcttctgca aaaaaatgtt agagctcagt tggttgatat gaaacgactg 480

gaggtggaca ttgatattaa gatccgatct tgtcgagggt catgcagtag ggctttagct 540

```
cgtgaagtag atctgaagga ctatgaagat cagcagaagc aacttgaaca ggtcattgcc      600
aaagacttac ttccctctag agataggcaa cacttaccac tgatcaaaat gaaaccagtt      660
ccagacttgg ttcccggaaa ttttaagagc cagcttcaga aggtaccccc agagtggaag      720
gcattaacag acatgccgca gatgagaatg gagttagaga gacctggtgg aaatgagatt      780
actcgaggag gctccacttc ttatggaacc ggatcagaga cggaaagccc aaggaaccct      840
agcagtgctg gaagctggaa ctctgggagc tctggacctg gaagtactgg aaaccgaaac      900
cctgggagct ctgggactgg agggactgca acctggaaac ctggaagctc tggacctgga      960
agtactggaa gctggaactc tgggagctct ggaactggaa gtactggaaa ccaaaaccct     1020
gggagcccta gacctggtag taccggaacc tggaatcctg gcagctctga acgcggaagt     1080
gctggacact ggacttctga gagctctgta tctggtagta ctggacaatg gcactctgaa     1140
tctggaagtt ttaggccaga tagcccaggc tctgggaacg cgaggcctaa caacccagac     1200
tggggcacat ttgaagaggt gtcaggaaat gtaagtccag ggacaaggag agagtaccac     1260
acagaaaaac tggtcacttc taaaggagat aaagagctca ggactggtaa agagaaggtc     1320
acctctggta gcacaaccac cacgcgtcgt tcatgctcta aaaccgttac taagactgtt     1380
attggtcctg atggtcacaa agaagttacc aaagaagtgg tgacctccga agatggttct     1440
gactgtcccg aggcaatgga tttaggcaca ttgtctggca taggcaccct ggatgggttc     1500
cgccataggc accctgatga agctgccttc ttcgacactg cctcaactgg aaaaacattc     1560
ccaggtttct tctcacctat gttaggagag tttgtcagtg agactgagtc taggggctca     1620
gaatctggca tcttcacaaa tacaaaggaa tccagttctc atcaccctgg gatagctgaa     1680
ttcccttccc gtggtaaatc ttcaagttac agcaaacaat ttactagtag cacgagttac     1740
aacagaggag actccacatt tgaaagcaag agctataaaa tggcagatga ggccggaagt     1800
gaagccgatc atgaaggaac acatagcacc aagagaggcc atgctaaatc tcgccctgtc     1860
agaggtatcc acacttctcc tttggggaag ccttccctgt ccccctagtg a              1911
```

<210> 11
<211> 625
<212> PRT
<213> homo sapiens

<400> 11

```
Ala Asp Ser Gly Glu Gly Asp Phe Leu Ala Glu Gly Gly Gly Val Arg
1               5                   10                  15

Gly Pro Arg Val Val Glu Arg His Gln Ser Ala Cys Lys Asp Ser Asp
            20                  25                  30
```

```
Trp Pro Phe Cys Ser Asp Glu Asp Trp Asn Tyr Lys Cys Pro Ser Gly
        35                  40                  45

Cys Arg Met Lys Gly Leu Ile Asp Glu Val Asn Gln Asp Phe Thr Asn
    50                  55                  60

Arg Ile Asn Lys Leu Lys Asn Ser Leu Phe Glu Tyr Gln Lys Asn Asn
65                  70                  75                  80

Lys Asp Ser His Ser Leu Thr Thr Asn Ile Met Glu Ile Leu Arg Gly
                85                  90                  95

Asp Phe Ser Ser Ala Asn Asn Arg Asp Asn Thr Tyr Asn Arg Val Ser
            100                 105                 110

Glu Asp Leu Arg Ser Arg Ile Glu Val Leu Lys Arg Lys Val Ile Glu
        115                 120                 125

Lys Val Gln His Ile Gln Leu Leu Gln Lys Asn Val Arg Ala Gln Leu
    130                 135                 140

Val Asp Met Lys Arg Leu Glu Val Asp Ile Asp Ile Lys Ile Arg Ser
145                 150                 155                 160

Cys Arg Gly Ser Cys Ser Arg Ala Leu Ala Arg Glu Val Asp Leu Lys
                165                 170                 175

Asp Tyr Glu Asp Gln Gln Lys Gln Leu Glu Gln Val Ile Ala Lys Asp
            180                 185                 190

Leu Leu Pro Ser Arg Asp Arg Gln His Leu Pro Leu Ile Lys Met Lys
        195                 200                 205

Pro Val Pro Asp Leu Val Pro Gly Asn Phe Lys Ser Gln Leu Gln Lys
    210                 215                 220

Val Pro Pro Glu Trp Lys Ala Leu Thr Asp Met Pro Gln Met Arg Met
225                 230                 235                 240

Glu Leu Glu Arg Pro Gly Gly Asn Glu Ile Thr Arg Gly Gly Ser Thr
                245                 250                 255

Ser Tyr Gly Thr Gly Ser Glu Thr Glu Ser Pro Arg Asn Pro Ser Ser
            260                 265                 270
```

Ala Gly Ser Trp Asn Ser Gly Ser Ser Gly Pro Gly Ser Thr Gly Asn
275 280 285

Arg Asn Pro Gly Ser Ser Gly Thr Gly Gly Thr Ala Thr Trp Lys Pro
290 295 300

Gly Ser Ser Gly Pro Gly Ser Thr Gly Ser Trp Asn Ser Gly Ser Ser
305 310 315 320

Gly Thr Gly Ser Thr Gly Asn Gln Asn Pro Gly Ser Pro Arg Pro Gly
325 330 335

Ser Thr Gly Thr Trp Asn Pro Gly Ser Ser Glu Arg Gly Ser Ala Gly
340 345 350

His Trp Thr Ser Glu Ser Ser Val Ser Gly Ser Thr Gly Gln Trp His
355 360 365

Ser Glu Ser Gly Ser Phe Arg Pro Asp Ser Pro Gly Ser Gly Asn Ala
370 375 380

Arg Pro Asn Asn Pro Asp Trp Gly Thr Phe Glu Glu Val Ser Gly Asn
385 390 395 400

Val Ser Pro Gly Thr Arg Arg Glu Tyr His Thr Glu Lys Leu Val Thr
405 410 415

Ser Lys Gly Asp Lys Glu Leu Arg Thr Gly Lys Glu Lys Val Thr Ser
420 425 430

Gly Ser Thr Thr Thr Thr Arg Arg Ser Cys Ser Lys Thr Val Thr Lys
435 440 445

Thr Val Ile Gly Pro Asp Gly His Lys Glu Val Thr Lys Glu Val Val
450 455 460

Thr Ser Glu Asp Gly Ser Asp Cys Pro Glu Ala Met Asp Leu Gly Thr
465 470 475 480

Leu Ser Gly Ile Gly Thr Leu Asp Gly Phe Arg His Arg His Pro Asp
485 490 495

Glu Ala Ala Phe Phe Asp Thr Ala Ser Thr Gly Lys Thr Phe Pro Gly
500 505 510

Phe Phe Ser Pro Met Leu Gly Glu Phe Val Ser Glu Thr Glu Ser Arg
515 520 525

Gly Ser Glu Ser Gly Ile Phe Thr Asn Thr Lys Glu Ser Ser Ser His
530 535 540

His Pro Gly Ile Ala Glu Phe Pro Ser Arg Gly Lys Ser Ser Ser Tyr
545 550 555 560

Ser Lys Gln Phe Thr Ser Ser Thr Ser Tyr Asn Arg Gly Asp Ser Thr
565 570 575

Phe Glu Ser Lys Ser Tyr Lys Met Ala Asp Glu Ala Gly Ser Glu Ala
580 585 590

Asp His Glu Gly Thr His Ser Thr Lys Arg Gly His Ala Lys Ser Arg
595 600 605

Pro Val Arg Gly Ile His Thr Ser Pro Leu Gly Lys Pro Ser Leu Ser
610 615 620

Pro
625

<210> 12
<211> 1389
<212> DNA
<213> homo sapiens

<400> 12
caaggtgtca acgacaatga ggagggtttc ttcagtgccc gtggtcatcg accccttgac 60

aagaagagag aagaggctcc cagcctgagg cctgccccac cgcccatcag tggaggtggc 120

tatcgggctc gtccagccaa agcagctgcc actcaaaaga aagtagaaag aaaagcccct 180

gatgctggag gctgtcttca cgctgaccca gacctggggg tgttgtgtcc tacaggatgt 240

cagttgcaag aggctttgct acaacaggaa aggccaatca gaaatagtgt tgatgagtta 300

aataacaatg tggaagctgt ttcccagacc tcctcttctt cctttcagta catgtatttg 360

ctgaaagacc tgtggcaaaa gaggcagaag caagtaaaag ataatgaaaa tgtagtcaat 420

gagtactcct cagaactgga aaagcaccaa ttatatatag atgagactgt gaatagcaat 480

atcccaacta accttcgtgt gcttcgttca atcctggaaa acctgagaag caaaatacaa 540

aagttagaat ctgatgtctc agctcaaatg gaatattgtc gcaccccatg cactgtcagt 600

tgcaatattc ctgtggtgtc tggcaaagaa tgtgaggaaa ttatcaggaa aggaggtgaa 660

acatctgaaa tgtatctcat tcaacctgac agttctgtca aaccgtatag agtatactgt 720

gacatgaata cagaaaatgg aggatggaca gtgattcaga accgtcaaga cggtagtgtt 780

gactttggca ggaaatggga tccatataaa cagggatttg gaaatgttgc aaccaacaca 840

gatgggaaga attactgtgg cctaccaggt gaatattggc ttggaaatga taaaattagc 900

cagcttacca ggatgggacc cacagaactt ttgatagaaa tggaggactg gaaaggagac 960

aaagtaaagg ctcactatgg aggattcact gtacagaatg aagccaacaa ataccagatc 1020

tcagtgaaca aatacagagg aacagccggt aatgccctca tggatggagc atctcagctg 1080

atgggagaaa acaggaccat gaccattcac aacggcatgt tcttcagcac gtatgacaga 1140

gacaatgacg gctggttaac atcagatccc agaaaacagt gttctaaaga agacggtggt 1200

ggatggtggt ataatagatg tcatgcagcc aatccaaacg gcagatacta ctggggtgga 1260

cagtacacct gggacatggc aaagcatggc acagatgatg gtgtagtatg gatgaattgg 1320

aaggggtcat ggtactcaat gaggaagatg agtatgaaga tcaggccctt cttcccacag 1380

caatagtga 1389

<210> 13
<211> 461
<212> PRT
<213> homo sapiens

<400> 13

Gln Gly Val Asn Asp Asn Glu Glu Gly Phe Phe Ser Ala Arg Gly His
1 5 10 15

Arg Pro Leu Asp Lys Lys Arg Glu Glu Ala Pro Ser Leu Arg Pro Ala
20 25 30

Pro Pro Pro Ile Ser Gly Gly Gly Tyr Arg Ala Arg Pro Ala Lys Ala
35 40 45

Ala Ala Thr Gln Lys Lys Val Glu Arg Lys Ala Pro Asp Ala Gly Gly
50 55 60

Cys Leu His Ala Asp Pro Asp Leu Gly Val Leu Cys Pro Thr Gly Cys
65 70 75 80

Gln Leu Gln Glu Ala Leu Leu Gln Gln Glu Arg Pro Ile Arg Asn Ser
85 90 95

Val Asp Glu Leu Asn Asn Asn Val Glu Ala Val Ser Gln Thr Ser Ser
100 105 110

Ser Ser Phe Gln Tyr Met Tyr Leu Leu Lys Asp Leu Trp Gln Lys Arg
115 120 125

Gln Lys Gln Val Lys Asp Asn Glu Asn Val Val Asn Glu Tyr Ser Ser
130 135 140

Glu Leu Glu Lys His Gln Leu Tyr Ile Asp Glu Thr Val Asn Ser Asn
145 150 155 160

Ile Pro Thr Asn Leu Arg Val Leu Arg Ser Ile Leu Glu Asn Leu Arg
165 170 175

Ser Lys Ile Gln Lys Leu Glu Ser Asp Val Ser Ala Gln Met Glu Tyr
180 185 190

Cys Arg Thr Pro Cys Thr Val Ser Cys Asn Ile Pro Val Val Ser Gly
195 200 205

Lys Glu Cys Glu Glu Ile Ile Arg Lys Gly Gly Glu Thr Ser Glu Met
210 215 220

Tyr Leu Ile Gln Pro Asp Ser Ser Val Lys Pro Tyr Arg Val Tyr Cys
225 230 235 240

Asp Met Asn Thr Glu Asn Gly Gly Trp Thr Val Ile Gln Asn Arg Gln
245 250 255

Asp Gly Ser Val Asp Phe Gly Arg Lys Trp Asp Pro Tyr Lys Gln Gly
260 265 270

Phe Gly Asn Val Ala Thr Asn Thr Asp Gly Lys Asn Tyr Cys Gly Leu
275 280 285

Pro Gly Glu Tyr Trp Leu Gly Asn Asp Lys Ile Ser Gln Leu Thr Arg
290 295 300

Met Gly Pro Thr Glu Leu Leu Ile Glu Met Glu Asp Trp Lys Gly Asp
305 310 315 320

Lys Val Lys Ala His Tyr Gly Gly Phe Thr Val Gln Asn Glu Ala Asn
325 330 335

Lys Tyr Gln Ile Ser Val Asn Lys Tyr Arg Gly Thr Ala Gly Asn Ala
340 345 350

Leu Met Asp Gly Ala Ser Gln Leu Met Gly Glu Asn Arg Thr Met Thr
355 360 365

Ile His Asn Gly Met Phe Phe Ser Thr Tyr Asp Arg Asp Asn Asp Gly
370 375 380

Trp Leu Thr Ser Asp Pro Arg Lys Gln Cys Ser Lys Glu Asp Gly Gly
385 390 395 400

Gly Trp Trp Tyr Asn Arg Cys His Ala Ala Asn Pro Asn Gly Arg Tyr
405 410 415

Tyr Trp Gly Gly Gln Tyr Thr Trp Asp Met Ala Lys His Gly Thr Asp
420 425 430

Asp Gly Val Val Trp Met Asn Trp Lys Gly Ser Trp Tyr Ser Met Arg
435 440 445

Lys Met Ser Met Lys Ile Arg Pro Phe Phe Pro Gln Gln
450 455 460

<210> 14
<211> 1272
<212> DNA
<213> homo sapiens

<400> 14
gctcttttat ttctctcttc aacatgtgta gcatatgttg ctaccagaga caactgctgc 60

atcttagatg aaagattcgg tagttattgt ccaactacct gtggcattgc agatttcctg 120

tctacttatc aaaccaaagt agacaaggat ctacagtctt tggaagacat cttacatcaa 180

gttgaaaaca aaacatcaga agtcaaacag ctgataaaag caatccaact cacttataat 240

cctgatgaat catcaaaacc aaatatgata gacgctgcta ctttgaagtc caggaaaatg 300

ttagaagaaa ttatgaaata tgaagcatcg attttaacac atgactcaag tattcgatat 360

ttgcaggaaa tatataattc aaataatcaa aagattgtta acctgaaaga gaaggtagcc 420

cagcttgaag cacagtgcca ggaaccttgc aaagacacgg tgcaaatcca tgatatcact 480

gggaaagatt gtcaagacat tgccaataag ggagctaaac agagcgggct ttactttatt 540

aaacctctga aagctaacca gcaattctta gtctactgtg aaatcgatgg gtctggaaat 600

ggatggactg tgtttcagaa gagacttgat ggcagtgtag atttcaagaa aaactggatt 660

caatataaag aaggatttgg acatctgtct cctactggca caacagaatt ttggctggga 720

aatgagaaga ttcatttgat aagcacacag tctgccatcc catatgcatt aagagtggaa 780

ctggaagact ggaatggcag aaccagtact gcagactatg ccatgttcaa ggtgggacct 840

gaagctgaca agtaccgcct aacatatgcc tacttcgctg gtggggatgc tggagatgcc 900

tttgatggct ttgattttgg cgatgatcct agtgacaagt tttcacatc ccataatggc 960

atgcagttca gtacctggga caatgacaat gataagtttg aaggcaactg tgctgaacag 1020

gatggatctg gttggtggat gaacaagtgt cacgctggcc atctcaatgg agtttattac 1080

caaggtggca cttactcaaa agcatctact cctaatggtt atgataatgg cattatttgg 1140

gccacttgga aaacccggtg gtattccatg aagaaaacca ctatgaagat aatcccattc 1200

aacagactca caattggaga aggacagcaa caccacctgg ggggagccaa acaggctgga 1260

gacgtttaat ga 1272

<210> 15
<211> 411
<212> PRT
<213> homo sapiens

<400> 15

Tyr Val Ala Thr Arg Asp Asn Cys Cys Ile Leu Asp Glu Arg Phe Gly
1 5 10 15

Ser Tyr Cys Pro Thr Thr Cys Gly Ile Ala Asp Phe Leu Ser Thr Tyr
20 25 30

Gln Thr Lys Val Asp Lys Asp Leu Gln Ser Leu Glu Asp Ile Leu His
35 40 45

Gln Val Glu Asn Lys Thr Ser Glu Val Lys Gln Leu Ile Lys Ala Ile
50 55 60

Gln Leu Thr Tyr Asn Pro Asp Glu Ser Ser Lys Pro Asn Met Ile Asp
65 70 75 80

Ala Ala Thr Leu Lys Ser Arg Lys Met Leu Glu Glu Ile Met Lys Tyr
85 90 95

Glu Ala Ser Ile Leu Thr His Asp Ser Ser Ile Arg Tyr Leu Gln Glu
100 105 110

Ile Tyr Asn Ser Asn Asn Gln Lys Ile Val Asn Leu Lys Glu Lys Val
115 120 125

```
Ala Gln Leu Glu Ala Gln Cys Gln Glu Pro Cys Lys Asp Thr Val Gln
    130                 135                 140

Ile His Asp Ile Thr Gly Lys Asp Cys Gln Asp Ile Ala Asn Lys Gly
145                 150                 155                 160

Ala Lys Gln Ser Gly Leu Tyr Phe Ile Lys Pro Leu Lys Ala Asn Gln
                165                 170                 175

Gln Phe Leu Val Tyr Cys Glu Ile Asp Gly Ser Gly Asn Gly Trp Thr
            180                 185                 190

Val Phe Gln Lys Arg Leu Asp Gly Ser Val Asp Phe Lys Lys Asn Trp
        195                 200                 205

Ile Gln Tyr Lys Glu Gly Phe Gly His Leu Ser Pro Thr Gly Thr Thr
    210                 215                 220

Glu Phe Trp Leu Gly Asn Glu Lys Ile His Leu Ile Ser Thr Gln Ser
225                 230                 235                 240

Ala Ile Pro Tyr Ala Leu Arg Val Glu Leu Glu Asp Trp Asn Gly Arg
                245                 250                 255

Thr Ser Thr Ala Asp Tyr Ala Met Phe Lys Val Gly Pro Glu Ala Asp
            260                 265                 270

Lys Tyr Arg Leu Thr Tyr Ala Tyr Phe Ala Gly Gly Asp Ala Gly Asp
        275                 280                 285

Ala Phe Asp Gly Phe Asp Phe Gly Asp Asp Pro Ser Asp Lys Phe Phe
    290                 295                 300

Thr Ser His Asn Gly Met Gln Phe Ser Thr Trp Asp Asn Asp Asn Asp
305                 310                 315                 320

Lys Phe Glu Gly Asn Cys Ala Glu Gln Asp Gly Ser Gly Trp Trp Met
                325                 330                 335

Asn Lys Cys His Ala Gly His Leu Asn Gly Val Tyr Tyr Gln Gly Gly
            340                 345                 350

Thr Tyr Ser Lys Ala Ser Thr Pro Asn Gly Tyr Asp Asn Gly Ile Ile
        355                 360                 365
```

Trp Ala Thr Trp Lys Thr Arg Trp Tyr Ser Met Lys Lys Thr Thr Met
    370                 375                 380

Lys Ile Ile Pro Phe Asn Arg Leu Thr Ile Gly Glu Gly Gln Gln His
385                 390                 395                 400

His Leu Gly Gly Ala Lys Gln Ala Gly Asp Val
                405                 410

<210>  16
<211>  39
<212>  PRT
<213>  homo sapiens

<220>
<221>  misc_feature
<222>  (6)..(7)
<223>  Xaa can be any naturally occurring amino acid

<220>
<221>  misc_feature
<222>  (14)..(14)
<223>  Xaa can be any naturally occurring amino acid

<220>
<221>  misc_feature
<222>  (16)..(16)
<223>  Xaa can be any naturally occurring amino acid

<220>
<221>  misc_feature
<222>  (19)..(20)
<223>  Xaa can be any naturally occurring amino acid

<220>
<221>  misc_feature
<222>  (25)..(26)
<223>  Xaa can be any naturally occurring amino acid

<220>
<221>  misc_feature
<222>  (29)..(29)
<223>  Xaa can be any naturally occurring amino acid

<220>
<221>  misc_feature
<222>  (32)..(32)
<223>  Xaa can be any naturally occurring amino acid

<400>  16

Ala Asn Thr Phe Leu Xaa Xaa Val Arg Lys Gly Asn Leu Xaa Arg Xaa
1               5                   10                  15

Cys Val Xaa Xaa Thr Cys Ser Tyr Xaa Xaa Ala Phe Xaa Ala Leu Xaa
20 25 30

Ser Ser Thr Ala Thr Asp Val
35

<210> 17
<211> 295
<212> PRT
<213> artificial

<220>
<223> Mutated Human alpha Thrombin M84A

<400> 17

Thr Phe Gly Ser Gly Glu Ala Asp Cys Gly Leu Arg Pro Leu Phe Glu
1 5 10 15

Lys Lys Ser Leu Glu Asp Lys Thr Glu Arg Glu Leu Leu Glu Ser Tyr
20 25 30

Ile Asp Gly Arg Ile Val Glu Gly Ser Asp Ala Glu Ile Gly Met Ser
35 40 45

Pro Trp Gln Val Met Leu Phe Arg Lys Ser Pro Gln Glu Leu Leu Cys
50 55 60

Gly Ala Ser Leu Ile Ser Asp Arg Trp Val Leu Thr Ala Ala His Cys
65 70 75 80

Leu Leu Tyr Pro Pro Trp Asp Lys Asn Phe Thr Glu Asn Asp Leu Leu
85 90 95

Val Arg Ile Gly Lys His Ser Arg Thr Arg Tyr Glu Arg Asn Ile Glu
100 105 110

Lys Ile Ser Ala Leu Glu Lys Ile Tyr Ile His Pro Arg Tyr Asn Trp
115 120 125

Arg Glu Asn Leu Asp Arg Asp Ile Ala Leu Met Lys Leu Lys Lys Pro
130 135 140

Val Ala Phe Ser Asp Tyr Ile His Pro Val Cys Leu Pro Asp Arg Glu
145 150 155 160

Thr Ala Ala Ser Leu Leu Gln Ala Gly Tyr Lys Gly Arg Val Thr Gly

```
                165                 170                 175

Trp Gly Asn Leu Lys Glu Thr Trp Thr Ala Asn Val Gly Lys Gly Gln
            180                 185                 190

Pro Ser Val Leu Gln Val Val Asn Leu Pro Ile Val Glu Arg Pro Val
        195                 200                 205

Cys Lys Asp Ser Thr Arg Ile Arg Ile Thr Asp Asn Met Phe Cys Ala
    210                 215                 220

Gly Tyr Lys Pro Asp Glu Gly Lys Arg Gly Asp Ala Cys Glu Gly Asp
225                 230                 235                 240

Ser Gly Gly Pro Phe Val Met Lys Ser Pro Phe Asn Asn Arg Trp Tyr
                245                 250                 255

Gln Met Gly Ile Val Ser Trp Gly Glu Gly Cys Asp Arg Asp Gly Lys
            260                 265                 270

Tyr Gly Phe Tyr Thr His Val Phe Arg Leu Lys Lys Trp Ile Gln Lys
        275                 280                 285

Val Ile Asp Gln Phe Gly Glu
    290                 295
```

## Description

This patent application hereby incorporates by reference in its entirety the material contained on the compact disc submitted to the USPTO in accordance with 37 C.F.R. § 1.77(b)(4). The CD containing said material was created on March 6, 2007, has a file size of 24.2 KB, and a file name of "070306 Sequence listing_WorkFile.txt".

## Field of the invention

The present invention relates to a novel concept and method for preparing compositions of recombinant human proteins, each prepared in a novel manner that create authentic or natural human proteins produced in human cells, human cell lines, human stem cells, or human precursor cells. Specifically, the present invention relates to novel methods for the preparation of human recombinant thrombin and human recombinant fibrinogen. Human recombinant thrombin can be prepared using two different routes, one starting from a point mutated prothrombin with gla and Kringle 1 and 2 domains, and maintaining these domains during the process; the other one starting with prothrombin (non-mutated), via a prethrombin with a HPC4-Kringle 2 domain and subjecting this prethrombin to a point mutation.

## Technological background

Thrombin and fibrinogen are proteins that are part of the fibrin clotting cascade. Thrombin is defined as a two chain, disulfide-bonded, glycosylated polypeptide that cleaves specific bonds in fibrinogen to produce fibrin monomers that self-assemble to form a fibrin clot.

When mixing the thrombin and the fibrinogen together, possibly with the addition of the bovine aprotinin, a final stage of the natural fibrin clotting cascade takes place. In addition to providing hemostasis, this fibrin sealant also provides ideal environment for fibroblastic proliferation as well as proliferation of other mesenchymal cells. The tissue sealant such as for instance Tisseel®, and modification of the individual amount of fibrinogen, thrombin and aprotinin decides the speed by which the clotting is taking place and the density of the clot. The tissue sealant combined in the manner that Tisseel is used, was tested by Mats Brittberg et al., in his dissertation from 1994, where he showed that for instance chondrocytes did in his studies not infiltrate the fibrin clot obtained using Tisseel, but the cells were merely lining up against the clot, which again might be theorized as appearing as a blocking mechanism, which would entrap chondrocytes in for instance cartilage defects, but not allowing the cells to infiltrate into the fibrin clot, also called fibrin glue or tissue sealant. Tisseel is routinely used as tissue sealant when autologous chondrocyte implantation are performed on patients, when either periosteal grafting are used as cover over the implanted chondrocytes or collagen type I/III (ChondroGide®) membranes as cover. The edge of the cover is sutured to the surrounding healthy cartilage, and tissue sealant (also called fibrin glue) such as for instance Tisseel is added to the suturing of the cover, so that cells can be injected below the cover and sealed off inside the defect. However, Mats Brittberg also found that autologous fibrin clot, made from the spontaneous clotting of the blood in the cartilage defects in the animals appeared to allow the chondrocytes to infiltrate and, contrary to Tisseel, be cultured in the fibrin clot. Recently it has been described that Tisseel® is used in a method called Matrix Assisted Chondrocyte Implantation (Marlovits S, et al. Knee Surg Sports Traumatol Arthrosc. (2005) 13: 451-7; Bartlett W, et al. J Bone Joint Surg Br. (2005) 87:640-5). Tissue sealant has also been used in animals to study a possible enforcement of a myocardial infarction as a scaffold capable of preserving the myocardial wall (Christman KL, et al. Tissue Eng. (2004) 10:403).

Fibrinogen and thrombin combined components, in general, appear to be significantly better for the use as sealant of tissue, such as for instance in controlling bleeding on the cut surface of organs difficult to place sutures in organs such as kidneys, where Tisseel, when compared to CoSeal (Baxter), which is made from polyethylene glycol components, showed superior advantages in the form of clotting and adhering to surfaces, where CoSeal appeared to be significantly inferior. CoSeal could not adhere or prevent sieving of blood from a cut surface, which make it non-useable when compared to fibrinogen-thrombin combinations of tissue sealant (Bernie JE, et al. J Endourol. (2005) 19: 1122-6). FLOSEAL (Baxter), which consists of collagen particles and thrombin appearing together with Tisseel, fibrinogen and thrombin, in combination was capable of preventing more extensively bleeding areas such as tested on kidneys by L'Esparance et al (L'Esparance JO, et al. J Endourol. (2005)19:1114-21), who combined the sealing using both types of tissue sealants, when compared to using Tisseel alone. In a number of other surgical interventions, tissue sealant, such as Tisseel has proven to be significantly better than suturing or stapling (Langrehr JM, Rozhl Chir. (2005) 84:399-402)

The ideal matrix for wound healing, hemostatic, has to be capable of adhering to any mammal tissue surfaces including bleeding surfaces to prevent bleeding during surgery and to conserve the patient's own blood volume, or at least minimize the necessity for the usage of natural blood products on the patient.

At the same time the tissue sealant having the above capabilities shall be safe in regards to minimize transmitting of pathogenic microbials and pathogenic prions including transfer of Mad Cow Disease. The current commercially available products used in large amount are tissue sealants such as Tisseel® (Baxter Immuno) and Beriplast® (Aventis/Behring); both these tissue sealants and many other tissue sealants build on the usage of natural human thrombin and natural human fibrinogen, - and furthermore, in the Tisseel - as well as in the Beriplast products the usage of bovine aprotinin; other tissue sealants available on the market are even more extensively based on bovine products.

Most of the commercial products used extensively, such as for instance Tisseel fibrin sealant (Baxter, Immuno) and alike contains bovine proteins such as aprotinin.

The Tisseel matrix is postulated to provide the optimum environment for fibroblast infiltration and proliferation, in addition providing hemostasis, tissue synthesis and wound healing as hypothesized. The natural thrombin used in Tisseel and in other tissue sealants has been used clinically in several years to control bleeding during surgery, for burns and in certain trauma situations (Nakamura et al. The Amer. Surgeon (1991) 57:226-230; Thompson et al. Ophthalmology (1986) 93:279-282; Harris et al., J. Bone Joint Surg. Am. (1978) 60:454-456; Craig and Asher, Spine (1997) 2:313-317; Prasad et al., Burns (1991) 17:70-71). Bovine thrombin is also a component of some commercial tissue sealants, also called tissue glues such as Costasis® (Angiotech Pharmaceuticals, Inc. Vancouver, BC, Canada), which also contains bovine collagen.

Commercial thrombin therapeutics are purified from pooled human and animal blood products and as such run the risk of contamination with viruses such as the HIV and hepatitis viruses. In comparing three commercial thrombin preparations, Suzuki and Sakuragawa (Suzuki and Sakuragawa, Thromb. Res. (1989) 53:271-278) found that the preparations contained contaminating proteins, and the human preparation contained immunoglobulin G, hepatitis B surface antigen antibodies and human immunodeficiency antibodies. Xenogeneic immunization with bovine thrombin has been reported in patients who have developed self-reactive antibodies to both human thrombin and human factor V (factor V is a contaminant in the bovine thrombin preparation) (Stricker et al., Blood (1988) 72:1375-1380); Flaherty and Weiner, Blood (1989) 73: 1388); Flaherty et al., Ann Int. Med. (1989) 111:631-634); Zehnder and Leung, Blood (1990) 76: 2011-2016); Lawson et al., Blood (1990) 76:2249-2257); Stricker et al., Blood (1988) 72:1375-1380); Berguer et al., J. Trauma (1991) 31:408-411). In 139 patients treated with Floseal (Baxter), 25 out of 139 (18%) of the patients presented with an increased titer over baseline for bovine thrombin antibodies. Whereas in a control group, consisting of patients treated with a gelatine - containing bovine thrombin sponge, 26/131 (20%) of the patients presented with an increased titer over baseline for bovine thrombin. The corresponding numbers for bovine Factor Va were 39/139 (28%) and 43/131 (33%) for the Floseal (Baxter and Control groups, respectively. The differences in the frequency of developing either bovine thrombin or bovine Factor Va antibodies between the 2 groups were not statistically significant (Winterbottom, N. et al., J. Applied Res. (2002) Vol. 2, Number1).

Results recently published by Wai Y et al. (Wai Y, Tsui V, Peng Z, Richardson R, Oreopoulos D, Tarlo SM, Clin. Exp. Allergy. (2003) 33:1730) indicate the potential for sensitization and clinical allergic responses to bovine thrombin when used for haemostasis topically in patients in hemodialysis and suggest that other haemostatic methods should be considered.

Bovine thrombin is used as an aid to hemostasis in medical and surgical procedures. At least 500,000 Americans are exposed to this therapeutic annually and reports suggest that exposure is associated with the development of autoreactive antibodies. To determine whether bovine thrombin can induce pathological autoimmunity Schoenecker et al. exposed non-autoimmune-prone galactose-$\alpha$1-3-galactose-deficient mice to the two bovine thrombin preparations currently approved for use in the United States, and found that, like humans exposed to bovine thrombin, mice developed an immune response against the therapeutic and the xenogeneic carbohydrate galactose-$\alpha$ 1-3-galactose, and some mice developed autoantibodies against clotting factors. Further, unexpectedly, a single exposure to this therapeutic also induced autoimmunity in mice with features characteristic of systemic lupus erythematosus including antibodies against nuclear antigens, native DNA, double-stranded DNA, and cardiolipin. High levels of these autoantibodies were correlated with glomerulonephritis in all mice evaluated. This autoimmune syndrome was detected in mice 15 weeks after a secondary exposure to bovine thrombin and female mice were found to develop the syndrome at a significantly greater frequency than males. Thus, these studies indicate that exposure to bovine thrombin preparations can induce a pathological systemic autoimmune syndrome with lupus-like serology (Schoenecker JG, Johnson RK, Lesher AP, Day JD, et al., American J. of Pathology, (2001) 159:1957).

In addition, concerns have recently been raised regarding the possible contamination of bovine products with pathogens such as the bovine spongiform encephalitis (BSE) agent, which is not detectable or inactivatable by conventional means. Therapeutic human blood products are also subject to contamination by viral particles such as the hepatitis virus and the human immunodeficiency virus.

In other expression systems such as bacteria (e.g., E. coli), yeast systems, to which a DNA sequence is transmitted or in insect cells, avian cells, and other animal cells to which a DNA sequence is transfected, the post translation of many human proteins, especially of the types described above, such as fibrinogen, thrombin, collagens, and other cross-linking proteins such as recombinant factor XIII, which is actually, as described in U.S. patent 6,780,411 is made in transmitted yeast, - actually expressed cytoplasmically by yeast (natural fibrinogen purified from blood contains recombinant factor XIII as the cross-linking protein stabilizing the coagulation process) referring to the U.S. patents, mainly described in U.S. patent 6,780,411, does not completely resemble the authentic natural protein as much as the recombinant human protein (e.g., fibrinogen, prothrombin, construction of Prothrombin Expression Units and Expression in Mammalian Cells, thrombin, factor XIII, collagen(s) produced in a system consisting of transfected human embryonic kidney cells and other cells (e.g., HEK 293, HEK 293T, HEK 293S, HEK 293 EBNA) maintained and producing the said protein or proteins in a serum free synthetic cell culture medium system.

As described in U.S. patent 6,780,411 recombinant human Fibrinogen, for example as produced in the milk of transgenic sheep, appears to be under-sialyated, which will be a significant difference, when compared to the invention described in this patent application, where the post translation provides a more authentical natural protein, also in the case of human recombinant fibrinogen, which is a significant difference from those products, for instance produced in cloned animals such as for instance a sheep. First, since the solubility of fibrinogen is known to depend on adequate sialyation, and as fibrinogen is in any event among the least soluble of plasma proteins, there is the real possibility that not enough soluble fibrinogen would be available in the present invention to form a fibrin gel of adequate tensile strength, when compared to the fibrinogen made using the methods described in this invention. On the contrary, in this invention the fibrinogen will be co-expressed by the fibrinogen human genes encoding $\alpha$, $\beta$, and $\gamma$ chains.

While recombinant thrombin may be produced in a variety of hosts, the most preferred recombinant thrombin until this invention was the production in CHO cells which actually, contrary to this invention requires the presence of foetal bovine serum, and thereby adding the possible disadvantages to the proteins made first of all from a source like the CHO cells, and next relying on the addition of foetal bovine serum as a significant contaminant both in regards to non human proteins added and in regards to the inherent danger of the presence of pathogenic prions derived from diseases such as mad cow disease.

Unlike the prothrombin produced according to this present invention, other recombinant prothrombin molecules have been prepared through recombinant means, where approximately 14% of the protein was abnormally carboxylated. According to U.S. Patent 5,502,034, the prothrombin made according to this invention was prepared in suitable yeast vectors for use in the present invention include YRp7 (Struhl et al., Proc. Natl. Acad, Sci. USA 76:1035-1039 (1978)), YEp13 (Broach et al., Gene 8: 121-133 (1979)), POT vectors (Kawasaki et al, U.S. Pat. No. 4,931,373, which is incorporated by reference herein), pJDB249 and pJDB219 (Beggs, Nature 275:104-108 (1978)) and derivatives thereof. The prothrombin made in yeast will not be identical to authentic natural human thrombin.

In U.S. patent # 6,037,457, a recombinant fibrinogen is produced in a preferred embodiment of the invention Chinese Hamster Ovary (CHO) cells which were used to produce recombinant fibrinogen. Illustrative conditions under which mammalian cells can be cultured to express fibrinogen are as follows: cells are grown in roller bottles with adherent microcarrier beads in a total of 200 ml of serum-free medium (Dulbecco's Modified Eagle's Medium/F12 medium containing 10 IU penicillin/ml, 10 mg streptomycin/ml, 10 U aprotinin/ml, and 10 .mu.g/ml each of insulin, sodium selenite, and transferrin) at 37.degree. C. for three weeks prior to commencing the collection of conditioned culture medium every four to seven days. However, none of the other proteins included in this invention is made in the same type of cell system, and is certainly not done in a human cell system such as for instance human kidney cell and human retina-derived PER-C6 cells or human embryonic kidney cells, or even in stem cells as well as precursor cells. The above invention actually only deals with recombinant fibrinogen alone, and therefore, does not solve the problem around having a complete pure recombinant human post translated system without the content of serum proteins.

**Summary of the invention**

The present invention provides recombinant human proteins, notably prothrombin, prethrombin, thrombin and fibrinogen, vectors and human expression systems for use in the preparation of the proteins. Moreover, the recombinant human thrombin provided in an embodiment of the invention comprises a mutation,(M84A), or in a more exact and descriptive manner according to this invention, the prothrombin analogue previously called M84A, will be "Prothrombin Analogue M400A", and the prethrombin analogue also previously called M84A, will be "Prethrombin Analogue M256A". The invention also relates to the use of the recombinant human proteins in medicine as described herein for the corresponding non-recombinant proteins. Two different routes for obtaining thrombin is described, one starting from a point mutated prothrombin with gla and Kringle 1 and 2 domains, and maintaining these domains during the whole process; the other one starting with prothrombin (non-mutated), via a prethrombin with a HPC4-Kringle 2 domain and subjecting this prethrombin to a point mutation.

The uniqueness of the invention described in this patent application to produce human recombinant authentic proteins builds upon using plasmid cDNA transfected human cells, capable of secreting the protein expressed by the particular message transfected. In this invention human cells such as human kidney cell, more specifically human embryonal kidney (HEK) cell lines (e.g., HEK 293, HEK 293T, HEK 293S, HEK 293 EBNA), and human retina-derived PER-C6 cells, or other cells such as stem cells or precursor cell lines are propagated in are producing the proteins in serum free synthetic medium.

The significant advantage in this novel human cell culturing system including stem cells and/or precursor cells, more specifically a kidney cell system, or even more specifically, a human embryonic cell line and other cell lines, called HEK 293, HEK 293T, HEK 293S, HEK 293 EBNA, and a human kidney cell and human retina-derived PER-C6 cells systems grown, maintained and capable of producing the proteins post translated from transfected DNA libraries into these cells under the unique serum free synthetic cell culture medium system, the combination of the above, is the central part of this new invention.

The efficacy of the tissue sealant made from the combination of these human recombinant proteins is, in efficacy, comparable to other known tissue sealants such as for instance Tisseel® (Baxter Immuno, Vienna, Austria), but the significant difference between the current tissue sealant available on the market - compared to the tissue sealants described in this invention, called HUMA-SEALANT™ and HUMA-SEALANT™-C is the fact that in the current tissue sealants on the market, some of the proteins used, are of natural origin and are made from human donor blood and/or some of the proteins are made from bovine material, making these products significantly unsafe when compared to HUMA-SEALANT™ and HUMA-SEALANT™-C, in regards to transfer of infections such as hepatitis, the issue around bovine proteins transfer of abnormal prions or transfer of Mad Cow Disease to patients; furthermore the bovine (xenogeneic) proteins will produce immunity in patients adding additional dimensions to the possible side effects of the current tissue sealants available on the market; on the contrary, the HUMA-SEALANT™ tissue sealant system, which this invention among others concerns, consists of recombinant human proteins post-translated in transfected human cells to obtain authentic, natural proteins making this tissue sealant system optimal in regards to safety both what concerns infectivity and immunogenecity.

The efficacy of the tissue sealant made from the combination of the human recombinant proteins claimed in this patent application is comparable to or better than other known tissue sealants made from natural protein sources such as blood, plasma or other body fluid from humans and from animals such as bovine species used in fibrin glues or tissue sealants such as for instance Tisseel® (Baxter Immuno, Vienna, Austria). One of the reasons why for instance one type of the recombinant proteins, namely the recombinant human thrombin mutants described in this patent application has intentionally been selected from the point of view that these mutants appear to show optimal ability to cleave fibrinogen and recombinant fibrinogen and at the same time show low efficacy in cleaving protein C, which is significantly different from natural thrombin. The significant difference between the current tissue sealant available on the market - compared to the tissue sealants described in this invention, named HUMA-SEALANT™ and HUMA-SEALANT™-C, is - that in the current tissue sealants on the market, the substantial proteins used, are of natural origin and are made from human donor blood and/or some of the proteins are made from bovine material, making these products significantly unsafe when compared to HUMA-SEALANT™ and HUMA-SEALANT™-C, in regards to transfer of infections such as hepatitis, the issue around bovine proteins transfer of abnormal prions or transfer of Mad Cow Disease (BSE) causing prions to patients; furthermore the bovine (xenogeneic) proteins will produce immunity in patients adding additional dimensions to the possible side effects of the current tissue sealants available on the market; on the contrary, the HUMA-SEALANT™ tissue sealant system, presented in this invention, consists of recombinant human proteins post-translated in transfected human cells to obtain authentic, natural proteins with optimal activity and with a high degree of lot to lot reproducibility, the method of which also make this tissue sealant system optimal in regards to safety both what concerns infectivity and immunogenecity. The novel and unique processing of human safe recombinant proteins for instance used to produce the tissue sealant products described in this invention is therefore significantly different when compared to the current tissue sealants available for use in patients and, furthermore, is significantly distant to other tissue sealants described in available patent applications and accessible patents.

The ideal matrix for wound healing, sealant, hemostatics, etc. has to be capable of adhering to any mammal tissue surfaces including bleeding surfaces to prevent bleeding during surgery and to conserve the patient's own blood volume, or at least minimize the necessity for the usage of natural blood products on the patient. At the same time the tissue sealant having the above capabilities shall be safe in regards to minimize transmitting of pathogenic microbials and parthogenic prions including transfer of Mad Cow Disease (or BSE). The current commercially available products used in large amount are tissue sealants such as Tisseel® (Baxter Immuno) and Beriplast® (Aventis/Behring); both these tissue sealants and many other tissue sealants build upon the usage of natural human thrombin and natural human fibrinogen, - and furthermore, in the Tisseel - as well as in the Beriplast products the usage of bovine aprotinin; other tissue sealants available on the market are even more extensively based on bovine products.

The novel and unique processing of safe recombinant proteins for instance used to produce the tissue sealant products described in this invention is therefore significantly different when compared to the current tissue sealants available for use in patients and furthermore is significantly distant to other tissue sealants described in available patent applications and accessible patents.

In particular, this particular invention is focusing on models capable of acting as Tissue Sealants and hemostatics as well as carriers of cells and carriers of drugs. The present invention is applying protein informatics to accurately identify proteins and isozymes or isoforms from the human genome, identifying the gene or genes in question, and using a novel vector system the gene or genes in question is transfected into a well defined human cell line, a stem cell line, a precursor cell line, or more specifically a human kidney cell and human retina-derived PER-C6 cells and human retina-derived PER-C6 cells, and even more specifically into a human kidney cell and human retina-derived PER-C6 cells or a human embryonal kidney cell line and other cell lines called HEK 293, HEK 293T, HEK 293S, HEK 293 EBNA. This system is in this invention used for producing proteins such as recombinant human thrombin, recombinant human fibrinogen and recombinant human collagen, or more specifically recombinant human collagen of one of the around 19-20 types of human collagens types I, II, III, IV, V, VI, VII, VIII, IX, X, XI, XII, XIII, XIV, XV, XVI, XVII, XVIII, and XIX as well as other known human collagens.

One aspect of the present invention is to provide methods for producing thrombin using recombinant methods in host cells, such as human cells, human cell lines, human stem cells, human precursor cells, more specifically, a human kidney cell and human retina-derived PER-C6 cells line, and even more specifically, a human embryonal kidney cell line and other cell lines (e.g., HEK 293, HEK 293T, HEK 293S, HEK 293 EBNA).

**Detailed description of the invention**

One objective of the invention is among others to provide biological and very safe tissue sealants, made in a novel way, in which, contrary to other compositions of tissue sealant, exclusively consists of producing all the interactive proteins each in human cell lines, which has been transfected with cDNA copies of the proteins claimed in this invention, and which is propagated by processing the cells containing the copy or copies of cDNA in serum-free medium and thereafter harvesting the specific proteins produced by said cells in serum-free medium to approach similarity to authentic human selected proteins from the cells in serum-free medium.

The present invention relates to a novel concept and method for combining certain recombinant human thrombin with fibrinogen as such as well as certain human recombinant fibrinogens to create a stable product, with an optimal fibrinogen cleavage activity and with a low effect on activation of protein C.

It is within the scope of this invention to include mammal cells and specifically the cells claimed in U.S. provisional patent application 60/779,474, which is incorporated herein by reference, with incorporated cDNA, aimed at expressing prethrombin, which are cultured according to proprietary methods in a serum-free synthetic culture medium containing nutrients required for the growth of the host cells and at the same time the secretion of the post translated prethrombin.

All the other methods described by the use of human DNA library from which cDNA libraries are produced by inserting into an expression vector driven by a strong promoter, described under "Construction of the expression vector" can be used. A stable human cell will under then produce said proteins, in certain individual amounts of each of said human recombinant proteins from one or more of the cell lines, or some of the recombinant proteins, and will create a fast working end product consisting of proteins involved in the controllable gelling or coagulation, and will be free of contaminating proteins for instance derived from any form of use of mammal cells as described in U.S. Patent Nos. 6,780,411, 5,476,777, 5,502,034 and 5,572,692, where it, on the contrary, is described that when using a transfecting method for instance recombinant human thrombin is produced either in inactive form and activated as part of the purification process. Processes for the production of rhThrombin in this way are disclosed in U.S. Pat. Nos. 5,476,777, 5,502,034 and 5,572,692, the teachings of which are incorporated herein by reference. In all these cases described, where mammal cells or other cells are used for obtaining either prothrombin such as gal prothrombin, where it is stated that the cells used to produce these proteins and actually also recombinant human proteins such as for instance recombinant human fibrinogen, as well as cross linking recombinant human proteins such as recombinant human factor XIII or other cross linking recombinant human proteins such as collagens and in particular recombinant human collagen type I and/or recombinant human collagen type III.

Any other protein combinations used in the field of creating tissue sealant for human use that are used to produce an optimal combination of proteins with the highest purity and the least risk for contaminations can not be compared to the highest purity, the smallest chance for contamination of infections, the highest level of compatibility of said proteins can not be obtained with hitherto available or accessible products, due to the fact that except for the human sealant product made as described in this invention, namely produced by transfected human cells or in particular human kidney cell and human retina-derived PER-C6 cells devoid of any added proteins usually added to the cell culture, cell growth or protein producing cell medium often consisting of natural proteins, and such as for instance bovine (e.g., foetal bovine serum) or human serum with all the uncertainties that these combination of proteins added to the cell culture might contribute, due to the fact that the only proteins that may be released from the human cells cultured, and maintained as well as brought to production of proteins in serum free synthetic medium.

All these proteins are according to this invention made in transfected human cells in serum free synthetic cell culture medium. This method will then definitely distance the proteins produced by the human cells, and more specifically human kidney cell and human retina-derived PER-C6 cells or even more specifically human embryonic kidney cells and other cell lines (e.g., HEK 293, HEK 293T, HEK 293S, HEK 293 EBNA) propagated in the serum free synthetic cell culture medium to be proteins that require authentic post-translational modification in order to have high biological activities or authentic antigenicity (for antibodies). Stem cells or precursor cells may also be used. At the same time the proteins produced in this manner will be less heterogenic, but on the contrary more homogenous, enabling a better consistency in the final protein product, more reproducible from lot to lot, functionally more active and less immunogeneic due to the lesser difference in the sugar molecules, which otherwise may be obtained in cells such as insect cells or CHO cells. At the same time the final product will also be virus free. According to the method as claimed in this invention we are capable of generating stable cell lines and easily make cells adapted to serum-free suspension.

In this manner the proteins purified from the human cell culture such as for instance the human kidney cell and human retina-derived PER-C6 cell cultures in serum free synthetic cell culture medium will be novel and very different from the protein mixture from any of the transfected cell lines to which serum proteins such as for instance fetal bovine serum or other natural proteins, human or other mammal natural proteins are added and will in more or possibly less amount be present in the final "purified" protein to be harvested. It can for instance not be excluded that proteins added to the transfected mammal cell cultures, to which serum (bovine, human or other species of proteins) is still to be considered theoretically and possibly also in practice - present in the final product, which may not exclude microorganisms, such as virus, virions, pathological prions, as for instance those sometimes present in animals that might be exposed or contaminated with "mad cow disease".

It is within the scope of this invention to include mammal cells or more specifically the human cells claimed in U.S. provisional patent application 60/779,474 with incorporated cDNA, when aimed at expressing gla-domain containing prothrombin. The N-terminal Gla domain of human prothrombin is a functional unit that has a binding site for factor Va. The prothrombin Gla domain is important for interaction of the substrate with the prothrombinase complex. The cells are cultured according to proprietary methods in a serum-free synthetic culture medium containing nutrients required for the growth of the host cells and at the same time the secretion of the post translated gla-domain containing prothrombin, which unique to this invention and which is certainly significantly different from gla-domainless prothrombin or prethrombin.

It is also within the scope of this invention to use human recombinant collagens, such as recombinant human collagen type I and/or collagen type III or a mixture of collagen type I and III, where it is shown in the U.S. provisional patent application No. 60/722.366 where the very small amount of thrombin used to enhance the collagen and fibrinogen gelating product, which provides an even stronger and better adhering tissue sealant.

It is anticipated that the proteins according to this invention are as near as human natural authentic proteins so that they do not induce immunogenecity in man.

The tissue sealant disclosed in this invention, made of for instance recombinant human thrombin or recombinant thrombin mutants or analogues with the previously described pro-coagulation effect (enzymatically cleaving fibrinogen or activating fibrinogen to build fibrin), which again has showed to have a low enzymatic effect against protein C, and an almost equal or nearly equal signaling PAR 1 effect can be included in the product, which is considered to be produced under one trade name, HUMA-SEALANT™, to, among other numerous effects and abilities (e.g., a significant cell friendly product) to be used in the same manner as for instance Tisseel, - but unlike Tisseel, and as previously described, as a vehicle or carrier for cell delivery, has to be capable of adhering well to a targeted tissue for days and even weeks to enable the matrix to produce the effect needed to be efficient in all of the above capabilities without being xenogenic, allergenic, immunogenic or without carrying pathogenic microbials as well as not exhibiting any significant toxicity.

The HUMA-SEALANT™ tissue sealant consists, as many of the competitors' fibrin sealants, at least of two active components, however as recombinant mammal fibrinogen and, more specifically a recombinant human fibrinogen, where the fibrinogen within the scope of this invention is made in cells where the cDNA contains A$\alpha$, B$\beta$, $\gamma$ fibrinogen expression thus capable of transcribing or producing a recombinant human A$\alpha$, B$\beta$, $\gamma$ fibrinogen. The other component of the HUMA-SEALANT™, recombinant thrombin or more specifically recombinant human thrombin for instance of the types described in this invention, which also is present as natural proteins in e.g., Tisseel, will, when mixed, simulate the final stages of the natural coagulation cascade, forming a fibrin clot, or a gelation and most probably also having an adhering effect, also defined as a biomatrix, but according to this invention a recombinant biomatrix, or even more specifically, a recombinant mammal biomatrix.

*Definitions*

An Expression Vector is usually a DNA molecule, which contains, among others, a DNA sequence, which is encoding a protein of interest together with a promoter and other sequences, such as a transcription terminator and polyadenylation signal, that facilitate expression of the protein.

The expression vectors contain genetic information which provides for replication in a host cell, either by autonomous replication or by integration into the host genome. It is evident to one skilled in the art that such information that provides for the autonomous replication of an expression vector in a host cell encompasses known yeast and bacterial origins of replication.

Transformation is generally applied to microorganisms.

Transfection is generally applied to cells from multicellular organisms.

Both processes consist of stably and hereditably changing the genotype of a recipient cell by introduction of purified DNA, a part selected from a DNA library.

Cultured cell: A cell capable of being cultured in medium over a number of generations. In the case of cells derived from multicellular organisms, a cultured cell is a cell isolated from the organism as a single cell, a tissue, or an explant from a tissue.

A DNA Construct is a DNA molecule, or it could be a clone of such a molecule, and could be either single or double-stranded. This molecule will have been modified through human manipulation of certain segments of DNA combined and juxtaposed in a manner, which normally does not exist in nature. A DNA construct may contain operably linked elements, which direct the transcription and translation of the DNA sequence encoding the polypeptides that are of interest. Such elements include promoters, enhancers and transcription terminators. If such a DNA construct encompass the encoding of a polypeptide of interest, which contains a secretory signal sequence, the DNA construct such elements will be considered to be capable of directing the secretion of the polypeptide.

In order to easily discriminate between the individual expressions such as prothrombins (either gla-domain (containing) prothrombin, gla-domainless prothrombin) as used by Zymogenetics for their production of recombinant human thrombin, prethrombin, and prethrombin-1, Fig. 1-4 provide an overview over these individual terms. As illustrated in Fig. 4, prethrombin is prothrombin without gla domain and without Kringle I domain (Gla-less domainless prothrombin). To facilitate cleavage and/or purification HPC4 epitope was added on N-terminal. The first part of Fig. 4 shows the prothrombin including the Gla domain, and the Kringle 1 and Kringle 2.

HPC 4 is defined as an attached marker, to which one has a monoclonal antibody to identify the molecule during the process of splitting the prothrombin to prethrombin.

By the prefix "h" of proteins is intended to mean the human wild type variant of the protein. Wild type is normally prefixed by wt.

By the prefix "r" of proteins is intended to mean a recombinant protein, which has been prepared by recombinant expression.

By the prefix "rh" of proteins is intended to mean the human wild type variant of the protein which has been prepared by recombinant expression.

By the term "sequence identity", such as "nucleic acid sequence identity" or "amino acid sequence identity" is intended to mean a quantitative measure of the degree of homology between two amino acid sequences or between two nucleic acid sequences that are optimally aligned. The two sequences must be aligned to give the best possible fit, allowing the insertion of gaps or, alternatively, truncation at the ends of the polypeptide sequences or nucleotide sequences. Sequence identity as used herein is the number of aligned amino acids which are identical, divided by the total number of amino acids in the shorter of the two sequences being compared.

By the term "more efficiently" in relation to clotting of fibrinogen by recombinant human thrombin as compared to human natural thrombin is intended to mean that a smaller amount of recombinant human thrombin is needed to provide a similar response to human natural thrombin.

Usage of filtrate through MWCO provides dialysis membrane pore sizes that are characterized by the molecular weight at which 90% of the solute will be retained (prevented from permeating) by the membrane. The permeability of a solute is dependent upon the shape of the molecule, its degree of hydration and its charge. Each of these may be influenced by the nature of the solvent, the pH and the ionic strength. As a general rule, it is best to choose a MWCO that is half the molecular weight of the solute to be retained. For effective separation, please refer to the following formula as a guide:

$$\frac{\text{MW retained}}{\text{MW passed}} \geq 25$$

This maximizes the sample recovery and minimizes the time required for dialysis.

*Protein Glycosylation*

It is an important part of this invention to assure that the recombinant human proteins are glycosylated correctly. The glycosylation of recombinant proteins in general, especially those destined for potential administration to human subjects, is of much larger critical importance than previously believed. Glycosylation profoundly affects biological activity, function, clearance from circulation, and crucially, antigenicity (Brooks, SA, Molecular Biotechnology, (2004) Vol 28, 241-256(16)) This Brooks Review gives a brief overview of human N- and O-linked protein glycosylation, summarizes what is known of the glycosylation potential of the cells of nonhuman species, and presents the implications for the biotechnology industry.

The cells of non-human species do not glycosylate their proteins in the same way as human cells do. In many cases it has been found that the differences between "human" glycosylation and "animal" glycosylation are profound. Overall, it is known that the more distant, in evolutionary terms, such as bacteria, yeasts, fungi, insects, and plants, the species used most commonly in expression systems have glycosylation repertoires least like humans, and even cells originated from animals such as for instance in family with rodents, hamsters, and actually up to the animals nearest humans, the pig, there are significant differences in the glycosylation which possibly also can explain that the apparent activity of even proteins, such as for instance recombinant human thrombin in produced hamster cells show less biological activity, different function, clearance, and, again crucially, the antigenicity. Therefore, in regards to recombinant human thrombin described in the following patents: in 5,527,692, in which BHK 570 cells and yeast were described as selected for the recombinant thrombin production; in patent 5,502,034 in which BHK 570 cells are used for the transfection of Gla-domainless prothrombin, activated to recombinant thrombin (wild type), was claimed to react with fibrinogen, but not specifically with recombinant human fibrinogen or recombinant human authentic fibrinogen. So, the host cells used as described in these patents are of either animal origin or of yeast origin, and the glycosylation is therefore not considered equal to or anywhere near to the glycosylation obtained by transfecting the human genes to be expressed into human cells, such as human embryonic kidney cells (HEK 293) as have been used in this invention.

In 5,476,777 the same cell lines are used for transfection of the gla-domainless prothrombin, and among others purified using Sepharose columns and activated using snake venom. The resulting glycosylation of the final product described in this patent will be as different as what has been described in the above patents (5,527,692 and 5,502,034).

It is within the scope of this invention to produce recombinant human authentic thrombin, due to the fact that the prothrombin was transfected in to human embryonic kidney cells in synthetic medium, and it is also within the scope of this invention to produce recombinant authentic fibrinogen, containing the 6 polypeptide chains, 2A$\alpha$, 2 B$\beta$, and 2 gamma chains constituting the intact authentic human fibrinogen, where we in the invention also have significantly purposely focused on ending up with the correct glycosylation as possible for human use, meaning that we avoided using animal cells, insect cells, yeast or bacteria as host for our two products, recombinant human thrombin or more specifically recombinant human thrombin analogue with a site mutation of Chy 84 from methionin to alanin, which we believe will not cause any antigenecity in humans, and the glycosylation will be correct, because the thrombin or thrombins claimed in this invention are produced using transfection of the plasmid into human cells, e.g., human embryonic kidney cells. Furthermore, the cells were cultured in serum free synthetic medium as a suspension culture, which appeared to give significantly higher yields, than previously described to be possible to achieve.

Many proteins in eukaryotic cells are glycoproteins because they contain oligosaccharide chains covalently linked to certain amino acids. Glycosylation is known to affect protein folding, localization and trafficking, protein solubility, antigenicity, biological activity and half-life, as well as cell-cell interactions.

Protein glycosylation can be divided into four main categories mainly depending on the linkage between the amino acid and the sugar. These are N-linked glycosylation, O-linked glycosylation, C-Mannosylation and GPI anchor attachments. N-glycosylation is characterized by the addition of a sugar to the amino group of an asparagine. In O-glycosylation, a sugar is attached to the hydroxyl group of a serine or threonine residue.

Complex carbohydrates are involved in multiple biological processes, from protein folding, oligomerization and stability, to the immune response and host-pathogen interactions (Varki, 1993). Glycoconjugates also play important roles in developmental processes, as revealed by the pathology of human diseases caused by abnormal glycosylation (Freeze and Aebi, 2005) and genetic studies in model organisms (Haltiwanger and Lowe, 2004).

There is a significant difference in glycosylation between different species. Human glycoproteins are glycosylated with a extremely diverse heterogeneous array of complex N- and O-linked glycans, which are the product of the coordinated activity of enzymes resident in the endoplasmic reticulum and Golgi apparatus of the cell. Glycosylation of proteins is highly regulated and changes during differentiation, development, under different physiological--and cell culture species from which the cell cultures derive, meaning that there is pronounced differences between the complex glycosylation in human cells versus animal cells, which will most certainly affect biological activity function, clearance in the organism, and of course antigenecity (Brooks SA, Mol. Biotechnol. (2004), Vol 28, 241-255). In many cases, the differences are profound. Overall, the species most distant to humans in evolutionary terms, such as bacteria, yeasts, fungi, insects and plants-the species used most commonly in expression systems-have glycosylation repertoires least like our own.

In addition, according to Apollo Cytokine Research (San Francisco, US) recombinant human proteins, in the case of the present invention recombinant human thrombin and recombinant human fibrinogen exhibits different glycosylation patterns when expressed in transfected human cells (e.g. HEK 293) as compared to expression, e.g., in other mammalian cells such as hamster cells (e.g., CHO, which is used by Zymogenetics for production of recombinant human thrombin) (http://www.apollocytokineresearch.com/Scientific/posters.html). The data from Apollo Cytokine Research shows that the protein TNF RII-Fc expressed in hamster cells (CHO cells) is more immunogenic, i.e. elicits immune reactions that yield production of antibodies, in mice than when expressed in human cells (modified 293).

Immunogenetic properties of proteins are determined both by their amino acid sequence and their glycosylation pattern.

*Thrombin*

Host cells containing DNA constructs of the present invention are then cultured to produce prothrombin, which in this particular invention will be activated the thrombin during purification by proteases such as factor Xa. The cells with incorporated cDNA are cultured according to proprietary methods in a serum-free synthetic medium containing nutrients required for the growth of the host cells and at the same time the section of the post translated protein or polypeptide.

Thrombin is defined as a two chain, disulfide-bonded, glycosylated polypeptide that cleaves specific bonds in fibrinogen to produce fibrin monomers that self-assemble to form a fibrin clot.

Host cells containing DNA constructs of the present invention are unique because unlike the methods used by Zymogenetics and others aimed at producing prothrombin, is in this particular invention prethrombin (which is different from prothrombin) will be activated to thrombin.

Host cells containing DNA constructs of the present invention are unique because unlike the Gla-domainless prothrombin methods used by Zymogenetics and others aimed at producing prothrombin with gla-domainless prothrombin-, is in this particular invention gla-domain-prothrombin (which is completely and significantly different from gla-domainless prothrombin) will be activated to thrombin. The presence of the gla-domain can be evidenced by using a monoclonal antibody that specifically recognize gamma-carboxyglutamyl (Gla) residues in proteins such as gla-domain containing prothrombin using a method consisting of monoclonal antibodies specific for Gla residues, as described by Brown et al. (Brown MA, Stenberg LM, Persson U, Stenflo J, J.Biol.Chem. (2000), 275:19795-19802).

The recombinant human thrombin mutants, that specifically have proven to be procoagulant thrombin mutants (with high procoagulation effect) and with the same signalling enzyme effects as the Wild Type of thrombin on cells, such as for instance mesenchymal cells. Of the procoagulant thrombin mutants which also have PAR1 signal enzyme effect on cells when compared and low anticoagulant effect - either as low as wild type thrombin or even lower. Among those recombinant thrombin mutants are for instance the type called W215P.

Of other recombinant human thrombin mutants that are within the scope of this invention, are such types as M84A, also called "Thrombin Analogue M400A" (when made from prothrombin analogue M400A), and "Thrombin Analogue M256A (when made from prethrombin analogue M256A), which clots fibrinogen at a faster rate than the wild type and the arg77A (or R77aA) mutant that lacks autolytic activity. Using the above nomenclature for Thrombin analogue M400A, when made from prothrombin analogue M400A, and Thrombin analogue M256A, when made from prethrombin analogue M256A, one can always easily distinguish, by which method the thrombin analogue in question was made, instead of having M84A as the common denominator for both activated thrombins. In the case of R77aA, the prothrombin analogue would be "Prothrombin analogue R393aA", and the R77aA analogue from prethrombin would be "Prethrombin Analogue R249aA".

Also within the scope of this invention, recombinant human thrombin mutants showing low grade - or no autolysis may be selected among the types of recombinant thrombin mutants as for instance arg77A (or R77aA). The specific aim is to produce a recombinant thrombin mutant, which include at least one or more of the recombinant human thrombin mutants described above, that can be stored refrigerated and still retain their enzymatic activity to polymerize fibrinogen and especially recombinant human fibrinogen, for instance made in mammal cells.

One object of the present invention is to provide methods for producing thrombin using recombinant methods in host cells to primarily produce precursor to thrombin called prethrombin, - such as human cells, human cell lines, human stem cells, human precursor cells, more specifically, a human kidney cell and human retina-derived PER-C6 cells line, and even more specifically, a human embryonic kidney (HEK) cell lines (e.g., HEK 293, HEK 293T, HEK 293S, HEK 293 EBNA).

One object of the present invention is to provide methods for producing thrombin using recombinant methods in host cells to primarily produce precursor to thrombin called gla-domaine containing prothrombin, - such as human cells, human cell lines, human stem cells, human precursor cells, more specifically, a human kidney cell and human retina-derived PER-C6 cells line, and even more specifically, a human embryonic kidney (HEK) cell lines (e.g., HEK 293, HEK 293T, HEK 293S, HEK 293 EBNA).

These recombinant human thrombin types will be compared to wild type human thrombin and will be recombinant human thrombin analogues or mutants.

The thrombin mutants that are part of this invention that retain comparable fibrinogen cleavage activity, but have higher expression level may have the advantage of being produced at lower manufacturing cost.

It is also within the scope of this invention that thrombin mutants retain comparable expression level but display higher fibrinogen cleavage activity and thus these mutants may lower therapeutic dosage.

Further, it is within the scope of this invention that the thrombin mutants that display reduced autolysis, which are an integrated part of this invention, may facilitate purification process and storage.

As described above it is further envisioned in this invention that mutants that lack protein C activation may have improved coagulation activity.

Mutation can be identified by sequence comparison among thrombin family members, rational site-directed mutagenesis or alanine-scanning. It is envisioned genes can be modified via insertions, substitutions, deletions or domain swapping.

The methods within the scope of this invention is built on producing recombinant mammal thrombin or more specifically recombinant human thrombin and even more specific recombinant human thrombin analogue(s), and yet even more specific a recombinant human thrombin analogue, called M84A; when all of the above recombinant human thrombins or thrombin analogue(s) are made from "gla domain prothrombin" gene, meaning that the deduced protein of recombinant human prothrombin contains signal peptide, propeptide, Gla domain, two kringle domains and a (two - chain) protease domain, the M84A analogue would be called "thrombin analogue M400A. The Gla domain is seen in Fig. 5.

In a specific embodiment of the present invention the gene sequence expressed according to the invention is any molecule that exhibit thrombin activity.

The initial fibrinogen polymerization activity can be determined by spectrophotometry.

In a specific embodiment of the present invention the gene sequence expressed according to the invention is any sequence, which encodes a molecule that exhibit thrombin activity. The gene sequence expressed according to the invention can encode natural human thrombin or any mutant thereof. Thus, in one embodiment of the invention, prothrombin encoded by the polynucleotide has a sequence 100% identical to the human prothrombin sequence.

*Fibrinogen*

Such recombinant human fibrinogen contain six polypeptide chains, 2 A$\alpha$, (blue) 2 B$\beta$, green and 2 $\gamma$, red, in a trinodular shape with a central E nodule linked to two distal D nodules (see Fig. 12) are prepared individually in a novel manner that create authentic or natural human proteins produced in mammal cells or human cells, mammal or human cell lines, mammal or human stem cells, or mammal or human precursor cells. Thrombin, including the recombinant human thrombin or the recombinant human thrombin analogue(s) encompassed in this invention catalyzes the conversion of fibrinogen, which has a trinodular shape by attaching to the FpA site at the central E nodule - linked to the two distal D nodules of the fibrinogen molecule to fibrin monomers. The fibrin monomers interact to form double-stranded protofibrils. The protofibrils aggregate to form fibrin fibres, and these fibrin fibres can theoretically be stabilized by factor XIIIa-catalyzed formation of crosslinks. The conversion of fibrinogen and in this case the conversion of recombinant human authentic fibrinogen can also be explained more simply as the conversion of fibrinogen to fibrin being triggered by thrombin, which cleaves fibrinopeptides A and B from alpha and beta chains, and thus exposes the N-terminal polymerization sites responsible for the formation of the soft clot.

In a specific embodiment of the present invention the gene sequence expressed according to the invention may be any sequence, which encodes a molecule that is a fibrinogen analogue, i.e. a molecule activated by cleavage by a serine protease such as thrombin and takes part in the clotting cascade. The gene sequence expressed according to the invention can encode natural human fibrinogen or any mutant thereof.

*Suitable expression systems*

Host cells for use in practicing this present invention can primarily include a well defined human cell lines, known to secrete proteins or peptides, which closely resembles the natural counterpart of proteins or peptides.

The present invention is applying protein informatics to accurately identify proteins and isozymes or isoforms from the human genome, identifying the gene or genes in question, and using a novel vector system the gene or genes in question is transfected into a well defined mammal or human cell line, a stem cell line, a precursor cell line, or more specifically a human kidney cell and human retina-derived cells, and even more specifically into a human kidney cell and human retina-derived PER-C6 cells or human embryonic kidney cell lines (HEK) as for instance HEK 293, HEK 293T, HEK 293S, HEK 293 EBNA. This system is in this invention used for producing proteins such as recombinant human thrombin of the types described in this invention, recombinant human fibrinogen and recombinant human collagen, or more specifically recombinant human collagen of one of the around 19- 20 types of human collagens types I, II, III, IV, V, VI, VII, VIII, IX, X, XI, XII, XIII, XIV, XV, XVI, XVII, XVIII, and XIX as well as other known human collagens.

*Construction of the expression vector*

Mammalian expression vectors for use in carrying out the production of the proteins, claimed in this invention will include a promoter capable of directing the transcription of a cloned gene or cDNA. Preferred promoters include viral promoters and cellular promoters. Viral promoters could for instance be the immediate early cytomegalovirus promoter (Boshart et al., Cell (1985) 41:521-530) or the SV40 promoter (Subramani et al., Mol. Cell. Biol. (1981) 1:854-864).

The cloned DNA sequences may be introduced into cultured mammalian cells using a variety of methods, for example, calcium phosphate-mediated transfection, described by several authors is one preferred method (Wigler M, Pellicer A, Silverstein S, Axel R. Cell (1978),14: 725; Corsaro CM, Pearson ML., Somatic Cell Genetics (1981), 7: 603; Graham FL, van der Eb AJ., Virology (1973) 52:456). Electroporation is another technique used for introducing cloned DNA sequences into mammalian cells as previously mentioned (Neumann E, Schaefer-Ridder M, Wang Y, Hofschneider PH., EMBO J. (1982) 1:841-845).

Cloned DNA sequences from DNA libraries may be introduced into cultured mammalian cells by, for example, calcium phosphate-mediated transfection (Graham FL and Van der Eb AJ, Virology (1973) 52:456; Wigler M, Pellicer A, Silverstein S, Axel R.., Cell (1978) 14:725; Corsaro CM and Pearson ML, Somatic Cell Genetics (1981) 7:603).

Neuman et al's technique introducing DNA sequences into mammalian cells by the use of electroporation may also be used (Neumann E, Schaefer-Ridder M, Wang Y, Hofschneider PH., EMBO J. (1982) 1:841-845), such as Electric impulses (8 kV/cm, 5 microseconds), which were found to increase greatly the uptake of DNA into mouse lyoma cells by electroporation in high electric fields.

To direct proteins of the present invention into the secretory pathway of human cell lines such as for instance human kidney cell and human retina-derived PER-C6 cells, human embryonic kidney cells (e.g., HEK 293, HEK 293T, HEK 293S, HEK 293 EBNA), stem cells, or even precursor cells, at least one signal sequence is linked to the DNA sequence of interest. Preferred signals may, among others, include the alpha factor signal sequence (pre-pro sequence; Kurjan and Herskowitz, Cell 30:933-943 (1982); Kurjan et al., U.S. Pat. No. 4,546,082; Brake, U.S. Pat. No. 4,870,008), and the PHO5 signal sequence (Beck et al., WO 86/00637), and the BAR1 secretory signal sequence (MacKay et al., U.S. Pat. No. 4,613,572; MacKay, WO 87/002670).

A selectable marker is normally used to identify cells along with the gene or cDNA of interest. Such preferred selectable markers for use in cultured mammalian cells could for instance include genes that confer resistance to drugs, such as neomycin, hygromycin, and methotrexate. The choice of selectable markers is within the level of ordinary skill in the art. Selectable markers can be introduced into a mammalian cell together with the gene of interest, or introduced, incorporated on the same plasmid. This type of constructs are known in the art (for example, Levinson and Simonsen, U.S. Pat. No. 4,713,339).

Transfected mammalian cells are allowed to grow for a period of time, typically a few days, to begin expressing the DNA sequence(s) introduced. Drug selection is then applied to select for growth of cells that are expressing the selectable marker in a stable fashion. For cells that have been transfected with an amplifiable selectable marker the drug concentration may be increased stepwise to select in order to increase the copy number of the cloned sequences, thereby increasing expression levels.

Methods thought to be useful for introducing expression vectors encoding gla-domainless prothrombin, is not used in this present invention due to the fact that, in the present invention encompasses the methodology in which thrombin will be activated during purification by proteases such as factor Xa.

*Culturing conditions*

Mammalian cells are generally cultured in commercially available serum-containing or serum-free media. Selection of a medium appropriate for the particular cell line used is within the level of ordinary skill in the art.

Variants of the commercially available HEK 293T cells lines and their suitable growth - and expression media may be used to further improve protein production yields. Variants of commercially available expression vectors including different promoters, secretion signals, transcription enhancers, etc., may also be used to improve protein production yields.

The scope of this invention encompasses the use of human cell lines as for instance immortalized human cells or even immortalized kidney cell lines such as human embryonic kidney (HEK) 293T cell line, or variations hereof, as well as stem cells or precursor cells.

Any cultured human cells may be used as host cells within the present invention. Preferred cultured mammalian cells for use include the COS-1 (ATCC CRL 1650), BHK, and 293 (ATCC CRL 1573; Graham et al., J. Gen. Virol. 6: 59-72 (1977)) cell lines. A preferred BHK cell line is the BHK 570 cell line (deposited with the American Type Culture Collection under accession number CRL 10314). However, we have found that a human cell line selected seems to secrete significantly better outcome with more authentic-like human proteins and/or peptides; other human cell lines may have these capabilities.

*Method for the preparation of recombinant human thrombin*

Methods are disclosed for producing thrombin. The protein is produced from host cells transformed or transfected with DNA construct(s) containing information necessary to direct the expression of thrombin precursors. The DNA constructs generally include the following operably linked elements: a transcriptional promoter, DNA sequence encoding a prothrombin, and a transcriptional terminator, however, as an integrate part of this invention a gla-domain containing prothrombin or rather, a recombinant human gla-domain containing prothrombin. Thrombin precursors produced from transformed or transfected host cells are activated either in vivo or in vitro. There is therefore a need in the art for methods for producing thrombin that is essentially free of contaminating proteins. The present invention fulfils this need and provides other related advantages.

It is within the scope of this invention that you may start up with a transfected monolayer culture of HEK 293 or HEK 293T, but it is within the scope of this invention that first, the cDNA for the particular protein, in this case the gla-domain containing prothrombin, is transfected into E. coli, and when one has increased the amount of DNA necessary to transfect the HEK 293 or HEK 293T cells, this is done using a monolayer culture of these cells, followed by an adaptation of these cells to a suspension cell culture, - transferred at the same time to an appropriate serum-free medium, after having performed the appropriate cloning and the cells are then cultured and optimized using the serum-free medium. The cell culture can then either be harvested batch wise every 7-8 days or even more or less, when the measurement of the supernatant reveals a satisfactory content of the protein to be harvested and purified, followed by the appropriate activation of the gla-domain containing prothrombin to thrombin using a method developed by HumanZyme (HumanZyme, Inc., Chicago).

In regards to the human kidney cell and human retina-derived PER-C6 cells or more specifically, the human embryonic kidney (HEK) 293T cell line - containing DNA constructs of the present invention encoding a prothrombin that will be activated during the purification of proteases such as factor Xa.

For all the types of proteins to be obtained from human immortalized cell lines encompassed in this invention are cDNA copies of certain human variants of recombinant prothrombin.

Selection of optimal media is within the level of the skill in the art. Certain thrombin precursors may preferably be produced from these transfectants by the addition of heparin or thrombin. The activation of thrombin precursors containing a thrombin cleavage site in place of the wild-type thrombin activation site (Arg-Ile) may be enhanced by heparin added to the medium. Preferably, in this scenario, between 0.5 and 5.0 U/ml of heparin is added to the serum-free medium, more preferably between 1 and 5 U/ml and most preferably 1 U/ml of heparin is added to the serum-free medium according to some authors. To activate the protein produced from human cells, more specifically human kidney cell and human retina-derived PER-C6 cells, or more specifically human embryonic kidney cell and other cell lines (e.g., HEK 293, HEK 293T, HEK 293S, HEK 293 EBNA), stem cells, precursor cells or alike, containing DNA constructs of the present invention encoding a prothrombin, or a gla-domain containing prothrombin, wherein the thrombin will be activated during the purification by proteases such as factor Xa or Va, a thrombin activation site, which will have an anticipated activity between 0.5 and 5 .mu.g/ml of thrombin, which when added to the serum-free medium, more preferably will have an anticipated activity between 1 and 2 mu.g/ml of thrombin, with 1 mu.g/ml of thrombin added to the serum-free medium as being particularly preferred.

Thrombin precursors may be purified by conventional chromatography, and the thrombin precursor may then be activated by for instance snake venom activator in a serial dilution related to the protein concentration. Alternatively, thrombin precursors may be purified by affinity chromatography using anti-thrombin antibodies. Other methods of thrombin purification have been suggested as for instance in U.S. Pat. No. 4,965,203. According to the present invention the recombinant human thrombin will be activated during purification by proteases such as factor Xa.

Purified thrombin precursors may also be activated using the proteolytical activation of prothrombin as described by Heldebrant et al. and others (Heldebrant CM, Butkowski RJ, Bajaj SP, Mann KG. J. Biol. Chem. (1973) 248:7149-7163; Downing MR, Butkowski RJ, Clark MM, Mann KG, J. Biol. Chem. (1975), 250:8897; Krishnaswamy S, Church WR, Nesheim ME, Mann KG. J. Biol. Chem. (1987) 262:3291). Thrombin precursors that contain a thrombin activation site may be activated by the addition of thrombin. The activated thrombin can then be purified using column chromatography with a salt gradient. Methods of protein purification are well known in the art, for general purposes, see Scopes R. (Scopes, R., Protein Purification, Springer-Verlag, NY (1982),; the higher purity that can be obtained the more clear-cut reaction of the thrombin, so a purification between 70 - 90 % would most probably be ideal.

In the present invention the recombinant human thrombin is produced by activating said protein during purification by proteases such as factor Xa using the novel invention in obtaining the final product thrombin, to be used as a coagulant, to stabilized clots as for instance together with fibrinogen or as a carrier of cells for transplantation or implantation into mammals including humans in order to keep the cells transplanted in the target area or target organ, alternatively together with fibrinogen and/or collagen, especially collagens of the types I, II, III, IV, V, VI, VII, VIII, IX, X, XI, XII, XIII, XIV, XV, XVI, XVII, XVIII, XIX, or a mixture of the collagens, which again chemicals or drugs (e.g., diagnostics or medicinal drugs).

One embodiment according to the present invention provides a polynucleotide encoding a recombinant human thrombin precursor molecule as defined in SEQ ID NO: 17, the polynucleotide containing i) Gla domain as defined SEQ ID NO: 16. I another embodiment, the present invention further comprises ii) Kringle 2. I yet another embodiment, the present invention further comprises a polynucleotide as described herein, wherein the polynucleotide further comprises iii) HCP4 (protein C), and the recombinant human thrombin precursor molecule encoded has HPC4 linked at the 5' end (SEQ ID NO: 6).

In an embodiment the recombinant human thrombin precursor molecule according to the present invention has autolytic activity.

One embodiment according to the present invention provides a vector comprising the polynucleotide as defined in the present invention. In a further embodiment the polynucleotide according to the present invention is further operably linked to control sequences recognised by a host cell transformed with said vector. In yet a further embodiment present invention the vector according the present invention is in the form of a plasmid vector.

One embodiment according to the present invention provides a human host cell comprising one or more vectors as defined in the present invention. In a further embodiment the human host cell is a human embryonic kidney (HEK) cell. In yet a further embodiment the human host cell is selected from the group consisting of HEK 293, HEK 293T, HEK 293S and HEK 293 EBNA.

One embodiment according to the present invention provides a method for the preparation of recombinant human prothrombin or thrombin using a human expression system. In a further embodiment the human expression system comprises a vector as defined herein. In yet a further embodiment the human expression system is a human host cell as defined herein. In yet a further embodiment the human expression system is cultured under serum-free conditions.

In an embodiment according to the present invention the recombinant human prothrombin or thrombin prepared according to the present invention contains a gla domain (SEQ ID NO: 16).

One embodiment according to the present invention provides the recombinant human prothrombin or thrombin has at least 85%, at least 90%, at least 95%, at least 96%, at least 97%, at least 98%, at least 99% or 100%, nucleic acid homology with the natural human prothrombin or thrombin gene.

One embodiment according to the present invention provides the recombinant human prothrombin or thrombin has at least 80%, at least 85%, at least 90%, at least 95%, at least 96%, at least 97%, at least 98%, at least 99% or 100%, amino acid homology with the natural human prothrombin or thrombin.

One embodiment according to the present invention provides at least 90%, such as at least 91 %, at least 92%, least 93%, at least 94%, least 95%, at least 96%, least 97%, at least 98%, least 99%, or 100%, of the protein has a glycosylation pattern that results in an immunogenicity response substantially identical to that of the natural human prothrombin or thrombin.

In an embodiment according to the present invention the recombinant human thrombin clots fibrinogen at a faster rate than the natural human thrombin.

In an embodiment according to the present invention the recombinant human thrombin retains at least 50%, such as at least 60%, at least 70%, at least 80%, at least 90%, or 100%, of the initial fibrinogen polymerization activity after one week of storage at 4-8°C.

In an embodiment according to the present invention the recombinant human prethrombin or prothrombin can be activated by any means that are known to a person skilled in the art. In a preferred embodiment of the present invention the recombinant human prethrombin or prothrombin is activated to recombinant human thrombin by use of ecarin. Activation with ecarin has the further advantages of making immobilisation of the activator possible; ecarin therefore provides for a one step purification method of recombinant human thrombin.

In an embodiment according to the present invention the recombinant human prothrombin has a significantly lower autolytic activity than human natural prothrombin.

In an embodiment according to the present invention the human recombinant prothrombin is as defined in SEQ ID NO: 2 or SEQ ID NO: 4. In a further embodiment the human recombinant prothrombin as defined herein is for use in medicine.

In an embodiment according to the present invention the human recombinant prethrombin is as defined in SEQ ID NO: 7 or SEQ ID NO: 4. In a further embodiment the human recombinant prothrombin as defined herein is for use in medicine.

In an embodiment according to the present invention the human recombinant thrombin is as defined in SEQ ID NO: 17. In a further embodiment the human recombinant thrombin as defined herein is for use in medicine.

*Method for the preparation of recombinant human fibrinogen*

Regarding the production of human recombinant fibrinogen, which approaches the authenticity of natural human fibrinogen, human cells or human kidney cell and human retina-derived PER-C6 cells lines, or more specifically the human embryonic kidney (HEK) 293T cell line may be the same type(s) of cells, which has been transfected with cDNA copies of human fibrinogen claimed in this invention, the cells propagated in serum-free medium and producing the human fibrinogen to approach similarity to authentic human fibrinogen from the cells due to the incorporation of the human genes encoding the $\alpha$, $\beta$, and $\gamma$ chains in the kidney cells secreting the more homogenous fibrinogen in serum-free medium with better functionality and with more consistent lot to lot reproducibility.

An embodiment according to the present invention provides a polynucleotide encoding recombinant human alpha fibrinogen corresponding to SEQ ID NO: 10 (the amino acid sequence in SEQ ID NO: 11) expressed in vitro in a human cell.

An embodiment according to the present invention provides a polynucleotide encoding recombinant human beta fibrinogen corresponding to SEQ ID NO: 12 (the amino acid sequence in SEQ ID NO: 13) expressed in vitro in a human cell.

An embodiment according to the present invention provides a polynucleotide encoding recombinant human gamma fibrinogen corresponding to SEQ ID NO: 14 (the amino acid sequence in SEQ ID NO: 15) expressed in vitro in a human cell.

One embodiment according to the present invention provides a vector comprising one or more of the polynucleotides as defined herein. In a further embodiment the vector comprises all three polynucleotides as defined herein. In yet a further embodiment the vector according to the present invention comprises the polynucleotide operably linked to control sequences recognised by a host cell transformed with said vector. In yet a further embodiment the vector according to the present invention is in the form of a plasmid vector.

One embodiment according to the present invention provides a human host cell comprising one or more vectors as defined herein. In a further embodiment the human host cell is a human embryonic kidney (HEK) cell. In yet a further embodiment the human host cell is selected from the group consisting of HEK 293, HEK 293T, HEK 293S and HEK 293 EBNA.

One embodiment according to the present invention provides a method for the preparation of recombinant human fibrinogen using a human expression system. In a further embodiment the human expression system comprises a vector as defined herein. In yet a further embodiment the human expression system is a human host cell as defined herein. In yet a further embodiment the human expression system is cultured under serum-free conditions.

One embodiment according to the present invention provides recombinant human fibrinogen has at least 85%, at least 90%, at least 95%, at least 96%, at least 97%, at least 98%, at least 99% or 100%, nucleic acid homology with the natural human fibrinogen gene.

One embodiment according to the present invention provides recombinant human fibrinogen has at least 80%, at least 85%, at least 90%, at least 95%, at least 96%, at least 97%, at least 98%, at least 99% or 100%, amino acid homology with the natural human fibrinogen.

One embodiment according to the present invention provides at least 90%, such as at least 91 %, at least 92%, least 93%, at least 94%, least 95%, at least 96%, least 97%, at least 98%, least 99%, or 100%, of the protein has a glycosylation pattern that results in an immunogenicity response substantially identical to that of the natural human fibrinogen.

One embodiment according to the present invention provides a human recombinant fibrinogen comprising alpha chain as defined in SEQ ID NO: 11, beta chain as defined in SEQ ID NO: 13 and gamma chain as defined in SEQ ID NO: 15 and expressed in vitro in a human cell. In a further embodiment according to the present invention a human recombinant fibrinogen according to claim 43 is for use in medicine.

*Method for the preparation of recombinant human collagen*

It is contemplated that the present method also may be used to produce other recombinant human proteins such as recombinant human collagen. Accordingly, in the following is given a brief description in this respect.

The collagen will be co-expressed with prolyl 4-hydroxylase, which hydroxylates specific proline residues of collagen, which has been transfected with cDNA copies of human collagen such as for instance any of the 19 - 20 known human collagens such as Collagen types I, II, III, IV, V, VI, VII, VIII, IX, X, XI, XII, XIII, XIV, XV, XVI, XVII, XVIII, XIX, - or any of these known collagens. In the absence of praline hydroxylation, the essential triple-helical conformation of collagen is thermally unstable - for instance below physiological temperature.

These human collagen types, propagated in cells propagated in serum-free medium and producing the human collagen(s) or, more specifically, collagen of any of the types of the following collagens, e.g., I, II, III, IV, V, VI, VII, VIII, IX, X, XI, XII, XIII, XIV, XV, XVI, XVII, XVIII, XIX, in serum-free medium, to approach similarity to authentic human collagen(s) and/or specifically collagen type III. In this invention we have preferred to use human cells (among these stem cells, precursor cells or alike) for all these proteins, and more specifically, human kidney cell and human retina-derived PER-C6 cell lines, and even more specifically, a human embryonic kidney (HEK) 293T cell line, which is an immortalized cell line, described by P. Chen et al. in 2002 (Chen, P., et al., Protein Expression and Purification (2002) 24:481-488).

The following collagens may be found in humans and theoretically used in the production of CFM or CFM-Cell: Collagen types I, II, III, IV, V, VI, VII, VIII, IX, X, XI, XII, XIII, XIV, XV, XVI, XVII, XVIII, XIX, - all of these known collagens. Then there are a few recently detected collagens such as collagen type XX: recently collagen subfamily; type XXI: Type XXI collagen is an extracellular matrix component of the blood vessel walls, secreted by smooth-muscle cells; XXIII: recently found collagen in metastatic tumors; XXIV a fibrillar vertebrate collagen; XXVI: new member of collagens found in ovary and testes; XXVII: novel highly conserved collagen (pro(alpha)1 (XXVII)., type XXIII: recently found collagen in metastatic tumors; XXIV a fibrillar vertebrate collagen; XXVI: new member of collagens found in ovary and testes; XXVII: novel highly conserved collagen (pro(alpha)1 (XXVII). Any of the above mentioned collagens may be used in form of human collagens or human recombinant collagens, or parts of human recombinant collagens, also called "synthetic human natural collagens". The human recombinant collagen types described in this invention such as for instance, collagen type I, collagen II and/or collagen III, but not limited to these three types, - may be added to create an optimal tissue sealant. The recombinant human collagen prepared according to this invention in human cells, and more specifically human kidney cell and human retina-derived PER-C6 cells or even more specifically, cell lines called HEK 293, HEK 293T, HEK 293S, and HEK 293 EBNA. Stem cells or precursor cells may also be used in this regard. The resulting secreted collagen will be prepared in an appropriate reagent such as e.g. in a medium capable of dissolving the collagen (there are over 20 known collagens, whereof human derived or human recombinant derived collagens will be preferred); examples of suitable media include diluted hydrochloric acid (e.g. 10 mM) or other diluted inorganic or organic acid or an aqueous medium having a pH of 7 or less.

*Applications of human recombinant proteins according to the present invention*

Recombinant thrombin and a human recombinant collagen based matrix named HUMA-SEALANT™- C, encompassed in this invention is a biological tissue sealant, which enables the usage of human recombinant thrombin produced by over-expression of the protein in specialized human cells, either together with human recombinant fibrinogen produced by over-expression of the protein in specialized human cells. Furthermore, human recombinant collagen or collagens such as for instance any of the approximately 20 human known types.

An optimal tissue sealant may also be accomplished by utilizing the human recombinant thrombin and the human recombinant fibrinogen, which even may be controlled in regards to the gelation of the tissue sealant by adding a recombinant aprotinin, and even this recombinant aprotinin may be produced by human cells or even by human kidney cell and human retina-derived PER-C6 cells. It may be an advantage to utilize multipotent cells from human umbilical cord or human umbilical cord blood to obtain a cell source that may be even more optimal in producing said proteins, obtaining a solid adherence, producing an easy and safe product as biological tissue sealant, to be used e.g. for wound healing, diffuse bleeding from organs and for any sealant purpose where fibrin glue or known tissue sealant normally containing either natural human proteins or in many cases, the addition of bovine (or other animal derived) proteins, which in relation to human natural non recombinant proteins may be capable of transmitting microbial (including viral) organisms or even, besides this risk, may be able to produce immunogenic reactions, due to for instance xenogeneic proteins (animal proteins as seen in tissue sealant products such as Tisseel® or Beriplast®, where the proteins are of natural human origin and not of recombinant origin, or in the case of tissue sealant such as both Tiseel and Beriplast, aprotinin is of bovine origin. The above described combinations or parts thereof may be used as vehicle for and guidance of cells during cell transplantation, and as carrier(s) of diagnostic or therapeutic drugs.

The present invention relates to a novel concept and method for preparing one or more compositions of human recombinant proteins that either is used in combinations as for instance recombinant human thrombin(s) and recombinant human thrombin mutants with high pro-coagulating activity and low anti-coagulating activity, and with cell signalling capability equal to or around the same signalling capability as wild type human thrombin and, in certain conditions with low or almost no existing degree of autolysis, rendering these capable of retaining at least their pro-coagulating enzymatic activity among others to human fibrinogen and recombinant human fibrinogen even when kept at refrigerator temperature (ranging from 3 to 17°C) rendering these relatively stable recombinant thrombin useable in hemostatic kits kept at refrigerated temperature for an extended period such as for instance weeks or months. These recombinant human thrombin and human recombinant thrombin mutants shall be capable of reacting with recombinant human fibrinogen(s) to create hemostatic effect, clotting effect, gelating effect and adhering effect at least around equal to or better than other recombinant or natural thrombin to create fibrin. The recombinant human thrombin and fibrinogen shall be non-toxic to mammal cells including human cells, and capable of being injected together with cells or tissue, and in that way be capable of acting as cell carrier or capable of having adhesive effect together with cells for said cells to be injected, or implanted to target areas thus aiding cells to work as repair cells or substitute cells when implanted in the organism or in particular organs or tissue. The fibrin produced by the recombinant thrombin and recombinant fibrinogen or with at least one of these components being recombinant and the other being natural (e.g., fibrinogen).

The recombinant fibrin will be capable of substituting all other products, where natural fibrin-, non- toxic to live cells, is used, but with the advantage that it solely consists of recombinant human proteins resembling authentic human proteins, and mutants thereof in order to specifically obtain for instance recombinant- human thrombin(s) mutants especially highly capable of cleaving fibrinogen(s) thereof recombinant mammal, including recombinant human fibrinogen (s) and at the same time capable of showing PAR 1 signalling activity, and at the same time showing little or no activity on protein C.

Said recombinant fibrin will have the advantage of not carrying any microbial or infective material that otherwise relatively frequently is found in natural fibrin, derived from natural (human or bovine or from any other species) thrombin that has been reacting with natural fibrinogen or human or mammal. It has relatively frequently been noted, that natural thrombin and natural fibrinogen from blood of mammal origin may carry unnecessary risks of infecting humans or mammals with microbials (e.g., hepatitis B, hepatitis C, virus transferred from other species, such as for instance bovine, etc.), and even pathological proteins such as prions from humans or mammals including those of bovine origin.

Apart from being a cell carrier, which is a minor niche for the product made by using recombinant proteins within the scope of this invention, this particular tissue sealant (contrary to tissue sealants such as for instance Tisseel) has a broad area of application during cell transplantation surgery.

It is also within the scope of this invention to combine recombinant collagens or more specifically, recombinant human collagens, e.g., collagen type I, and/or type 2, and/or type 3 with recombinant or natural fibrinogen as described in U.S. provisional patent no. 60/722,366, in which application it has been shown that recombinant human collagen (e.g., type III) and form a gelating and adhering (clotting) effect together with fibrinogen.

It is within the scope of this invention to produce recombinant human fibrinogen such as fibrinogen made in mammal cells containing the cDNA for all three A$\alpha$, B$\beta$ and $\gamma$ genes that are expressed to create the protein. It is also within the scope of the invention that recombinant fibrinogen and/or recombinant thrombin can be combined with recombinant mammal such as recombinant human collagens (e.g., recombinant collagen type I or III) as described above.

Another approach within the scope of this invention is the usage of one single recombinant human protein such as for instance certain recombinant Human thrombin analogue(s) and/or recombinant thrombin mutant(s), produced in mammal cells intended to be either used individually as sole product(s) without other combinations for local hemostasis or in certain bleeding areas in the organism where the applied recombinant human thrombin can react with fibrinogen present in the bleeding area, and thus prevent or stop the bleeding. It is also within the scope of this invention to produce recombinant human fibrinogen such as fibrinogen made in mammal cells containing all three A$\alpha$, B$\beta$ and $\gamma$ genes that are expressed to create the protein where this product can be used as one single product. This recombinant human fibrinogen having a HMW at around 330,000 to 340,000 daltons for the treatment of genetic disorders such as hypofibrinogenimia or dysfibrogenimic disorders, and especially as part of the treatment of disseminated intravascular coagulation (DIC). The recombinant human fibrinogen product, when used as an individually administered biologics, it will be formulated in such a manner that it can be administered by injection/infusion in mammals including humans.

In particular, it is also within the scope of this invention to focus on creating said sole proteins that may mimic human active proteins participating in repairing bleeding disorders such as for instance disseminated coagulation disorders, or more inherent disorders, where patients may be lacking or having dys-functioning proteins that otherwise would participate in appropriate coagulation, as for instance disorders with impaired inherited fibrinogen deficiency syndroms or other abnormalities in the creation of intact fibrinogen proteins, such as dysfibrinogenesis, hypofibrinogenesis disorders or especially in disorders such as intravascular disseminated coagulation (DIC).

*The Unique Recombinant Tissue sealant made from the same type of human cell lines to obtain authentic natural proteins constituting the Tissue sealant*

In order to obtain a unique tissue sealant, the procedure in producing the recombinant human proteins must approach proteins that appears to be next to or virtually the same as authentic natural human proteins. In other inventions the usage of CHO cells have been described as producers of recombinant thrombin, but we claim that one will not obtain a protein approaching a uniquely authentic protein optimally without using a technology that utilize the cDNA copies transfected into human cells or especially into human kidney cell and human retina-derived PER-C6 cells, or even more specifically into human embryonic kidney (HEK) 293T cell line.

In one embodiment the concentration of thrombin according to the present invention, such as recombinant human natural thrombin or recombinant human thrombin with one or more point mutations, may be less than 20 NIH units/ml, such as 1-20 NIH units/ml, such as less than 15 NIH units/ml, less than 10 NIH units/ml, less than 5 NIH units/ml, or less than 1 NIH unit/ml.

In one embodiment the concentration of fibrinogen according to the present invention, such as recombinant human natural fibrinogen, may be less than around 50 mg/ml, such as 1-50 mg/ml, such as less than 40 mg/ml, less than 30 mg/ml, less than 20 mg/ml, less than 10 mg/ml, or less than 2 mg/ml.

If the recombinant fibrinogen obtained furthermore, is produced using a quite different technology that make the other component of the tissue sealant kit different due to the fact that it may not live up to the requirement set forth in this invention by being produced in yet another way that may distance the protein in the kit, in regards to its authenticity to a human natural protein, the tissue sealant would thus not at all be as unique as the tissue sealant kits claimed in this invention. For instance in U.S. patent # 6,780,411 a combination of recombinant fibrinogen, described as being produced as a recombinant fibrinogen being prepared by a process which involve the production of the recombinant fibrinogen in body fluids of transgenic mammals, such as sheep, pigs, cattle, goats, rabbits and camels. Such process is described in WO-A-9523868, incorporated in the above patent (U.S. Patent # 6,780,411). The other protein involved in the tissue sealant in this invention is recombinant thrombin which is described as being produced by mammal cells such as CHO cells. This combination is therefore significant different from the tissue sealants claimed in our invention from the point of view of the significant different concept in the production of the proteins constituting one of the tissue sealants described by us from the point of view that one protein (thrombin) is made in animal cells (CHO cells) and the other protein (fibrinogen) is made in body fluids of transgenic animals.

Recombinant human thrombin has a wide range of uses ranging from the treatment of coagulation disorders in humans to act as enzymatic initiator of clotting, treatment of burns, of skin grafting, both in minor and in major surgery either alone or as part of a product, such as tissue sealants..

The formulation of various wound tissue adhesives is discussed in detail in U.S. Pat. Nos. 4,427,650, 4,442,655, and 4,655,211, each of which is incorporated herein by reference.

The effective doses of recombinant human thrombin will vary considerably ranging from 100 to 15,000 units depending on the manner in which thrombin is used (the specific activity of pure thrombin is 3,000 units per μg protein).

The recombinant human thrombin made according to this invention may extremely small amounts that normally would not initiate a significant clotting, but when used with collagen of any type, such as for instance recombinant human collagens such as types I, II, III, IV, V, VI, VII, VIII, IX, X, XI, XII, XIII, XIV, XV, XVI, XVII, XVIII, XIX. Therefore, it is anticipated that - contrary to other inventions encompassing the usage of thrombin for creating gelating as well as adhering compounds - where thrombin are used in the area of 100 units to 15,000 units - the amount of thrombin used to induce a gelating (clotting) and/or adhering substance may indeed according to this invention, when used together with collagen and fibrinogen, or with collagen alone, may be significantly lower than people well experienced in the art would have anticipated as possible, and is therefore considered unique for this invention - namely the use of trace of recombinant natural or recombinant thrombin up to 90 units of thrombin added, in order to activate a gelation (clotting) and/or adherence, may indeed be sufficient to obtain said gelation (clotting).

Quite another model of this invention could be the use of some or one of the proteins which are within the scope of this invention that conveniently could be incorporated in substances which again could react with substances added to provoke gelation, adherence or gluing effect, as well as hemostatic effects.

Recombinant human prothrombin or thrombin according to the present invention may be formulated with any known pharmaceutically acceptable excipients.

Recombinant human fibrinogen according to the present invention may be formulated with any known pharmaceutically acceptable excipients.

The present invention additionally encompasses various kits which contain the ingredients and instruments required to carry out and implement the methods herein described, including use as a tissue sealant and/or a hemostatic agent. In one aspect, such a kit may include one or more substances selected from the group consisting of the recombinant human thrombins as recited herein, human fibrinogens as recited collagen, such as collagen of the types I, II, and III and such as recombinant human collagen, protease, such as serine protease, e.g. aprotinin, and one or more pharmaceutically acceptable excipients for use in medicine.

Additional compositions, kits, ingredient assemblies are included within the scope of the present invention for use in connection with the methods herein described as the follows.

**Items**

1. A composition including the following proteins expressed in human cell lines transfected with the individual plasmid constructs containing the cDNA, which individually expresses the following proteins

   a. recombinant human prothrombin
   b. recombinant human thrombin
   b. recombinant human fibrinogen
   c. recombinant human collagen

2. Any of the recombinant human collagens of the types I, II, III, IV, V, VI, VII, VIII, IX, X, XI, XII, XIII, XIV, XV, XVI, XVII, XVIII, XIX comprising the recombinant human collagen described in item 1c.

3. A composition of a mixture of any of the human recombinant proteins described in items 1 and 2, constituting a gel-forming consistency.

4. A composition of a mixture of recombinant human collagen and recombinant human thrombin used in non-physiological extremely small amounts such as a number of units of thrombin from trace of thrombin to 90 units of thrombin will create a gel-forming consistency with or without the addition of fibrinogen.

5. A composition of a mixture of any of the proteins itemed in items 1 - 2, which, when applied to a surface, which may be biological of origin, which in vitro and in vivo adheres to said surface.

6. Composition of the above human recombinant proteins referred to under 1a - 1c comprising human recombinant proteins using cDNA transfection in human stem cells and/or in further differentiated human precursor cells that will secrete one or more of the proteins in item 1, which, when brought into combinations with each other, will form a gel-like substance.

7. Compositions of the above human recombinant proteins referred to under 1a - 1c comprising human recombinant proteins prepared using cDNA transfection in HEK 293, HEK 293T, HEK 293S, HEK 293 EBNA cell lines that will secrete one or more of the proteins in item 1, which, when brought into combinations with each other, will form a gel-like substance

8. Compositions of the above human recombinant proteins referred to under 1a - 1c comprising human recombinant proteins prepared using cDNA transfection in HEK 293, HEK 293T, HEK 293S, HEK 293 EBNA cell lines that will secrete one or more of the proteins in item 1, which, when brought into combinations with each other, will form a gel-like substance that will adhere to surfaces such as biological surfaces in vitro and in vivo.

9. Any composition according to items 1 to 5, which under said conditions can be mixed with live cells and be used as carrier of said cells.

10. Any composition according to items 1 to 5, which under said conditions can be mixed with chemical compounds, acting as carrier for said chemical compounds in vivo for diagnostic and/or for therapeutic application.

11. A composition according to any of the preceding items for use in carrying medicinal drugs to target tissue or target organs.

12. Recombinant mammal thrombin with high pro-coagulant activity and low protein C (anti-coagulant) activating capability expressed in mammal cells

13. Recombinant mammal fibrinogen produced in mammal cells with cDNA expressing, with six polypeptide chains, 2 A$\alpha$, 2 B$\beta$, 2$\gamma$ capable of functioning as a mammal plasma derived fully functioning mammal fibrinogen.

14. A product consisting of a kit containing recombinant mammal or human thrombin as described in item 12 for use in mammals.

15. A product consisting of a kit containing recombinant mammal or human fibrinogen as itemed in item 13 for use in mammals.

16. A product consisting of a kit containing recombinant mammal or human thrombin as itemed in item 12 and containing recombinant human fibrinogen as described in item 13, which is capable of creating fibrin.

17. A product consisting of a kit containing recombinant mammal fibrinogen as itemed in item 13 and recombinant mammal collagen, of collagen types I, II, and/or III for use as a sealant and a dermafilling product.

18. A recombinant mammal thrombin as itemed in item 12 and a recombinant mammal collagen of types I, II, and III for use as a sealant.

19. A method as defined herein, wherein the cells are cultured for a period of 5 to 20 days in suspension culture, and the cells are separated from the supernatant which contains the proteins.

20. A method as defined herein, wherein the recombinant human fibrinogen is purified by one or more methods selected from the group of anion exchange chromatography, affinity chromatography, affinity chromatography with protamine-agarose, immunoprecipitation with monoclonal or polyclonal antibody, affinity chromatography with monoclonal antibody, precipitation with ammonium sulphate.

21. A method according one or more of the above-recited items, wherein the fibrinogen is purified in the presence of at least one protease inhibitor.

An overview of the sequence listings enclosed is shown in Table 1 below.

Table 1

| SEQ ID NO | Description |
|---|---|
| 1 | Polynucleotide sequence of BC051332 (Human Prothrombin) - M400A (Base# 1198,1199,1200; ATG → gcc) |
| 2 | Amino acid sequence of BC051332 (Human Prothrombin) - M400A (Base# 1198,1199,1200; ATG → gcc) |
| 3 | Polynucleotide sequence of BC051332 (Human Prothrombin) - wt |
| 4 | Amino acid sequence of BC051332 (Human Prothrombin) - wt |
| 5 | Polynucleotide sequence of BC051332 (Human Prethrombin)-M256A (Base# 766,767,768; ATG → gcc) |
| 6 | Polynucleotide sequence of BC051332 (Human Prethrombin)-M256A (Base# 766,767,768; ATG → gcc) with HPC4 |
| 7 | Amino acid sequence of BC051332 (Human Prethrombin)-M256A (Base# 766,767,768; ATG → gcc) with HPC4 |
| 8 | Polynucleotide sequence of BC051332 (Human Prethrombin)-wt |
| 9 | Amino acid sequence of BC051332 (Human Prethrombin)-wt |
| 10 | Polynucleotide sequence of Fibrinogen a (NM_021871) |
| 11 | Amino acid sequence of Fibrinogen a (NM_021871) |
| 12 | Polynucleotide sequence of Fibrinogen b (BC106760) |
| 13 | Amino acid sequence of Fibrinogen b (BC106760) |
| 14 | Polynucleotide sequence of Fibrinogen r (BC021674) |
| 15 | Amino acid sequence of Fibrinogen r (BC021674) |
| 16 | GLA domain (where X is defined as gamma-carboxyglutamic acid (Gla)). |
| 17 | Sequence of thrombin analogue M84A from Seq. (calculated from prothrombin Seq. no. 285) comprising alpha-thrombin light (A) chain and heavy (B) chain |

**Legends of Figures**

Fig. 1 show the Prepro Prothrombin Structure (Degen & Davie (1987) Biochemistry 26: 6165 - 6177)

Fig. 2 is an overview of the structure of prethrombin M256A including the domains HPC4, Kringle 2, and Peptidase S. In addition, the site of the point mutation M256A is indicated.

Fig. 3 is an overview of the structure of prothrombin M400A including the domains Gla, Kringle 1, Kringle 2, and Peptidase S. In addition, the site of the point mutation M256A is indicated.

Fig. 4. In order to easily discriminate between the individual expressions such as prothrombins (either gla-domain (containing) prothrombin, gla-domainless prothrombin as used by Zymogenetics for their production of recombinant human thrombin, prethrombin, and prethrombin-1, Fig. 4 provides an overview over these individual terms. As illustrated in Fig. 4, prethrombin = prothrombin without gla domain and without kringle I domain (Gla-less domainless prothrombin). To facilitate purification HPC4 epitope was added on N-terminal. So, the first part of Fig. 4 shows the prothrombin including the Gla domain, and the Kringle 1 and Kringle 2. Furthermore,Fig. 4 illustrates the method of preparing recombinant human prethrombin starting with non-mutated prothrombin.

Fig. 5. The Gla domain of human prothrombin. GLA6 denotes γ-carboxyglutamic acid residues, which when substituted by aspartic acid have little impact on prothrombin function; GLA7, GLA14, GLA19, GLA25, and GLA32 denote residues of intermediate importance to prothrombin function and GLA16, GLA26, and GLA29 denote those residues which are crucial to prothrombin function.

Fig. 6. Lane 1 is the molecular marker, Lane 2 is the control lane, Lane 3 is Prothrombin, Lane 4 is Prethrombin (wild type), Lane 5 is filtrate through MWCO of Lane # 4, Lane 6 is Prethrombin-1 M84A (Prethrombin analogue M256A), and Lane 7 is filtrate through 50 kD MWCO of Lane # 6.

Fig. 7. Untransfected Control cells, which died out (top 6 wells). The M84A (or Prethrombin analogue M256A) transfected cells showed continuous growth in the presence of antibiotics (antibiotic selection) are shown.

Fig. 8. The recombinant human thrombin analogue [M84A (or thrombin analogue M256A)] is shown. To the left a Coomassie blue staining of the medium containing 10% serum. To the right a Western blot reacted with monoclonal antibody against HPC4 (mAb-HPC4), where M84A (or analogue M256A) is visualized as a unique strong band, indicating a high concentration of M84A (or analogue M256A). This shows that when you have a relatively high amount of protein loaded (as seen in the Coomassie blue staining), a relatively high amount of contaminating proteins have to be removed during a purification of M84A (analogue M256A) from a serum containing medium.

Fig. 9. A Western blot is reacted with monoclonal anti HPC4 (mAb-HPC4) from serum-free suspension cell (HEK 293, adapted to suspension) culture.

Fig. 10 shows the Coomassie blue staining of serum-free protein yield, which is significantly more pure than samples containing 10% serum (left gel); the arrow in the left gel indicates the M84A location (or analogue M256A). The Western blot (right gel) is reacted with monoclonal antibody against HPC4 (mAb-HPC4) indicating a strong signal from M84A (or analogue M256A). It is observed that in lanes 2 and 3 on the left side, using Coomassie blue, the total amount of available proteins are visible. The amount of proteins observed is evaluated to be very small. The clones containing M84A (or analogue M256A) appears to be a relatively big part of the proteins (as shown using antibody detection of HPC 4) indicating that it will be very easy to purify the active protein from so relatively small amount of total protein,

Fig. 11 shows the purification of recombinant human thrombin analogue M84A (or analogue M256A). Lane 1 is the molecular marker. Lane 2 is the crude protein containing M84A, and in Lane 3 a completely purified M84A elute is observed.

Fig. 12. The recombinant human fibrinogen contained six polypeptide chains (a hexamer) (340 kDa), distributed as follows: 2A$\alpha$ (66 kDa), 2 B$\beta$ (56-58 kDa), and 2 $\gamma$ (43-45 kDa).

Fig. 13. The gel was stained with simple blue staining. Lane 1 contained the Molecular Marker, Lane 2 fibrinogen, no heat and no DTT, Lane 3 Fibrinogen no heat no DTT, Lane 4 fibrinogen with heat and DTT, and Lane 5 fibrinogen with heat and DTT, meaning that Lane 3, 4 and 5 are under reducing conditions.

Fig. 14. Western Lane Order: Lane 1. MW marker, Lane 2. Fibrinogen, no heat, no DTT, Lane 3. Fibrinogen with heat & DTT,Lane 4. Fibrinogen with heat & DTT, Lane 5. Fibrinogen with heat & DTT. Lane 3, 4 and 5 are under reducing conditions, under which the fibrinogen bands will disappear.

Fig. 15 shows Coomassie blue staining and Western blot analysis on the conditioned medium with Anti-Human prothrombin (Enzyme Research Laboratories). M: Marker, 1. Control medium; 2 prothrombin M84A (or Prothrombin Analogue M400A) ; 3 Prothrombin M84A (or Prothrombin Analogue M400A); 4 Prothrombin M84A (or Prothrombin Analogue M400A); 5 Prothrombin M84A (or Prothrombin Analogue M400A)

Fig. 16. Prethrombin M84A (or Prethrombin analogue M256A was observed using mAb Anti-Protein C (Roche).

Fig. 17. Prethrombin was eluted from crude Prethrombin from serum-free medium using Resin: anti-Protein C Affinity Matrix (Roche).

Fig. 18. Purification and activation of Prethrombin M84A (or Prethrombin analogue M256A to thrombin ($\alpha$-thrombin M84A (or $\alpha$-thrombin analogue M256A).

Fig. 19. A-thrombin Purification from heparin Sepharose Chromatography. Lane 1 shows the Ecarin activated α-thrombin mixture (or α-thrombin analogue M256A) was loaded on a heparin column pre-equilibrated with 10 mM Tris (pH 7.4)/200 mM Choline chloride. Lane 2 shows the Flow - thru which was collected until the baseline was obtained. Lane 3 shows Non-specific contaminant(s) which was washed with 10 mM Tris (pH 7.4)/500 mM Choline chloride. Lane 4 shows the α-thrombin M84A (or α-thrombin analogue M256A) eluted with 10 mM Tris (pH 7.4)/800 mM Choline chloride.

Fig. 20. Fibrinogen Expression from Serum-free suspension of the cells (suspension culture). S lane shows the human fibrinogen obtained in CHO cells (purified fibrinogen (1.4 μg) which were compared to Lane 1 and Lane 2. the human fibrinogen from HEK 293 (e.g., HEK 293T) cells from crude medium after day 6, (15 μl) in suspension culture in duplicate. Lanes S', 1' and 2' show the fibrinogen split up into α, β, and γ after treatment with heat and DTT. Western blot reacted with poly rabbit anti-human fibrinogen (American Diagnostica Inc. #313R) were performed showing S and S' (human fibrinogen (purified) from CHO cells before and after heat and DTT treatment, compared to 2 and 2' recombinant human fibrinogen (crude) before and after heat and DTT treatment.

## Examples

### Example 1

**Method for the preparation of recombinant human prethrombin via a prethrombin with a HPC4 domain**

The human prothrombin gene was purchased from the Gene Bank (GB accession No. BC051332). PCR Gla-domain containing prothrombin region was obtained and an HPC4 (protein C) epitope was added to the 5' end in the DNA. Protein C undergoes $Ca^{2+}$-induced conformational changes required for activation by the thrombin-thrombomodulin complex. A $Ca^{2+}$-dependent monoclonal antibody (HPC4) that blocks protein C activation was used to study conformational changes near the activation site in protein C as shown by Stearns et al (Stearns DJ, Kurosawa S, Sims PJ, Esmon NL, Esmon CT, The interaction of a Ca2+-dependent monoclonal antibody with the protein C activation peptide region. Evidence for obligatory Ca2+ binding to both antigen and antibody, J Biol Chem. (1988) 263(2):826-32).

Point mutation on 84 was made from Methionine to Alanine (ATG → GCC), see sequence of human M84A (SEQ ID NO: 6 and SEQ ID NO: 17), where the sequence (SEQ) numbering on B 116, corresponding to chymotrypsin "84" or prothrombin "400" (or prothrombin analogue M400A) was site mutated as described here, as a point mutation.

Another method for the production of recombinant human thrombin is to activate this thrombin from cloned recombinant Human prethrombin -1 as for instance M84A (or Prethrombin analogue M256A) was inserted into a pHZsec vector, which is a proprietary vector owned by HumanZyme Inc. (HumanZyme Inc., Chicago, U S A). The M84A gene was then amplified in E. coli. The amplified gene was then transfected into human embryonic kidney cells (HEK 293) line in a monolayer cell culture in medium containing serum according to the method developed by HumanZyme Inc. (HumanZyme Inc., Chicago). The expression of the human recombinant thrombin analogue (M84A) was verified by Western blot analysis using mAb-HPC4. From Fig. 6 it is observed that both wild type prethrombin-1 and M84A prethrombin-1 have been successfully expressed from HEK 293 cells.

### Example 2

Recombinant human thrombin analogue (M84A (or analogue M256A) was expressed in HEK 293 cells. Twelve (12) clones were selected from 6 wells to larger plates. The two top clones based on Western blot analysis using monoclonal antibody against HPC4 (mAb-HPC4). The cells were adapted for serum free medium and processed as a suspension culture. The recombinant human prethrombin-1 M84A (or prethrombin analogue M256A) was purified using a pilot size purification method. The yield from a serum-free suspension culture of recombinant HU thrombin analogue (M84A (or thrombin analogue M256A)) per Liter was satisfactory. Untransfected cells (controls) died out while M84A (or analogue M256A) transfected cell lines showed continuous growth in the presence of antibiotics see Fig. 7.

Fig. 8 shows the screening of 12 HEK 293 cell clones from which clones 2 and 11 were selected. Fig. 9 shows a Western blot reacted with monoclonal anti HPC4 (mAb-HPC4) from serum-free suspension cell (HEK 293, adapted to suspension) culture. Fig. 10 shows the two clones judged to give the top results in Western blot selected from Fig. 9. Fig. 11 shows the result of the purification of recombinant human prethrombin-1, M84A crude protein prior to purification, and eluted, purified M84A protein is shown in Fig. 10. Fig. 11 shows the purification of recombinant human thrombin analogue M84A. Lane 1 is the molecular marker. Lane 2 is the crude protein containing M84A (or prethrombin analogue M256A), and in Lane 3 a completely purified M84A (or prethrombin analogue M256A) elute is observed.

Insertion of the DNA sequence and the preparation, transfection, expression, purification, and activation are done by HumanZyme Inc. (HumanZyme Inc. Chicago, USA).

Amino acid sequence of human thrombin analogue M84A from Seq. (calculated from prothrombin Seq. no. 285) is according to SEQ ID NO 17.

This shows that it is relatively easy to purify recombinant human thrombin analogue M84A, when the crude protein prior to the elution only contains relatively few bands of proteins, when compared to the amount of proteins present in cell culture containing serum (as for instance 10% serum).

**Example 3**

**Method for preparing prothrombin with intact Gla domain**

Selection of stable cell line transfected with recombinant human gla-domain containing prothrombin, which can be activated to recombinant thrombin, recombinant human thrombins whereof one of the recombinant human thrombins is the recombinant human thrombin analogue, M84A or even recombinant wild type thrombin derived from activation of gla-domain containing prothrombin. The entire Gla-domain containing prothrombin gene is amplified in *E. coli*, and the amplified Gla-domain containing prothrombin is transfected into HEK 293 (e.g., HEK 293T) cells and grown in monolayer culture containing serum in the medium. Several clones were harvested and the clones indicating the content of M84A (or analogue M400A) was isolated and these clones were adapted into suspension cell culture in serum-free medium. The M84A gla-domain prothrombin was harvested from the suspension cell culture by separating the cells from the supernatant. The M84A (or analogue M400A was then precipitated and purified and activated by one step method using a proteinase.

Amino acid sequence of human thrombin analogue M84A from Seq. (calculated from prothrombin Seq. no. 285) is according to SEQ ID NO: 17.

During the process, the recombinant Gla-domain prothrombin, a prothrombin containing signal peptide, propeptide, Gla domain, two kringle domains and protease domain (e.g., trypsin) is retained.

**Example 4**

**Method for the preparation of fibrinogen**

The recombinant human fibrinogen, and more specifically the recombinant human fibrinogen cloned in Human Embryonic Kidney cells (HEK 293), and even more specifically described as recombinant human authentic fibrinogen, due to the fact that the 6 polypeptides, 2 Aα, 2Bβ, and 2 γ genes could most probably have been cloned into any suitable host cell, but in this invention were cloned into the HEK 293 cells. The human fibrinogen α,β, and γ genes was purchased as GB accession No.: NM_021871 for Fb_Aα; BC106760 fpr Fb_Bβ and BC021674 for Fb_γ, and were tested using PCR for fibrinogen α,β,γ genes. Each gene was cloned into a pHZsec vector. The genes were amplified in E. coli. The polypeptide sequence of human alpha (precursor) fibrinogen is according to SEQ ID NO: 10 and the amino acid sequence of human alpha (precursor) fibrinogen is according to SEQ ID NO: 11. The nucleic acid sequence of the beta chain of human fibrinogen is according to SEQ ID NO: 12 and the amino acid sequence of beta chain of fibrinogen is according to SEQ ID NO: 13. The polypeptide sequence of human gamma fibrinogen is according to SEQ ID NO: 14 and the amino acid sequence of human gamma fibrinogen is according to SEQ ID NO: 15. Thereafter the three genes were transfected into the HEK 293 cell line and grown in monolayer culture with serum. The expression was verified by Western blot analysis of the conditioned medium with poly Ab rabbit antihuman fibrinogen IgG. When comparing the structure of the recombinant fibrinogen, it appeared to be authentic when compared to natural human fibrinogen as seen in Fig. 12, which shows that the recombinant human fibrinogen contains six polypeptide chains (a hexamer) (340 kDa), distributed as follows: 2Aα (66 kDa), 2 Bβ (56-58 kDa), and 2 γ (43-45 kDa).

The expression of the recombinant (authentic) human fibrinogen in HEK 293 cell system is visualized in Fig. 13, where the gel is Coomassie blue stain gel, and in Fig. 14 the recombinant (authentic) human fibrinogen is visualized on Western blot using rabbit antihuman fibrinogen IgG.

The clones giving the highest yield was "antibiotic" selected and the yield of recombinant (authentic human fibrinogen was found to provide a satisfactory amount of recombinant human authentic fibrinogen from HEK 293 cells).

**Example 5**

The human prothrombin gene was purchased from the Gene Bank (GB accession No. BC051332). PCR Gla-domain containing prothrombin region aa#44-622 (579 aa) was subjected to point mutation on 84 (Chy) (or at prothrombin analogue M400A) from Met to Ala (ATG → GCC)

Point mutation on amino acid position 84 (or at prothrombin analogue position 400) was made from methionine to alanine (ATG → GCC);

- M84A-mF: 5' -GAAAAGATATCC**GCC**TTGGAAAAGATC-3'
- M84a-mR: 5' -GATCTTTTCCAA**GGC**GGATATCTTTTC-3'

The recombinant Human Prothrombin M84A (or Human Prothrombin analogue M400A) was then inserted into the pHZsec vector, and amplification of the M84A (or analogue M400A) gene was done in E. coli. The amplified gene was then transfected into HEK 293 (e.g., HEK293T) cell line in monolayer in 10% serum containing medium. The expression was found and verified by Western blot analysis on the conditioned medium with Anti-Human prothrombin (Enzyme Research Laboratories).

As can be seen on Fig. 15, the gla-domain Prothrombin M84A (or prothrombin analogue M400A) is expressed in Monolayer as evidenced by the Coomassie blue and Western analysis, which was performed from the medium supernatant from the cell culture. It appeared that all transfected HEK 293 cells in various wells expressed the prothrombin M84A (or prothrombin analogue M400A).

**Example 6**

Human prothrombin gene was purchased from the Gene Bank (GB accession no. BC051332). PCR prethrombin region aa#206 - 622 (417 aa) and added HPC4 epitope (18 aa) at N-terminus (5'end in DNA). Reference for HPC4 Stearns (Stearns DJ, Kurosawa S, Sims PJ, Esmon NL, Esmon CT. J. Biol. Chem. (1988), 263:826-832) as follows:

**gcagcaaagcttgaagaccaagtagatccgcggctcattgatgggaaggtcgac**ctgtca

**A A K L E D Q V D P R L I D G K V D** L S

Point mutation M84A on amino acid position 84 (or at analogue position M256A) from Met to Ala (ATG → GCC) was done.

- M84A-mF: 5' -GAAAAGATATCC**GCC**TTGGAAAAGATC-3'
- M84a-mR: 5' -GATCTTTTCCAA**GGC**GGATATCTTTTC-3'

The cloned recombinant Human Thrombin M84A (or thrombin analogue M256A) was inserted into the pHZsec vector. The cloned gene was amplified in E. coli. The gene was transfected into HEK 293 (e.g., HEK 293T) cell line in monolayer culture. The expression was verified by Western analysis on the conditioned medium with mAb-HPC4 (see Fig. 8). Twelve clones were selected and cells from 6 wells were transferred to a larger plate. The top two clones based on Western analysis (mAB-HPC4) were selected. These cells were adapted to serum-free medium.

*Pilot Purification of Prethrombin M84A (or analogue M256A)*

As it can be seen from Fig. 16, Prethrombin M84A (or analogue M256A was observed using mAb Anti-Protein C (Roche). In Fig. 17 it can be observed that Prethrombin is eluted from crude Prethrombin from serum-free medium using Resin: anti-Protein C Affinity Matrix (Roche).

*Thrombin M84A Activation by Ecarin*

Ecarin was obtained from Sigma (Sigma E0504), and is Echis carinatus venom, it is independent of Ca++, phospholipids, and plasma clotting factors. It can be used as possible immobilization agent.

The activation reaction condition was as follows, 50 μl of Ecarin (50 EU/ml) was added to 5 ml of M84A prethrombin (or prethrombin analogue M256A) (0.5 mg/ml) either in crude media or in TBS (after purification through HPC4 column). Determination of the activation was done by using the thrombin assay with a chromogenic substrate (FPRpNA from Midwest Bio-Tech # 710013) at room temperature, and the following was done:

- 970 μl of 1 x TBS (Tris-based saline, pH 7.4)
- 25 μl 1.7 mM FPRpNA (final concentration 40 μM)
- 5 μl reaction mixture at each time period.

The purification and activation of Prethrombin M84A (or prethrombin analogue M256A) to thrombin (α-thrombin M84A) is shown in Fig. 18.

*A-thrombin Purification from heparin Sepharose Chromatography*

According to Fig. 19, Lane 1 shows the Ecarin activated α-thrombin mixture was loaded on a heparin column pre-equilibrated with 10 mM Tris (pH 7.4)/200 mM Choline chloride. Lane 2 shows the Flow - thru which was collected until the baseline was obtained. Lane 3 shows Non-specific contaminant(s) which was washed with 10 mM Tris (pH 7.4) /500 mM Choline chloride. Lane 4 shows the α-thrombin M84A eluted with 10 mM Tris (pH 7.4)/800 mM Choline chloride (see Fig. 19).

**Example 7**

Human fibrinogen α, β, and γ genes were purchased from the Gene Bank (GB accession no: NM_021871 for Fb_Aα, BC 106760 for Fb_Bβ, and BC021674 for Fb_γ. The PCR fibrinogen α, β, and γ genes were cloned, each gene into pHZec vector, and the genes were amplified in *E. coli.* The three genes were transfected into HEK 293 (e.g., HEK 293T) cell line in monolayer culture with 10% serum. The expression of fibrinogen was verified by Western blot analysis on the conditioned medium with poly AB rabbit anti-human fibrinogen IgG.

*Fibrinogen Expression from Serum-free suspension of the cells (suspension culture)*

As shown in Fig. 20, S lane shows the human fibrinogen obtained in CHO cells (purified fibrinogen (1.4 μg), which were compared to Lane 1 and Lane 2, the human fibrinogen from HEK 293 (e.g., HEK 293T) cells from crude medium after day 6, (15 μl) in suspension culture in duplicate. Lanes S', 1' and 2' show the fibrinogen split up into α, β, and γ after treatment with heat and DTT. Western blot reacted with poly rabbit anti-human fibrinogen (American Diagnostica Inc. #313R) were performed showing S and S' (human fibrinogen (purified) from CHO cells before and after heat and DTT treatment, compared to 2 and 2' recombinant human fibrinogen (crude) before and after heat and DTT treatment.

The invention thus relates to the following embodiments:

Embodiment 1. A polynucleotide encoding a recombinant human thrombin precursor molecule with a point mutation ATG to GCC.

Embodiment 2. A polynucleotide according to embodiment 1, wherein the polynucleotide has SEQ ID NO: 1 further comprising

i) Gla domain according to its amino acid sequence in SEQ ID NO: 16.

Embodiment 3. A polynucleotide according to embodiment 2, wherein the polynucleotide further comprises

ii) Kringle 1.

Embodiment 4. A polynucleotide according to embodiment 2 or 3, wherein the polynucleotide further comprises

iii) Kringle 2.

Embodiment 5. A polynucleotide according to any of embodiments 1-4, wherein the thrombin precursor molecule is prothrombin.

Embodiment 6. A polynucleotide according to embodiment 1, wherein the recombinant human thrombin precursor molecule has SEQ ID NO: 5.

Embodiment 7. A polynucleotide according to embodiment 6, wherein the thrombin precursor molecule is prethrombin.

Embodiment 8. A polynucleotide according to embodiment 6 or 7, wherein the polynucleotide further comprises HCP4 (protein C), and the recombinant human prethrombin encoded has HPC4 linked at the 5' end (SEQ ID NO: 6).

Embodiment 9. The recombinant human thrombin precursor molecule as defined in any of the embodiments 1-8, wherein said precursor has autolytic activity.

Embodiment 10. A vector comprising the polynucleotide as defined in any of the embodiments 1-9.

Embodiment 11. A vector according to embodiment 10, wherein the polynucleotide is operably linked to control sequences recognised by a host cell transformed with said vector.

Embodiment 12. A vector according to embodiment 10 or 11 in the form of a plasmid vector.

Embodiment 13. A human host cell comprising one or more vectors as defined in any of the embodiments 10-12.

Embodiment 14. A human host cell according to embodiment 13, wherein the cell is a human embryonic kidney (HEK) cell.

Embodiment 15. A human host cell according to embodiment 14, wherein the HEK cell is selected from the group consisting of HEK 293, HEK 293T, HEK 293S and HEK 293 EBNA.

Embodiment 16. A method for the preparation of recombinant human prethrombin, prothrombin, or thrombin using a human expression system.

Embodiment 17. A method according to embodiment 16, wherein the human expression system comprises a vector as defined in any of embodiments 10-12.

Embodiment 18. A method according to embodiment 16 or 17, wherein the human expression system is a human host cell as defined in any of embodiments 13-15.

Embodiment 19. A method according to any of embodiments 16-18, wherein the human expression system is cultured under serum-free conditions.

Embodiment 20. A method according to any of embodiments 16-19, wherein recombinant human prothrombin or thrombin prepared contains a gla domain (SEQ ID NO: 16).

Embodiment 21. A method according to any of embodiments 16-20, wherein the recombinant human prethrombin, prothrombin, or thrombin has at least 85%, at least 90%, at least 95%, at least 96%, at least 97%, at least 98%, at least 99% or 100%, nucleic acid sequence identity with the natural human prethrombin, prothrombin, or thrombin gene.

Embodiment 22. A method according to any of embodiments 16-21, wherein the recombinant human prethrombin, prothrombin, or thrombin has at least 80%, at least 85%, at least 90%, at least 95%, at least 96%, at least 97%, at least 98%, at least 99% or 100%, amino acid sequence identity with the natural human prethrombin, prothrombin, or thrombin.

Embodiment 23. A method according to any of embodiments 16-22, wherein at least 90%, such as at least 91 %, at least 92%, least 93%, at least 94%, least 95%, at least 96%, least 97%, at least 98%, least 99%, or 100%, of the protein has a glycosylation pattern that results in an immunogenicity response substantially identical to that of the natural human prothrombin or thrombin.

Embodiment 24. A method according to any of embodiments 16-23, wherein the recombinant human thrombin clots fibrinogen more efficiently than the natural human thrombin.

Embodiment 25. A method according to any of embodiments 16-24, wherein the recombinant human thrombin retains at least 50%, such as at least 60%, at least 70%, at least 80%, at least 90%, or 100%, of the initial fibrinogen polymerization activity after one week of storage at 4-8°C.

Embodiment 26. A method according to any of embodiments 16-25, wherein the recombinant human prethrombin or prothrombin is activated to recombinant human thrombin by use of ecarin.

Embodiment 27. A human recombinant prothrombin as defined in SEQ ID NO: 2 or SEQ ID NO: 4.

Embodiment 28. A human recombinant prothrombin according to embodiment 27 for use in medicine.

Embodiment 29. A human recombinant prethrombin as defined in SEQ ID NO: 7 or SEQ ID NO: 4.

Embodiment 30. A human recombinant prethrombin according to embodiment 29 for use in medicine.

Embodiment 31. A human recombinant thrombin as defined in SEQ ID NO: 17.

Embodiment 32. A human recombinant thrombin according to embodiment 31 for use in medicine.

Embodiment 33. A polynucleotide encoding recombinant human alpha fibrinogen corresponding to SEQ ID NO: 10 expressed *in vitro* in a human cell.

Embodiment 34. A polynucleotide encoding recombinant human beta fibrinogen corresponding to SEQ ID NO: 12 expressed *in vitro* in a human cell.

Embodiment 35. A polynucleotide encoding recombinant human gamma fibrinogen corresponding to SEQ ID NO: 13 expressed *in vitro* in a human cell.

Embodiment 36. A vector comprising one or more of the polynucleotides as defined in embodiments 33-35.

Embodiment 37. A vector comprising all three polynucleotides as defined in embodiments 33-36.

Embodiment 38. A vector according to embodiment 36 or 37, wherein the polynucleotide is operably linked to control sequences recognised by a host cell transformed with said vector.

Embodiment 39. A vector according to any of embodiments 36-38 in the form of a plasmid vector.

Embodiment 40. A human host cell comprising one or more vectors as defined in any of embodiments 36-39.

Embodiment 41. A human host cell according to embodiment 40, wherein the cell is a human embryonic kidney (HEK) cell.

Embodiment 42. A human host cell according to embodiment 41, wherein the HEK cell is selected from the group consisting of HEK 293, HEK 293T, HEK 293S and HEK 293 EBNA.

Embodiment 43. A method for the preparation of recombinant human fibrinogen using a human expression system.

Embodiment 44. A method according to embodiment 43, wherein the human expression system comprises a vector as defined in any of embodiments 30-33.

Embodiment 45. A method according to embodiment 43 or 44, wherein the human expression system is a human host cell as defined in any of embodiments 40-42.

Embodiment 46. A method according to any of embodiments 43-45, wherein the human expression system is cultured under serum-free conditions.

Embodiment 47. A method according to any of embodiments 43-46, wherein the recombinant human fibrinogen has at least 85%, at least 90%, at least 95%, at least 96%, at least 97%, at least 98%, at least 99% or 100%, nucleic acid sequence identity with the natural human fibrinogen gene.

Embodiment 48. A method according to any of embodiments 43-47, wherein the recombinant human fibrinogen has at least 80%, at least 85%, at least 90%, at least 95%, at least 96%, at least 97%, at least 98%, at least 99% or 100%, amino acid sequence identity with the natural human fibrinogen.

Embodiment 49. A method according to any of embodiments 43-48, wherein at least 90%, such as at least 91 %, at least 92%, least 93%, at least 94%, least 95%, at least 96%, least 97%, at least 98%, least 99%, or 100%, of the protein has a glycosylation pattern that results in an immunogenicity response substantially identical to that of the natural human fibrinogen.

Embodiment 50. A human recombinant fibrinogen comprising alpha chain as defined in SEQ ID NO: 11, beta chain as defined in SEQ ID NO: 13, and gamma chain as defined in SEQ ID NO: 15 and expressed *in vitro* in a human cell.

Embodiment 51. A human recombinant fibrinogen according to embodiment 50 for use in medicine.

## Claims

1. A method for the preparation of recombinant human fibrinogen using a human expression system.

2. A method according to claim 1, wherein the human expression system comprises a vector as defined in any of claims 8 or 9.

3. A method according to claim 1 or 2, wherein the human expression system is a human host cell as defined in claim 10 or 11.

4. A method according to any of claims 1-3, wherein the human expression system is cultured under serum-free conditions.

5. A method according to any of claims 1-4, wherein the recombinant human fibrinogen is encoded by a gene which has at least 85%, at least 90%, at least 95%, at least 96%, at least 97%, at least 98%, at least 99% or 100%, nucleic acid sequence identity with the natural human fibrinogen gene, or wherein the recombinant human fibrinogen has at least 80%, at least 85%, at least 90%, at least 95%, at least 96%, at least 97%, at least 98%, at least 99% or 100%, amino acid sequence identity with the natural human fibrinogen.

**6.** A method according to any of claims 1-5, wherein at least 90%, such as at least 91 %, at least 92%, least 93%, at least 94%, least 95%, at least 96%, least 97%, at least 98%, least 99%, or 100%, of the protein has a glycosylation pattern that results in an immunogenicity response substantially identical to that of the natural human fibrinogen.

**7.** A polynucleotide selected from a polynucleotide encoding recombinant human alpha fibrinogen corresponding to SEQ ID NO: 10 expressed *in vitro* in a human cell, a polynucleotide encoding recombinant human beta fibrinogen corresponding to SEQ ID NO: 12 expressed *in vitro* in a human cell, and a polynucleotide encoding recombinant human gamma fibrinogen corresponding to SEQ ID NO: 13 expressed *in vitro* in a human cell.

**8.** A vector comprising one or more of the polynucleotides as defined in claim 7, such as a vector comprising all three polynucleotides defined in claim 7.

**9.** A vector according to claim 8, such as a plasmid vector, wherein the polynucleotide is operably linked to control sequences recognised by a host cell transformed with said vector.

**10.** A human host cell comprising one or more vectors as defined in claim 8 or 9.

**11.** A human host cell according to claim 10, wherein the cell is a human embryonic kidney (HEK) cel, such as HEK 293, HEK 293T, HEK 293S and HEK 293 EBNA.

**12.** A human recombinant fibrinogen comprising alpha chain as defined in SEQ ID NO: 11. beta chain as defined in SEQ ID NO: 13, and gamma chain as defined in SEQ ID NO: 15 and being expressed *in vitro* in a human cell.

**13.** A human recombinant fibrinogen according to claim 12 for use in medicine.

**14.** A polynucleotide encoding a recombinant human thrombin precursor molecule with a point mutation ATG to GCC.

**15.** A polynucleotide according to claim 14, wherein the polynucleotide has SEQ ID NO: 1 further comprising

i) Gla domain according to its amino acid sequence in SEQ ID NO: 16 and/or
ii) Kringle 1, and/or
iii) Kringle 2.

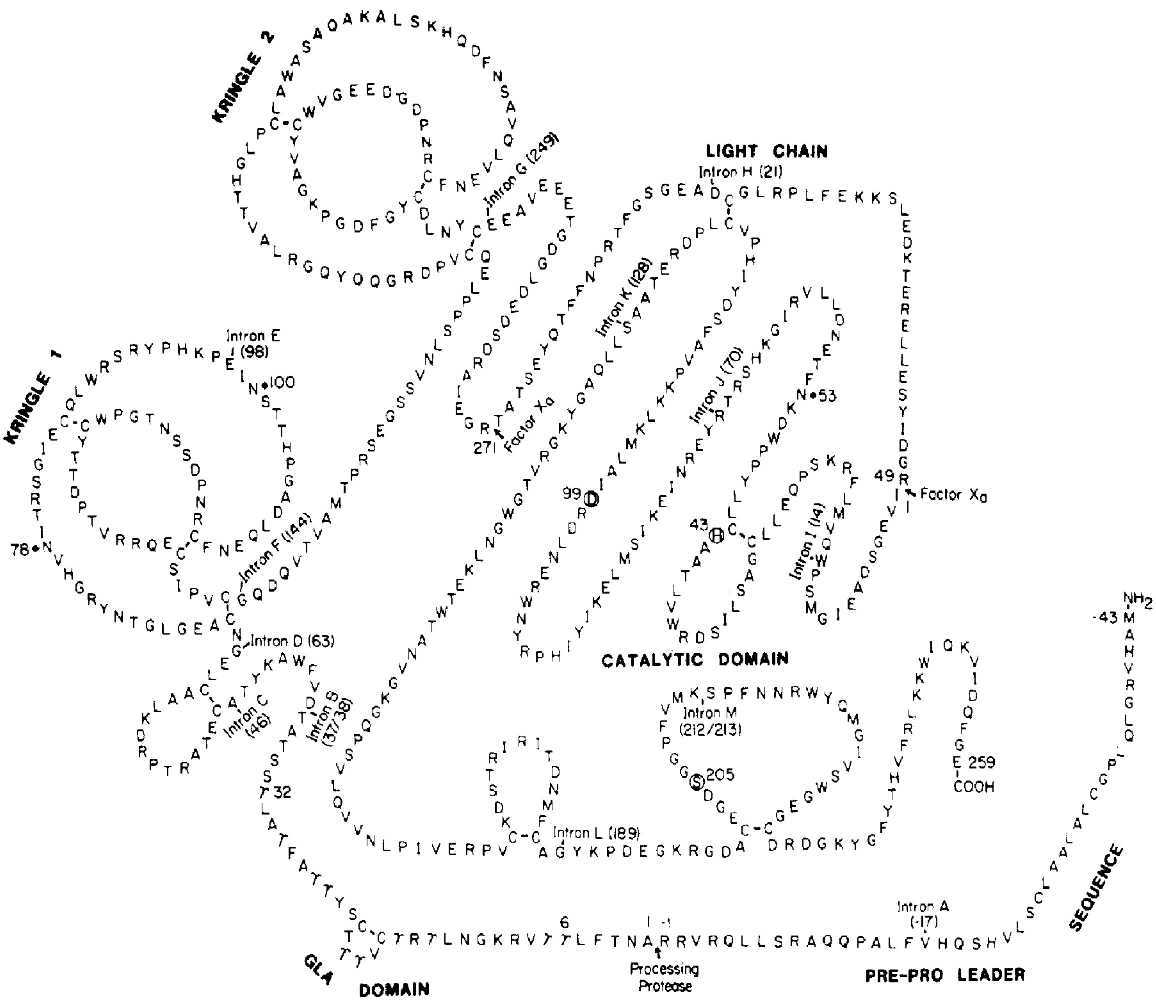
KRINGLE 2
KRINGLE 1
LIGHT CHAIN
Intron H (21)
Intron G (249)
Intron K (128)
Intron E (98)
N•100
Intron J (70)
N•53
271 Factor Xa
49 Factor Xa
99 D
43 H
Intron F (144)
Intron I (14)
78•N
Intron D (63)
Intron C (46)
Intron B (37/38)
CATALYTIC DOMAIN
NH2
-43 M
Intron M (212/213)
S 205
E 259 COOH
32
Intron L (189)
6
1 -1
Intron A (-17)
SEQUENCE
GLA DOMAIN
Processing Protease
PRE-PRO LEADER

# Prethrombin M256A

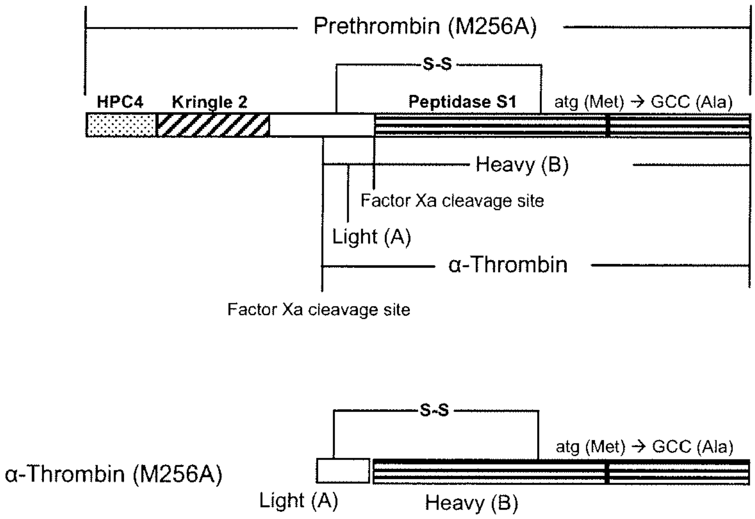


**Fig. 2**

# Prothrombin M400A

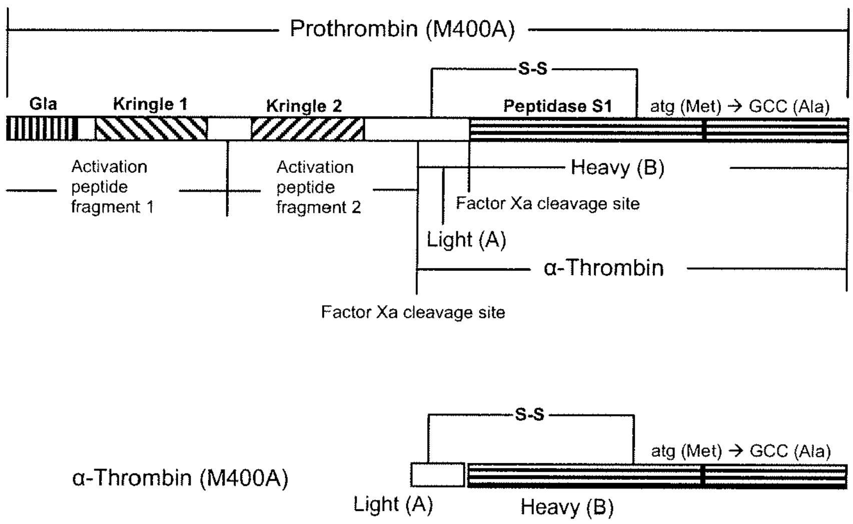


**Fig. 3**

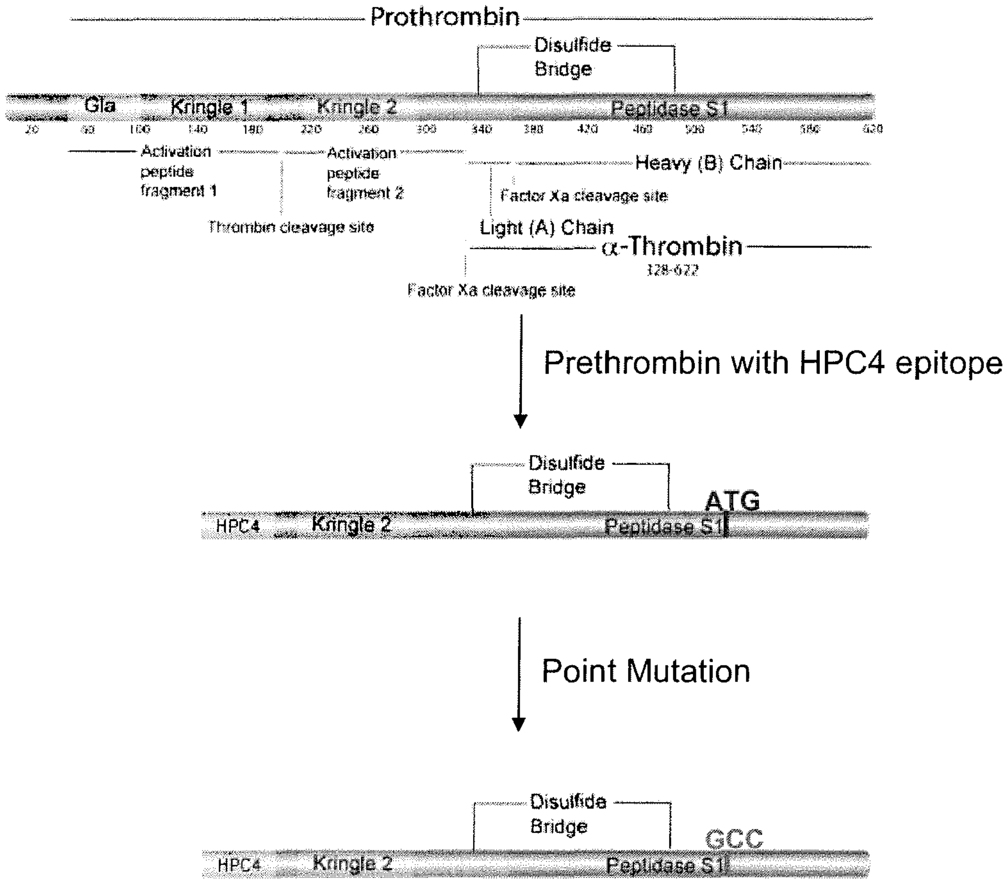

Prothrombin
Disulfide Bridge
Gla
Kringle 1
Kringle 2
Peptidase S1
Activation peptide fragment 1
Activation peptide fragment 2
Heavy (B) Chain
Factor Xa cleavage site
Thrombin cleavage site
Light (A) Chain
α-Thrombin
328-622
Factor Xa cleavage site
Prethrombin with HPC4 epitope
Disulfide Bridge
ATG
HPC4
Kringle 2
Peptidase S1
Point Mutation
Disulfide Bridge
GCC
HPC4
Kringle 2
Peptidase S1


**Fig. 4**

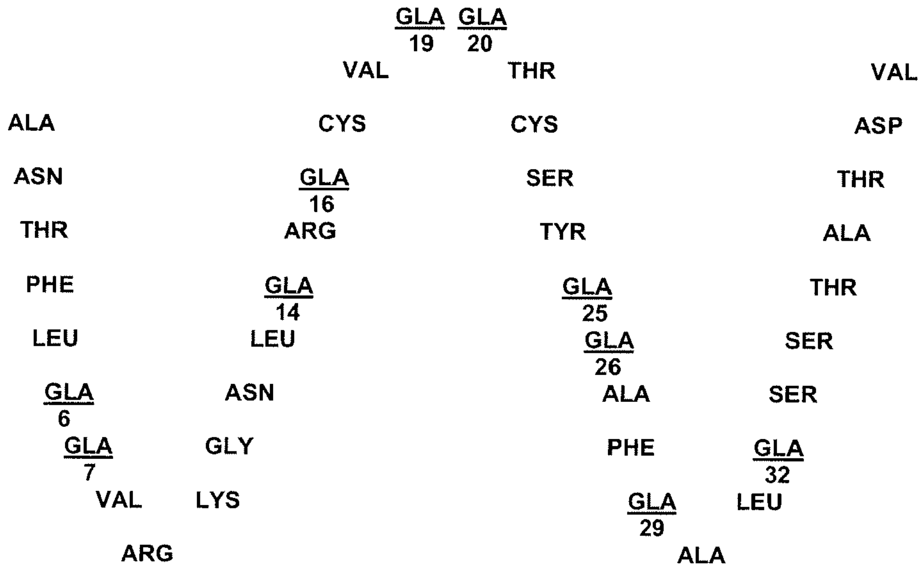
GLA 19
GLA 20
VAL
THR
VAL
ALA
CYS
CYS
ASP
ASN
GLA 16
SER
THR
THR
ARG
TYR
ALA
PHE
GLA 14
GLA 25
THR
LEU
LEU
GLA 26
SER
GLA 6
ASN
ALA
SER
GLA 7
GLY
PHE
GLA 32
VAL
LYS
GLA 29
LEU
ARG
ALA

Fig. 5

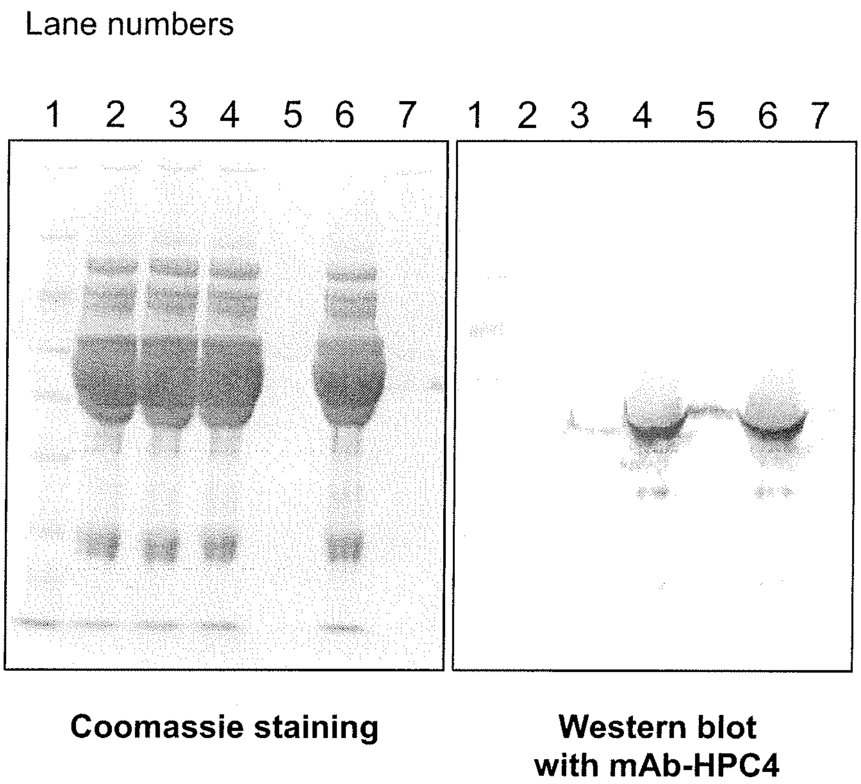
Lane numbers
1 2 3 4 5 6 7 1 2 3 4 5 6 7
Coomassie staining
Western blot
with mAb-HPC4

Fig. 6

| | Viability of cells in the presence of antibiotics |
|---|---|
| Untransfected Control cells | - |
| M84A transfected cells | + |

Fig. 7

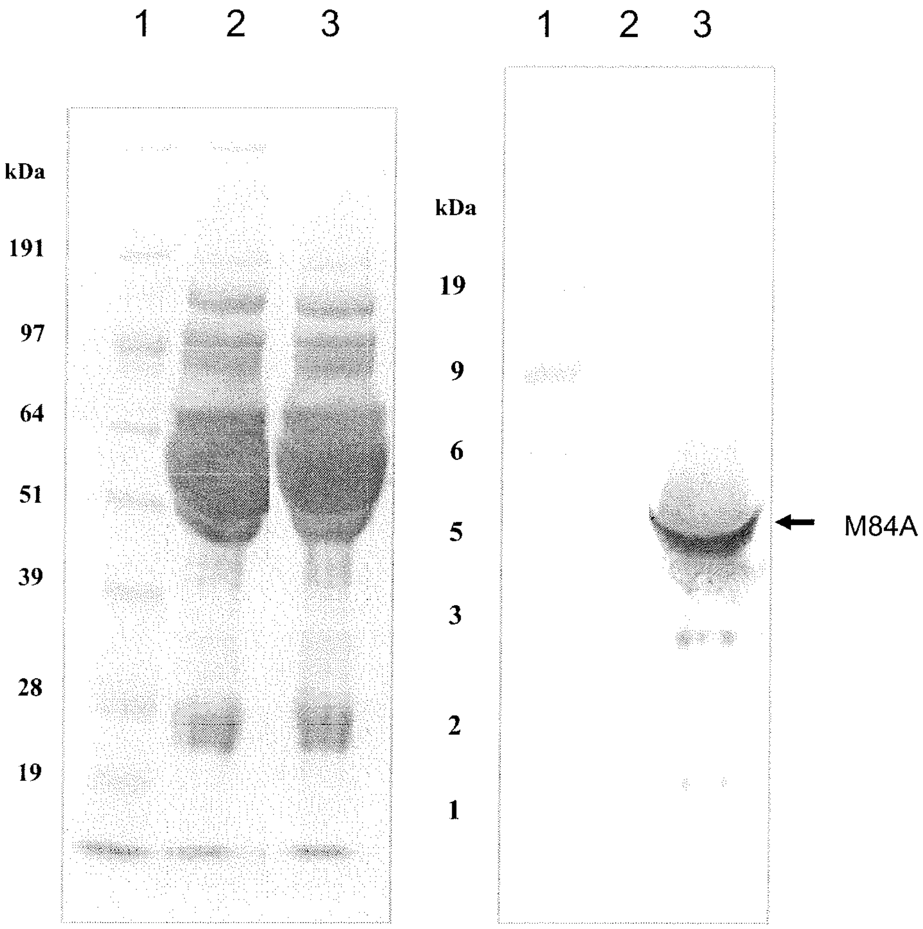


**Fig. 8**

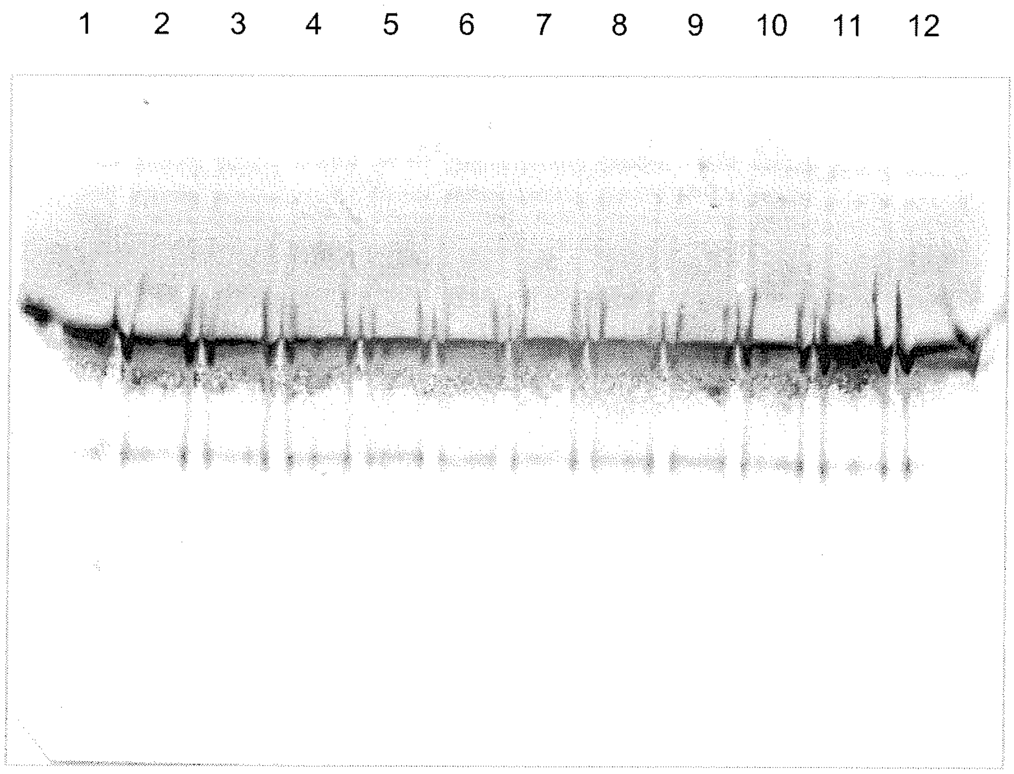


Fig. 9

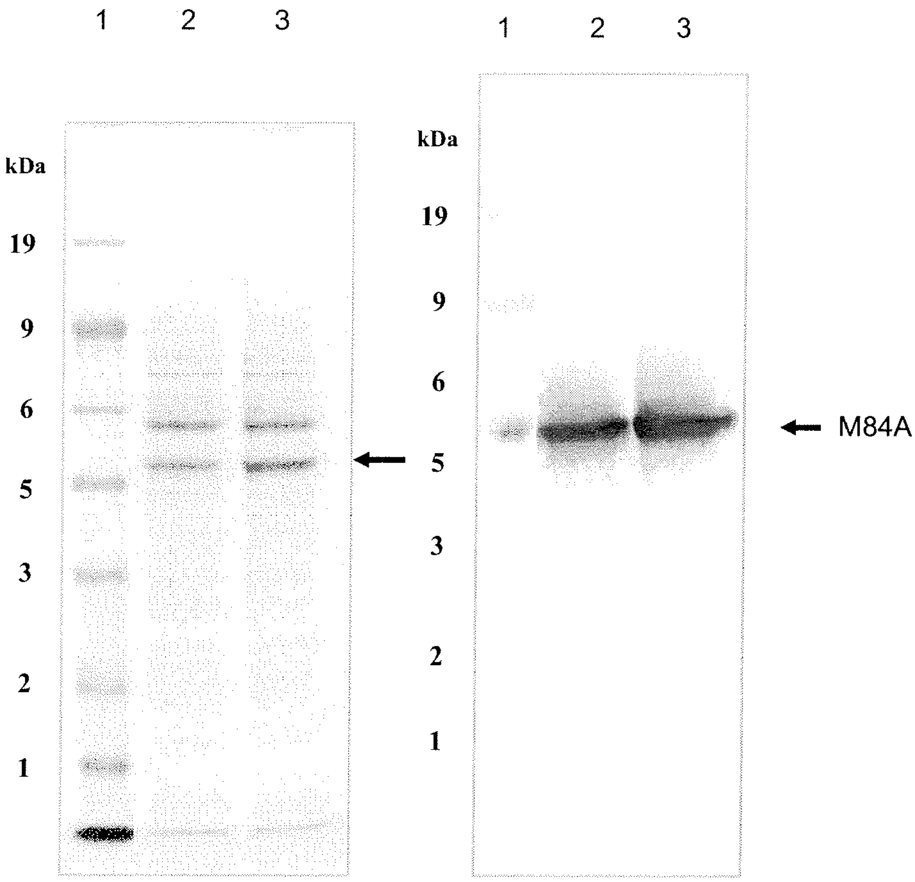


Fig. 10

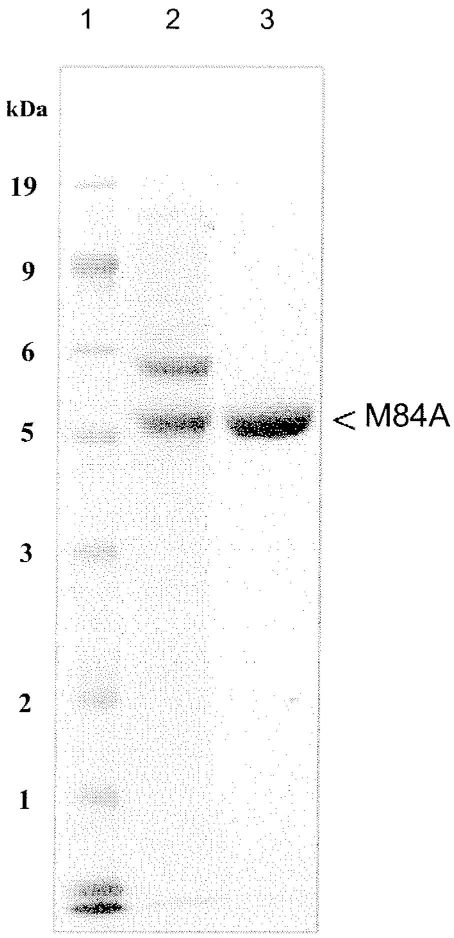


Fig. 11

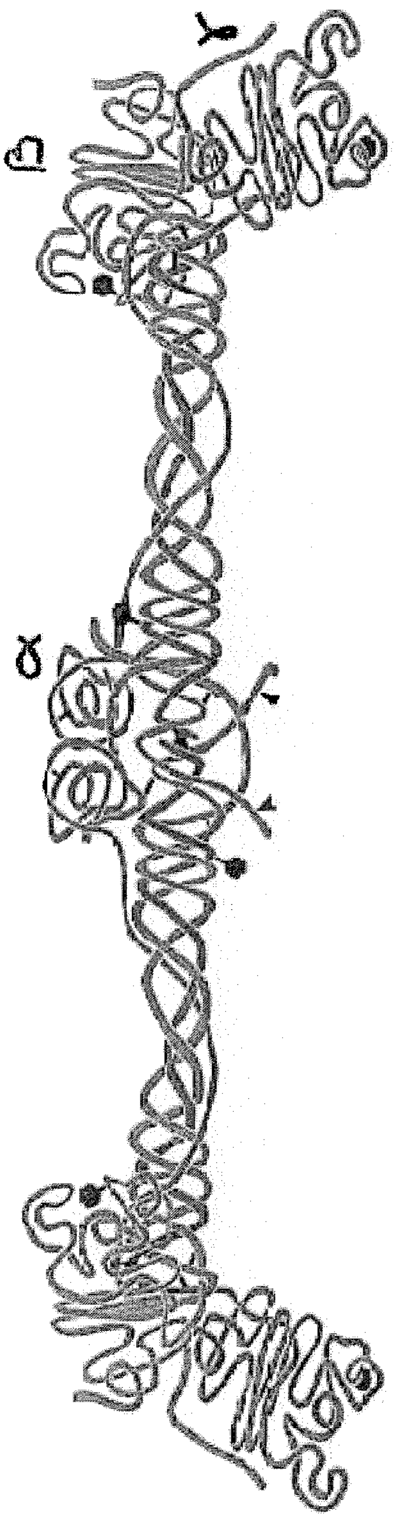


Fig. 12

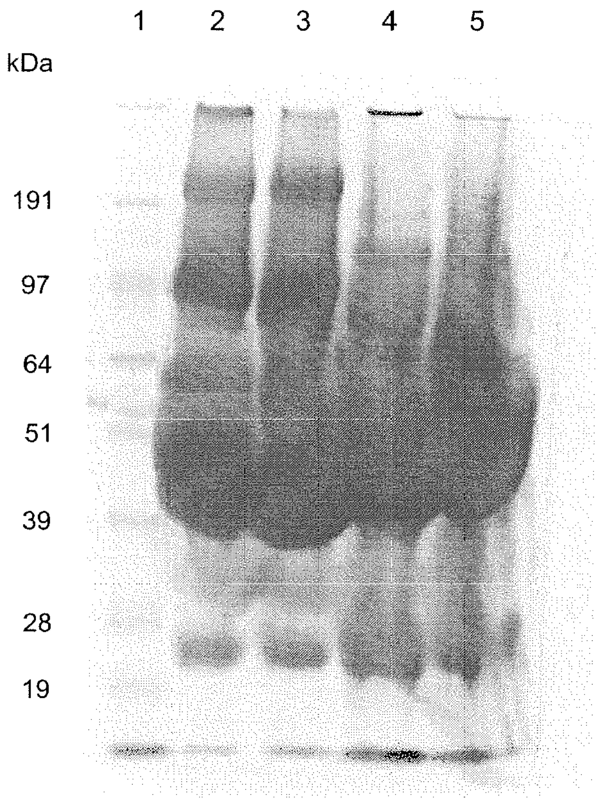


Coomassie blue Gel

Fig. 13

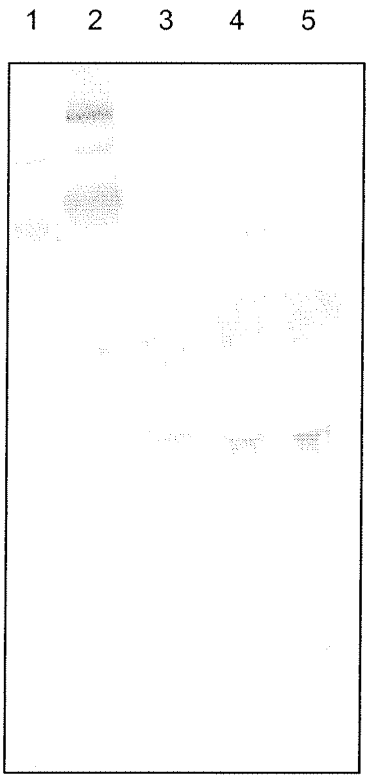


Rabbit Anti-Human Fibrinogen IgG

Fig. 14

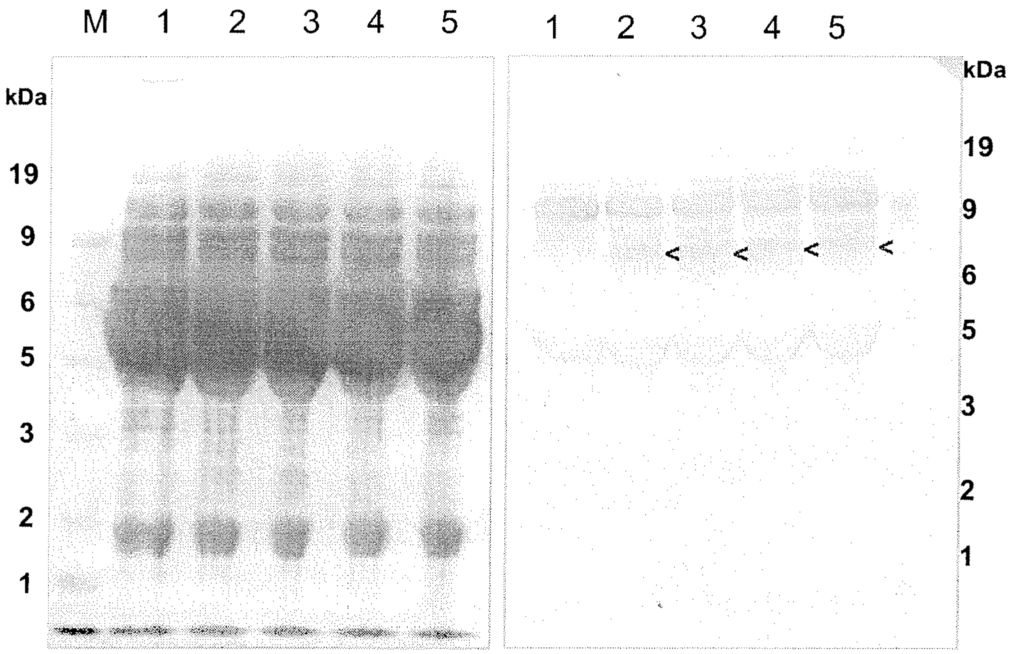


Coomassie blue staining

Western blot analysis on the conditioned medium with Anti-Human prothrombin (Enzyme Research Laboratories)

M: Marker, 1. Control medium; 2 protrhombin M84A; 3 Prothrombin M84A; 4 Prothrombin M84A; 5 Prothrombin M84A

**Fig. 15**

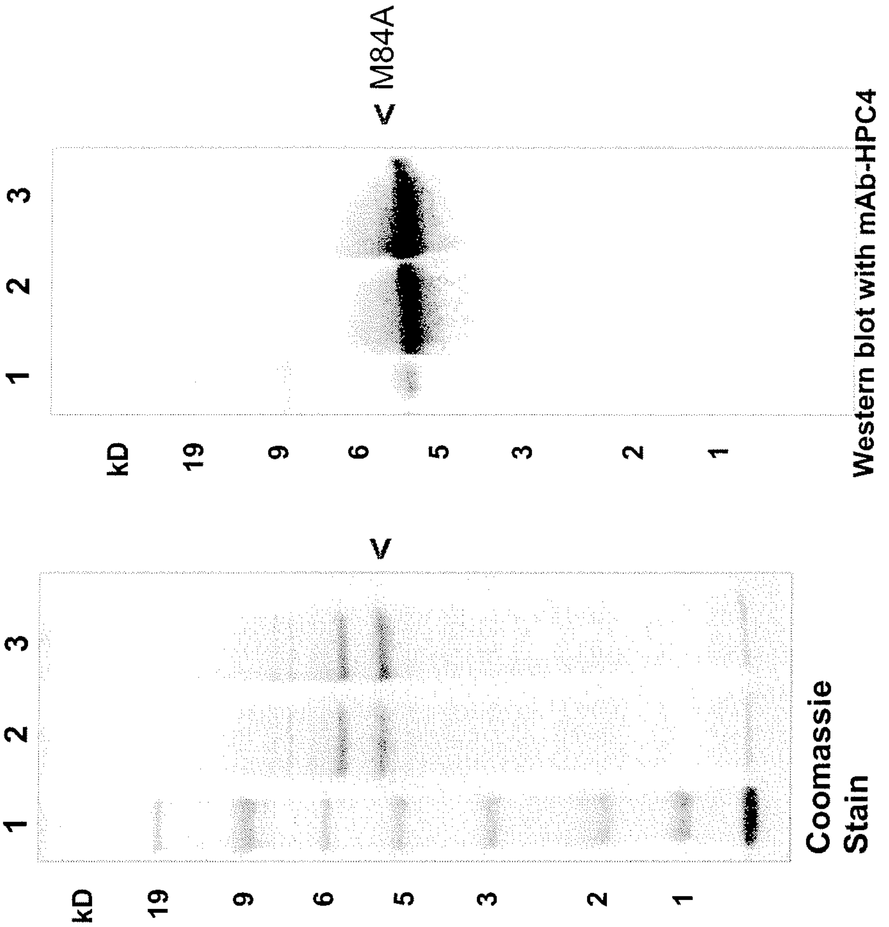
1
2
3
kD
19
9
6
5
3
2
1
Coomassie
Stain
< M84A
Western blot with mAb-HPC4

Fig. 16

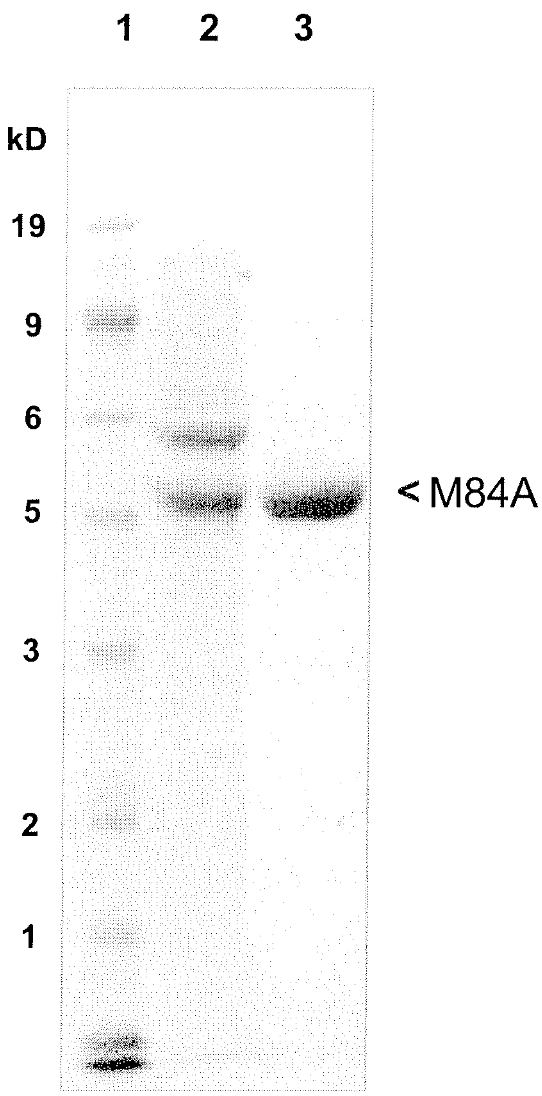


Fig. 17

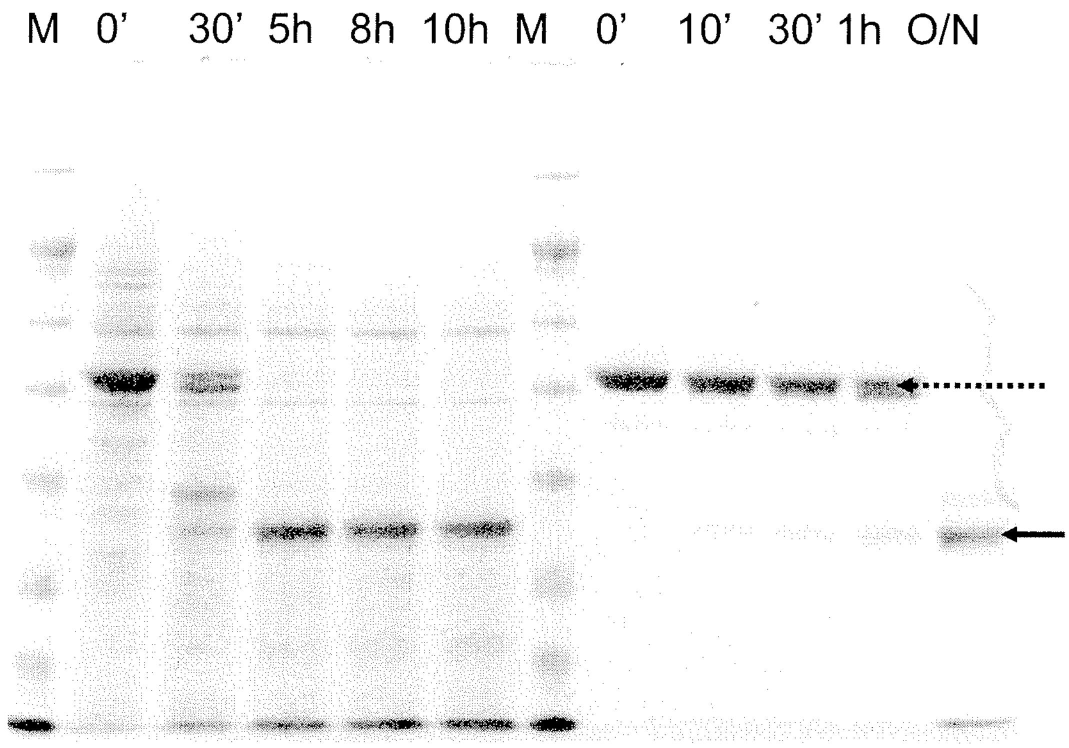


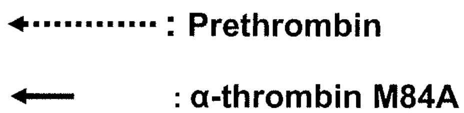


**0'- 10h; (Left gel): Prethrombin M84A crude media**

**0'- 1 h O/N (right gel): Prethrombin M84A purified from HPC4 affinity column**

# Fig. 18

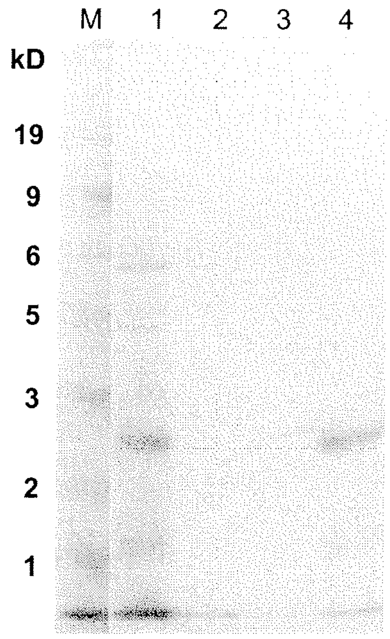


Fig. 19

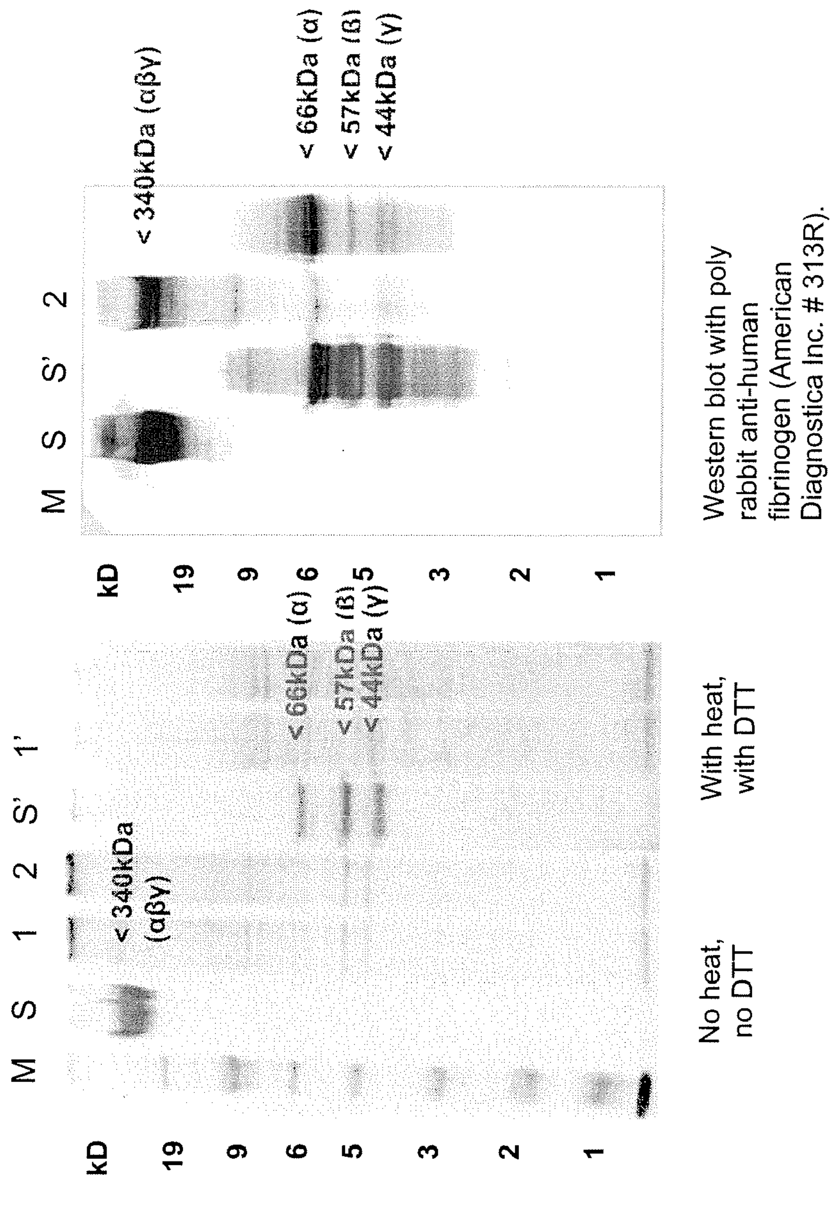
kD
19
9
6
5
3
2
1
M S 1 2 S' 1'
< 340kDa (αβγ)
< 66kDa (α)
< 57kDa (β)
< 44kDa (γ)
No heat, no DTT
With heat, with DTT
M S S' 2
< 340kDa (αβγ)
< 66kDa (α)
< 57kDa (β)
< 44kDa (γ)
Western blot with poly rabbit anti-human fibrinogen (American Diagnostica Inc. # 313R).

Fig. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6780411 B
- US 5502034 A
- US 4931373 A, Kawasaki
- US 6037457 A
- US 779474 P
- US 5476777 A
- US 5572692 A
- US AND5572692 A
- US 722366 P
- US 5527692 A
- US 4546082 A, Kurjan
- US 4870008 A, Brake
- WO 8600637 A, Beck
- US 4613572 A, MacKay
- WO 87002670 A, MacKay
- US 4713339 A
- US 4965203 A
- US 60722366 B
- WO 9523868 A
- US 4427650 A
- US 4442655 A
- US 4655211 A


### Non-patent literature cited in the description


- **MARLOVITS S et al.** *Knee Surg Sports Traumatol Arthrosc.,* 2005, vol. 13, 451-7
- **BARTLETT W et al.** *J Bone Joint Surg Br.,* 2005, vol. 87, 640-5
- **CHRISTMAN KL et al.** *Tissue Eng.,* 2004, vol. 10, 403
- **BERNIE JE et al.** *J Endourol.,* 2005, vol. 19, 1122-6
- **L'ESPARANCE JO et al.** *J Endourol.,* 2005, vol. 19, 1114-21
- **LANGREHR JM.** *Rozhl Chir.,* 2005, vol. 84, 399-402
- **NAKAMURA et al.** *The Amer. Surgeon,* 1991, vol. 57, 226-230
- **THOMPSON et al.** *Ophthalmology,* 1986, vol. 93, 279-282
- **HARRIS et al.** *J. Bone Joint Surg. Am.,* 1978, vol. 60, 454-456
- **CRAIG ; ASHER.** *Spine,* 1997, vol. 2, 313-317
- **PRASAD et al.** *Burns,* 1991, vol. 17, 70-71
- **SUZUKI ; SAKURAGAWA.** *Thromb. Res.,* 1989, vol. 53, 271-278
- **STRICKER et al.** *Blood,* 1988, vol. 72, 1375-1380
- **FLAHERTY ; WEINER.** *Blood,* 1989, vol. 73, 1388
- **FLAHERTY et al.** *Ann Int. Med.,* 1989, vol. 111, 631-634
- **ZEHNDER ; LEUNG.** *Blood,* 1990, vol. 76, 2011-2016
- **LAWSON et al.** *Blood,* 1990, vol. 76, 2249-2257
- **BERGUER et al.** *J. Trauma,* 1991, vol. 31, 408-411
- **WINTERBOTTOM, N. et al.** *J. Applied Res.,* 2002, vol. 2 (1
- **WAI Y ; TSUI V ; PENG Z ; RICHARDSON R ; OREOPOULOS D ; TARLO SM.** *Clin. Exp. Allergy.,* 2003, vol. 33, 1730
- **SCHOENECKER JG ; JOHNSON RK ; LESHER AP ; DAY JD et al.** *American J. of Pathology,* 2001, vol. 159, 1957
- **STRUHL et al.** *Proc. Natl. Acad, Sci. USA,* 1978, vol. 76, 1035-1039
- **BROACH et al.** *Gene,* 1979, vol. 8, 121-133
- **BEGGS.** *Nature,* 1978, vol. 275, 104-108
- **BROOKS, SA.** *Molecular Biotechnology,* 2004, vol. 28, 241-256
- **BROOKS SA.** *Mol. Biotechnol.,* 2004, vol. 28, 241-255
- **BROWN MA ; STENBERG LM ; PERSSON U ; STENFLO J.** *J.Biol.Chem.,* 2000, vol. 275, 19795-19802
- **BOSHART et al.** *Cell,* 1985, vol. 41, 521-530
- **SUBRAMANI et al.** *Mol. Cell. Biol.,* 1981, vol. 1, 854-864
- **WIGLER M ; PELLICER A ; SILVERSTEIN S ; AXEL R.** *Cell,* 1978, vol. 14, 725
- **CORSARO CM ; PEARSON ML.** *Somatic Cell Genetics,* 1981, vol. 7, 603
- **GRAHAM FL ; VAN DER EB AJ.** *Virology,* 1973, vol. 52, 456
- **NEUMANN E ; SCHAEFER-RIDDER M ; WANG Y ; HOFSCHNEIDER PH.** *EMBO J.,* 1982, vol. 1, 841-845

- **GRAHAM FL ; VAN DER EB AJ.** *Virology,* 1973, vol. 52, 456
- **WIGLER M ; PELLICER A ; SILVERSTEIN S ; AXEL R..** *Cell,* 1978, vol. 14, 725
- **CORSARO CM ; PEARSON ML.** *Somatic Cell Genetics,* 1981, vol. 7, 603
- **NEUMANN E ; SCHAEFER-RIDDER M ; WANG Y ; HOFSCHNEIDER PH.** *EMBO J.,* 1982, vol. 1, 841-845
- **KURJAN ; HERSKOWITZ.** *Cell,* 1982, vol. 30, 933-943
- **GRAHAM et al.** *J. Gen. Virol.,* 1977, vol. 6, 59-72
- **HELDEBRANT CM ; BUTKOWSKI RJ ; BAJAJ SP ; MANN KG.** *J. Biol. Chem.,* 1973, vol. 248, 7149-7163
- **DOWNING MR ; BUTKOWSKI RJ ; CLARK MM ; MANN KG.** *J. Biol. Chem.,* 1975, vol. 250, 8897
- **KRISHNASWAMY S ; CHURCH WR ; NESHEIM ME ; MANN KG.** *J. Biol. Chem.,* 1987, vol. 262, 3291
- **SCOPES, R.** Protein Purification. Springer-Verlag, 1982
- **CHEN, P. et al.** *Protein Expression and Purification,* 2002, vol. 24, 481-488
- **DEGEN ; DAVIE.** *Biochemistry,* 1987, vol. 26, 6165-6177
- **STEARNS DJ ; KUROSAWA S ; SIMS PJ ; ESMON NL ; ESMON CT.** The interaction of a Ca2+-dependent monoclonal antibody with the protein C activation peptide region. Evidence for obligatory Ca2+ binding to both antigen and antibody. *J Biol Chem.,* 1988, vol. 263 (2), 826-32
- **STEARNS DJ ; KUROSAWA S ; SIMS PJ ; ESMON NL ; ESMON CT.** *J. Biol. Chem.,* 1988, vol. 263, 826-832